# EUROPEAN PATENT SPECIFICATION

(11) **EP 2 788 483 B1**
(45) Date of publication and mention of the grant of the patent: **27.06.2018**
(21) Application number: 12816132.0
(22) Date of filing: 05.12.2012
(51) Int. Cl.: C12N 15/09

(54) **EXPRESSION CASSETTE**
EXPRESSIONSKASSETTE
CASSETTE D'EXPRESSION

(30) Priority: 07.12.2011 US 201161567675 P
(43) Date of publication of application: 15.10.2014
(73) Proprietor: Glenmark Pharmaceuticals S.A., 2300 La Chaux-de-Fonds (CH)
(72) Inventor: LUESCHER, Daniel, CH-2300 La Chaux-de-Fonds (CH); AEBISCHER-GUMY, Christel, CH-2300 La Chaux-de-Fonds (CH); MORETTI, Pierre, 2300 La Chaux-de-Fonds (FR); BERTSCHINGER, Martin, 2300 La Chaux-de-Fonds (DE)
(74) Representative: Thomas, Dean
(86) International application number: PCT/IB2012/056977
(87) International publication number: WO 2013/084157

(56) References cited:
- EP-A1- 1 308 510
- GRAVEN KRISTA K ET AL: "Identification of an oxygen responsive enhancer element in the glyceraldehyde-3-phosphate dehydrogenase gene", BIOCHIMICA ET BIOPHYSICA ACTA, vol. 1447, no. 2-3, 28 October 1999 (1999-10-28), pages 208-218, XP002694451, ISSN: 0006-3002
- LIMA JULIANA O ET AL: "The glyceraldehyde-3-phosphate dehydrogenase gene of Moniliophthora perniciosa, the causal agent of witches' broom disease of Theobroma cacao", GENETICS AND MOLECULAR BIOLOGY, vol. 32, no. 2, 2009, pages 362-366, XP002694452, ISSN: 1415-4757
- ERCOLANI L ET AL: "ISOLATION AND COMPLETE SEQUENCE OF A FUNCTIONAL HUMAN GLYCERALDEHYDE-3-PHOSPHATE DEHYDROGENASE GENE", JOURNAL OF BIOLOGICAL CHEMISTRY, AMERICAN SOCIETY FOR BIOCHEMISTRY AND MOLECULAR BIOLOGY, US, vol. 263, no. 30, 25 October 1988 (1988-10-25), pages 15335-15341, XP000020878, ISSN: 0021-9258
- SEIDLER NORBERT W: "Basic biology of GAPDH.", ADVANCES IN EXPERIMENTAL MEDICINE AND BIOLOGY 2013, vol. 985, 2013, pages 1-36, XP009168347, ISSN: 0065-2598

### SEQUENCE LISTING

<110> Glenmark Pharmaceuticals SA
<120> Expression Cassette
<130> GBR2001/PCT
<150> US 61/567,675
   <151> 2011-12-07
<160> 39
<170> PatentIn version 3.3
<210> 1
   <211> 39
   <212> DNA
   <213> Artificial
<220>
   <223> Primer 1
<400> 1
   attattcgcg atggctcctg gcatctctgg gaccgaggc 39
<210> 2
   <211> 39
   <212> DNA
   <213> Artificial
<220>
   <223> Primer 2
<400> 2
   atcgtcgcga agcttgagat tgtccaagca ggtagccag 39
<210> 3
   <211> 36
   <212> DNA
   <213> Artificial
<220>
   <223> Primer 3
<400> 3
   agcaagtact tctgagcctt cagtaatggc tgcctg 36
<210> 4
   <211> 39
   <212> DNA
   <213> Artificial
<220>
   <223> Primer 4
<400> 4
   tggcagtact aagctggcac cactacttca gagaacaag 39
<210> 5
   <211> 3179
   <212> DNA
   <213> Artificial
<220>
   <223> 5' GAPDH fragment
<400> 5
   atcgtcgcga agcttgagat tgtccaagca ggtagccaga gagcgccatc agccaagaaa      60
   ccatccactg gtacgtaagg cagcctgtgc gggcgagacc agactgggcc ctcccctcct     120
   gcagtgattt gtttcttctt cttttttaaa tcacgttttc ctgccttttc taggttctag     180
   gtaccagcct ctggcttcta cagcctcaga caatgacttt gtcacaccag agccccgccg     240
   tactacccgt cggcatccaa acacccagca gcgagcttcc aaaaagaaac ccaaagttgt     300
   cttctcaagt gatgagtcca gtgaggaagg tatgatgctc ccgcctgttc ccggccgaga     360
   aggcacacag ctagggtgca gagggctggt ttccatagga cctgctgcgg gggcctgagt     420
   gtagatgctc tgccccactg ccgcagaagg gcctctcctg tacagcttgg attttatttc     480
   ttctgtgcgg tgtgggattg tctcacttgt tctctgatat ctattttttc accatctttg     540
   tgactcagct ttttcttatt cctttaattc tttgcataga tctttcagca gagatgacag     600
   aagacgagac acccaagaaa acaactccca ttctcagagc atcggctcgc aggcacagat     660
   cctaggaagt ctgttcctgt cctccctgtg cagggtatcc tgtagggtga cctggaattc     720
   gaattctgtt tcccttgtaa aatatttgtc tgtctctttt ttttaaaaaa aaaaaaggcc     780
   gggcactgtg gctcacgcct gtaatcccag cactttgcga taccaaggcg ggtggataac     840
   ctgaggtagg gagttcgaga ccagcctgac caacatggag aaaccccatc tctactaaaa     900
   ataaaaaatt agccgggcgt attggcgtgc gcctgtaatc ccagctactc aagaggctga     960
   ggcaggagaa tcgcctgaac ccagaggcgg aggttgtagt gagccgaaat cacaccattg    1020
   cactccagct tgggcaacaa tagcgaacct ccatctcaaa ttaaaaaaaa aatgcctaca    1080
   cgctctttaa aatgcaaggc tttctcttaa attagcctaa ctgaactgcg ttgagctgct    1140
   tcaactttgg aatatatgtt tgccaatctc cttgttttct aatgaataaa tgtttttata    1200
   tacttttaga cattttttcc taagcttgtc tttgtttcat ctttcacatt agcccagttt    1260
   catgcagcag agagagggtt atcagtgcag agagagatga gtgagcccag agtcctaggg    1320
   cctgtcccgg gatggcagat gagcttcctg ccccgtcact gccacctttc ccctctcaac    1380
   ctctggaccc tgcacagtga ccagacagcc tctctgggga gaattatgca gtgcctaggc    1440
   tccagatcag tgcttctgaa ccgggggcaa ttttgtctgc cagaggacat ctgacaacac    1500
   ctggggcctg ttttgttgtc atagcctata ggggaagaat gctaccagca tttgtgggaa    1560
   gaggccaggg atgtggctca acatcctgca gtgcacagga tggcccctca acaaagaatc    1620
   acacggccca caatgtcaat agcgtcacag ttgagaaaac ctgctctaga ccaagggttg 1680
   ctttctgccg tgtgcctcac cccaccccca ctcgtgttcc ctaatcccat ctccaaaggt 1740
   tggcagcaga ccggcccagg ctcgtggaag ttcagatcat gatcccctcc agctctgcag 1800
   gagacaagac ctgtctccca gcattcctca ttgttcccgg gtctgcagag ggcgtgagct 1860
   atgctgcagg cgggctgccc cctgaagcct gcgcacccct ctccagctcc tcaagtcttc 1920
   tctgctgagt caccttcgaa ccggaggctg tgagctggct gtcgtgacca cactggtgcc 1980
   tctgctgtca tgacaacagc acactacgtc agtagtgctc cctgggcact gagctccctc 2040
   tttgcgggga gaagacagta atgaaaaatg acaagcatga ggcagagggg aagatcacgc 2100
   ttgggtggtg caggagcatg gaggtgctct taatgctctc aatgagaaag ggttaacggt 2160
   cctggttgca ggaatagctg agtcagaggt ggggcttcct ccactccccc accccacccc 2220
   tttcaccatt agggaccttc ttgccttgct cttgctactc tgctctgggt ggtcattgtg 2280
   aaaagcccgc accaaccatg ccagtggcag ccagacgagg acacagcctg gctctgggtc 2340
   ccagcaggaa aggcaatccc agaaaggcag ggtcagggac tggagtcctg tgggtgcttt 2400
   ttaagcaaag attatcacca ggcaggctaa acttagcaac cggcttttag ctagaagggc 2460
   agggggctgg tgtcaggtta tgctgggcca gcaaagaggc ccgggatccc cctcccatgc 2520
   acctgctgat gggccaaggc caccccaccc cacccccttc cttacaagtg ttcagcaccc 2580
   tcccatccca cactcacaaa cctggccctc tgccctccta ccagaagaat ggatcccctg 2640
   tgggaggggg caggggacct gttcccaccg tgtgcccaag acctcttttc ccactttttc 2700
   cctcttcttg actcaccctg ccctcaatat cccccggcgc agccagtgaa agggagtccc 2760
   tggctcctgg ctcgcctgca cgtcccaggg cggggaggga cttccgccct cacgtcccgc 2820
   tcttcgcccc aggctggatg gaatgaaagg cacactgtct ctctccctag gcagcacagc 2880
   ccacaggttt ccaggagtgc ctttgtggga ggcctctggg cccccaccag ccatcctgtc 2940
   ctccgcctgg ggccccagcc cggagagagc cgctggtgca cacagggccg ggattgtctg 3000
   ccctaattat caggtccagg ctacagggct gcaggacatc gtgaccttcc gtgcagaaac 3060
   ctccccctcc ccctcaagcc gcctcccgag cctccttcct ctccaggccc ccagtgccca 3120
   gtgcccagtg cccagcccag gcctcggtcc cagagatgcc aggagccatc gcgaataat 3179
<210> 6
   <211> 3238
   <212> DNA
   <213> Artificial
<220>
   <223> 3'-GAPDH fragment
<400> 6
   tggcagtact aagctggcac cactacttca gagaacaagg ccttttcctc tcctcgctcc 60
   agtcctaggc tatctgctgt tggccaaaca tggaagaagc tattctgtgg gcagccccag 120
   ggaggctgac aggtggagga agtcagggct cgcactgggc tctgacgctg actggttagt 180
   ggagctcagc ctggagctga gctgcagcgg gcaattccag cttggcctcc gcagctgtga 240
   ggtcttgagc acgtgctcta ttgctttctg tgccctcgtg tcttatctga ggacatcgtg 300
   gccagcccct aaggtcttca agcaggattc atctaggtaa accaagtacc taaaaccatg 360
   cccaaggcgg taaggactat ataatgttta aaaatcggta aaaatgccca cctcgcatag 420
   ttttgaggaa gatgaactga gatgtgtcag ggtgacttat ttccatcatc gtccttaggg 480
   gaacttgggt aggggcaagg cgtgtagctg ggacctaggt ccagacccct ggctctgcca 540
   ctgaacggct cagttgcttt gggcagttac tcccgggcct cactttgcac gtgtgcttac 600
   ctagtggaga caaaagtaca tacctcggta gagcgcgcac gcctgtaacc ccagcacttt 660
   gggaggccaa ggtgggtgta tcacctgagg tcaggagttt gagaccagcc tggccaacat 720
   ggtgaaactc cgtctctact aaaattacaa aaatcagcca ggcttcatgg cacatgccta 780
   tagtcccagc tacaggcatg ctgaagcagg agaatcgctt gcaccccgga ggcagaggct 840
   gcagtgagct gagaccacac cactgcactc cagcctaggc aacagagtat gagactccat 900
   ctcaaaaaaa aaaaaagtac ctacctcaga gttcaaacta gtgaatatta ggaagtgctt 960
   gagacagtga caccaaagtg cacaataaat actcgccagt ttcattatta ttaaagaatc 1020
   catttgaatg tcagctcaac acagcctcct ataccgaggc attgtgaacc gcatctcccc 1080
   agcttctcca ggcttttcca agaatcaggg acactgtagc ctgttggtct cagtgtatga 1140
   cagacacgga ggaagcacat ctttagctga tacttaaaca gagaccctga gcgcacatac 1200
   acccgcgcac acatgcatgg agcttcacct tctctgtcat tctgcagtga ccaggagagc 1260
   aagagctccc acctcccttc aaaacactgt gcccatcccg ggcactaagg cctctttaaa 1320
   gcacggcacc tccacgaggg agggccacag ccacatacac tccacctggc aggtggacag 1380
   cgtgagcacg tggaccatag cagggacaag gtgccccggc cagccccaac gccctctgcc 1440
   gctgacaggg acagaagccc tctccagctg cgtgtgctgc agaggccatg cgtagcctcc 1500
   agctgcattc tattccactc cagtgcctgg gccagttagc accagtgtgg aagacagtga 1560
   gctggctccg gacaacaggg atggaggaaa ggtcccacat tcacattcct gatacgtgga 1620
   caaggtgagg ggccgcaatc gctctggcag cattttaaag atggggaagt agcagacacc 1680
   cacgcgtgaa ggcaggagag ccccaactgt ggtggaaatg gccccagaat ggtagggcca 1740
   agcctagctc cagacacccc agagccctgg agaagccaag actgagggag aaagcctgag 1800
   ggaggagcgc cccagtcccc agggaccggc ctggtgcaga gctgcagctg atgttcccct 1860
   ctgtgcagcc ccaccctctg cctcgctgag ctccctgctg cgagggcctc gggtgcaagg 1920
   gggaggcagg tctctatctc atggagctgt cagatgagac atcgcgatcg gagtcctcag   1980
   cctcgcttgg cggcggcggc gggtcgctaa gcgggaccgc agtgaaagca ggagactttc   2040
   tagaaaaaaa caccagttgt caaccttggg gcaggcagga atcctgaaga cggacggcac   2100
   tcctcctcct gctgcctcac cctctggcag cccgtgagaa gtaccggaag cgagggcggg   2160
   gccgcgggat ggcgagggag cggcagggac tgaactctct ccaaacccac cctgacaggg   2220
   aaatgggccc cgcctgtgtc ttgggaactc agaggctgag gtcaggcatg atggctcacg   2280
   cctgtaattc cagcactttg ggaggcagag gcgggtggat cacgacgtca ggagttcaag   2340
   gcaagcctgg ccaacatggt gaaaccccat ctctactaaa aatgcaaaaa ttagccaggt   2400
   gtggtggcgg gcgccttgga ggctgaggca agataatcac ttgaacctgg gaggcggagg   2460
   ttgtagtgag ccaaaaaaaa aaaaaaaaga aatagctgaa gtcacagtag gagagaagct   2520
   gctgagcctc cagcaccctg actctagggc cttggcttta tgtctatctg cagtattttt   2580
   gtgattttta aaaattcact ttcttgttgc ggtgtaactt acacagggtc aaatgcacaa   2640
   atcatggccc tgactttaga taaaaatctg cccccacaac ccttctgttc cttgccagtt   2700
   tttaaactgc ctctaaccag gggaaccacc agagctggtg gccttgggag gtttcagccc   2760
   tcccgtcatg aatggacata gctcatccaa ctgccaaggg agagagctgt gggtctgggc   2820
   cagccccacc aggtaactcc caaagggcag ccccacagca agatgtgacc cagtcattgc   2880
   ctgagggtct ctggggctgt gttccaacct ttctccccgc tgtgtccccc tggaaggccc   2940
   catgcccagg ggaggcgcct accggtctcc agactgggtg atgagccggc ggcaggtctc   3000
   catctgcacg tccaggccgc gcttcatgct gcacatctcc atgtactcgt gcaggtgccg   3060
   gttcatgtcg ttcttggccg tggccaactc cagctgtggt ggggcaggca gggccatcgg   3120
   ggttaacagg tggcgttcac agcgcctctg ttgcccccgc caggaggcca acacgccaag   3180
   agcagtggct gggccggggg cccaggcagc cattactgaa ggctcagaag tacttgct     3238
<210> 7
   <211> 3164
   <212> DNA
   <213> Artificial
<220>
   <223> Upstream GAPDH flanking region
<400> 7
   cgaagcttga gattgtccaa gcaggtagcc agagagcgcc atcagccaag aaaccatcca     60
   ctggtacgta aggcagcctg tgcgggcgag accagactgg gccctcccct cctgcagtga    120
   tttgtttctt cttctttttt aaatcacgtt ttcctgcctt ttctaggttc taggtaccag    180
   cctctggctt ctacagcctc agacaatgac tttgtcacac cagagccccg ccgtactacc    240
   cgtcggcatc caaacaccca gcagcgagct tccaaaaaga aacccaaagt tgtcttctca    300
   agtgatgagt ccagtgagga aggtatgatg ctcccgcctg ttcccggccg agaaggcaca 360
   cagctagggt gcagagggct ggtttccata ggacctgctg cgggggcctg agtgtagatg 420
   ctctgcccca ctgccgcaga agggcctctc ctgtacagct tggattttat ttcttctgtg 480
   cggtgtggga ttgtctcact tgttctctga tatctatttt ttcaccatct ttgtgactca 540
   gctttttctt attcctttaa ttctttgcat agatctttca gcagagatga cagaagacga 600
   gacacccaag aaaacaactc ccattctcag agcatcggct cgcaggcaca gatcctagga 660
   agtctgttcc tgtcctccct gtgcagggta tcctgtaggg tgacctggaa ttcgaattct 720
   gtttcccttg taaaatattt gtctgtctct tttttttaaa aaaaaaaaag gccgggcact 780
   gtggctcacg cctgtaatcc cagcactttg cgataccaag gcgggtggat aacctgaggt 840
   agggagttcg agaccagcct gaccaacatg gagaaacccc atctctacta aaaataaaaa 900
   attagccggg cgtattggcg tgcgcctgta atcccagcta ctcaagaggc tgaggcagga 960
   gaatcgcctg aacccagagg cggaggttgt agtgagccga aatcacacca ttgcactcca 1020
   gcttgggcaa caatagcgaa cctccatctc aaattaaaaa aaaaatgcct acacgctctt 1080
   taaaatgcaa ggctttctct taaattagcc taactgaact gcgttgagct gcttcaactt 1140
   tggaatatat gtttgccaat ctccttgttt tctaatgaat aaatgttttt atatactttt 1200
   agacattttt tcctaagctt gtctttgttt catctttcac attagcccag tttcatgcag 1260
   cagagagagg gttatcagtg cagagagaga tgagtgagcc cagagtccta gggcctgtcc 1320
   cgggatggca gatgagcttc ctgccccgtc actgccacct ttcccctctc aacctctgga 1380
   ccctgcacag tgaccagaca gcctctctgg ggagaattat gcagtgccta ggctccagat 1440
   cagtgcttct gaaccggggg caattttgtc tgccagagga catctgacaa cacctggggc 1500
   ctgttttgtt gtcatagcct ataggggaag aatgctacca gcatttgtgg gaagaggcca 1560
   gggatgtggc tcaacatcct gcagtgcaca ggatggcccc tcaacaaaga atcacacggc 1620
   ccacaatgtc aatagcgtca cagttgagaa aacctgctct agaccaaggg ttgctttctg 1680
   ccgtgtgcct caccccaccc ccactcgtgt tccctaatcc catctccaaa ggttggcagc 1740
   agaccggccc aggctcgtgg aagttcagat catgatcccc tccagctctg caggagacaa 1800
   gacctgtctc ccagcattcc tcattgttcc cgggtctgca gagggcgtga gctatgctgc 1860
   aggcgggctg ccccctgaag cctgcgcacc cctctccagc tcctcaagtc ttctctgctg 1920
   agtcaccttc gaaccggagg ctgtgagctg gctgtcgtga ccacactggt gcctctgctg 1980
   tcatgacaac agcacactac gtcagtagtg ctccctgggc actgagctcc ctctttgcgg 2040
   ggagaagaca gtaatgaaaa atgacaagca tgaggcagag gggaagatca cgcttgggtg 2100
   gtgcaggagc atggaggtgc tcttaatgct ctcaatgaga aagggttaac ggtcctggtt 2160
   gcaggaatag ctgagtcaga ggtggggctt cctccactcc cccaccccac ccctttcacc   2220
   attagggacc ttcttgcctt gctcttgcta ctctgctctg ggtggtcatt gtgaaaagcc   2280
   cgcaccaacc atgccagtgg cagccagacg aggacacagc ctggctctgg gtcccagcag   2340
   gaaaggcaat cccagaaagg cagggtcagg gactggagtc ctgtgggtgc tttttaagca   2400
   aagattatca ccaggcaggc taaacttagc aaccggcttt tagctagaag ggcagggggc   2460
   tggtgtcagg ttatgctggg ccagcaaaga ggcccgggat ccccctccca tgcacctgct   2520
   gatgggccaa ggccacccca ccccaccccc ttccttacaa gtgttcagca ccctcccatc   2580
   ccacactcac aaacctggcc ctctgccctc ctaccagaag aatggatccc ctgtgggagg   2640
   gggcagggga cctgttccca ccgtgtgccc aagacctctt ttcccacttt ttccctcttc   2700
   ttgactcacc ctgccctcaa tatcccccgg cgcagccagt gaaagggagt ccctggctcc   2760
   tggctcgcct gcacgtccca gggcggggag ggacttccgc cctcacgtcc cgctcttcgc   2820
   cccaggctgg atggaatgaa aggcacactg tctctctccc taggcagcac agcccacagg   2880
   tttccaggag tgcctttgtg ggaggcctct gggcccccac cagccatcct gtcctccgcc   2940
   tggggcccca gcccggagag agccgctggt gcacacaggg ccgggattgt ctgccctaat   3000
   tatcaggtcc aggctacagg gctgcaggac atcgtgacct tccgtgcaga aacctccccc   3060
   tccccctcaa gccgcctccc gagcctcctt cctctccagg cccccagtgc ccagtgccca   3120
   gtgcccagcc caggcctcgg tcccagagat gccaggagcc atcg                     3164
<210> 8
   <211> 3224
   <212> DNA
   <213> Artificial
<220>
   <223> Downstream GAPDH flanking region
<400> 8
   actaagctgg caccactact tcagagaaca aggccttttc ctctcctcgc tccagtccta      60
   ggctatctgc tgttggccaa acatggaaga agctattctg tgggcagccc cagggaggct     120
   gacaggtgga ggaagtcagg gctcgcactg ggctctgacg ctgactggtt agtggagctc     180
   agcctggagc tgagctgcag cgggcaattc cagcttggcc tccgcagctg tgaggtcttg     240
   agcacgtgct ctattgcttt ctgtgccctc gtgtcttatc tgaggacatc gtggccagcc     300
   cctaaggtct tcaagcagga ttcatctagg taaaccaagt acctaaaacc atgcccaagg     360
   cggtaaggac tatataatgt ttaaaaatcg gtaaaaatgc ccacctcgca tagttttgag     420
   gaagatgaac tgagatgtgt cagggtgact tatttccatc atcgtcctta ggggaacttg     480
   ggtaggggca aggcgtgtag ctgggaccta ggtccagacc cctggctctg ccactgaacg     540
   gctcagttgc tttgggcagt tactcccggg cctcactttg cacgtgtgct tacctagtgg     600
   agacaaaagt acatacctcg gtagagcgcg cacgcctgta accccagcac tttgggaggc 660
   caaggtgggt gtatcacctg aggtcaggag tttgagacca gcctggccaa catggtgaaa 720
   ctccgtctct actaaaatta caaaaatcag ccaggcttca tggcacatgc ctatagtccc 780
   agctacaggc atgctgaagc aggagaatcg cttgcacccc ggaggcagag gctgcagtga 840
   gctgagacca caccactgca ctccagccta ggcaacagag tatgagactc catctcaaaa 900
   aaaaaaaaag tacctacctc agagttcaaa ctagtgaata ttaggaagtg cttgagacag 960
   tgacaccaaa gtgcacaata aatactcgcc agtttcatta ttattaaaga atccatttga 1020
   atgtcagctc aacacagcct cctataccga ggcattgtga accgcatctc cccagcttct 1080
   ccaggctttt ccaagaatca gggacactgt agcctgttgg tctcagtgta tgacagacac 1140
   ggaggaagca catctttagc tgatacttaa acagagaccc tgagcgcaca tacacccgcg 1200
   cacacatgca tggagcttca ccttctctgt cattctgcag tgaccaggag agcaagagct 1260
   cccacctccc ttcaaaacac tgtgcccatc ccgggcacta aggcctcttt aaagcacggc 1320
   acctccacga gggagggcca cagccacata cactccacct ggcaggtgga cagcgtgagc 1380
   acgtggacca tagcagggac aaggtgcccc ggccagcccc aacgccctct gccgctgaca 1440
   gggacagaag ccctctccag ctgcgtgtgc tgcagaggcc atgcgtagcc tccagctgca 1500
   ttctattcca ctccagtgcc tgggccagtt agcaccagtg tggaagacag tgagctggct 1560
   ccggacaaca gggatggagg aaaggtccca cattcacatt cctgatacgt ggacaaggtg 1620
   aggggccgca atcgctctgg cagcatttta aagatgggga agtagcagac acccacgcgt 1680
   gaaggcagga gagccccaac tgtggtggaa atggccccag aatggtaggg ccaagcctag 1740
   ctccagacac cccagagccc tggagaagcc aagactgagg gagaaagcct gagggaggag 1800
   cgccccagtc cccagggacc ggcctggtgc agagctgcag ctgatgttcc cctctgtgca 1860
   gccccaccct ctgcctcgct gagctccctg ctgcgagggc ctcgggtgca agggggaggc 1920
   aggtctctat ctcatggagc tgtcagatga gacatcgcga tcggagtcct cagcctcgct 1980
   tggcggcggc ggcgggtcgc taagcgggac cgcagtgaaa gcaggagact ttctagaaaa 2040
   aaacaccagt tgtcaacctt ggggcaggca ggaatcctga agacggacgg cactcctcct 2100
   cctgctgcct caccctctgg cagcccgtga gaagtaccgg aagcgagggc ggggccgcgg 2160
   gatggcgagg gagcggcagg gactgaactc tctccaaacc caccctgaca gggaaatggg 2220
   ccccgcctgt gtcttgggaa ctcagaggct gaggtcaggc atgatggctc acgcctgtaa 2280
   ttccagcact ttgggaggca gaggcgggtg gatcacgacg tcaggagttc aaggcaagcc 2340
   tggccaacat ggtgaaaccc catctctact aaaaatgcaa aaattagcca ggtgtggtgg 2400
   cgggcgcctt ggaggctgag gcaagataat cacttgaacc tgggaggcgg aggttgtagt 2460
   gagccaaaaa aaaaaaaaaa agaaatagct gaagtcacag taggagagaa gctgctgagc 2520
   ctccagcacc ctgactctag ggccttggct ttatgtctat ctgcagtatt tttgtgattt 2580
   ttaaaaattc actttcttgt tgcggtgtaa cttacacagg gtcaaatgca caaatcatgg 2640
   ccctgacttt agataaaaat ctgcccccac aacccttctg ttccttgcca gtttttaaac 2700
   tgcctctaac caggggaacc accagagctg gtggccttgg gaggtttcag ccctcccgtc 2760
   atgaatggac atagctcatc caactgccaa gggagagagc tgtgggtctg ggccagcccc 2820
   accaggtaac tcccaaaggg cagccccaca gcaagatgtg acccagtcat tgcctgaggg 2880
   tctctggggc tgtgttccaa cctttctccc cgctgtgtcc ccctggaagg ccccatgccc 2940
   aggggaggcg cctaccggtc tccagactgg gtgatgagcc ggcggcaggt ctccatctgc 3000
   acgtccaggc cgcgcttcat gctgcacatc tccatgtact cgtgcaggtg ccggttcatg 3060
   tcgttcttgg ccgtggccaa ctccagctgt ggtggggcag gcagggccat cggggttaac 3120
   aggtggcgtt cacagcgcct ctgttgcccc cgccaggagg ccaacacgcc aagagcagtg 3180
   gctgggccgg gggcccaggc agccattact gaaggctcag aagt 3224
<210> 9
   <211> 511
   <212> DNA
   <213> Artificial
<220>
   <223> Fragment 1
<400> 9
   cgaagcttga gattgtccaa gcaggtagcc agagagcgcc atcagccaag aaaccatcca 60
   ctggtacgta aggcagcctg tgcgggcgag accagactgg gccctcccct cctgcagtga 120
   tttgtttctt cttctttttt aaatcacgtt ttcctgcctt ttctaggttc taggtaccag 180
   cctctggctt ctacagcctc agacaatgac tttgtcacac cagagccccg ccgtactacc 240
   cgtcggcatc caaacaccca gcagcgagct tccaaaaaga aacccaaagt tgtcttctca 300
   agtgatgagt ccagtgagga aggtatgatg ctcccgcctg ttcccggccg agaaggcaca 360
   cagctagggt gcagagggct ggtttccata ggacctgctg cgggggcctg agtgtagatg 420
   ctctgcccca ctgccgcaga agggcctctc ctgtacagct tggattttat ttcttctgtg 480
   cggtgtggga ttgtctcact tgttctctga t 511
<210> 10
   <211> 2653
   <212> DNA
   <213> Artificial
<220>
   <223> Fragment 2
<400> 10
   atctattttt tcaccatctt tgtgactcag ctttttctta ttcctttaat tctttgcata     60
   gatctttcag cagagatgac agaagacgag acacccaaga aaacaactcc cattctcaga    120
   gcatcggctc gcaggcacag atcctaggaa gtctgttcct gtcctccctg tgcagggtat    180
   cctgtagggt gacctggaat tcgaattctg tttcccttgt aaaatatttg tctgtctctt    240
   ttttttaaaa aaaaaaaagg ccgggcactg tggctcacgc ctgtaatccc agcactttgc    300
   gataccaagg cgggtggata acctgaggta gggagttcga gaccagcctg accaacatgg    360
   agaaacccca tctctactaa aaataaaaaa ttagccgggc gtattggcgt gcgcctgtaa    420
   tcccagctac tcaagaggct gaggcaggag aatcgcctga acccagaggc ggaggttgta    480
   gtgagccgaa atcacaccat tgcactccag cttgggcaac aatagcgaac ctccatctca    540
   aattaaaaaa aaaatgccta cacgctcttt aaaatgcaag gctttctctt aaattagcct    600
   aactgaactg cgttgagctg cttcaacttt ggaatatatg tttgccaatc tccttgtttt    660
   ctaatgaata aatgttttta tatactttta gacatttttt cctaagcttg tctttgtttc    720
   atctttcaca ttagcccagt ttcatgcagc agagagaggg ttatcagtgc agagagagat    780
   gagtgagccc agagtcctag ggcctgtccc gggatggcag atgagcttcc tgccccgtca    840
   ctgccacctt tcccctctca acctctggac cctgcacagt gaccagacag cctctctggg    900
   gagaattatg cagtgcctag gctccagatc agtgcttctg aaccgggggc aattttgtct    960
   gccagaggac atctgacaac acctggggcc tgttttgttg tcatagccta taggggaaga   1020
   atgctaccag catttgtggg aagaggccag ggatgtggct caacatcctg cagtgcacag   1080
   gatggcccct caacaaagaa tcacacggcc cacaatgtca atagcgtcac agttgagaaa   1140
   acctgctcta gaccaagggt tgctttctgc cgtgtgcctc accccacccc cactcgtgtt   1200
   ccctaatccc atctccaaag gttggcagca gaccggccca ggctcgtgga agttcagatc   1260
   atgatcccct ccagctctgc aggagacaag acctgtctcc cagcattcct cattgttccc   1320
   gggtctgcag agggcgtgag ctatgctgca ggcgggctgc cccctgaagc ctgcgcaccc   1380
   ctctccagct cctcaagtct tctctgctga gtcaccttcg aaccggaggc tgtgagctgg   1440
   ctgtcgtgac cacactggtg cctctgctgt catgacaaca gcacactacg tcagtagtgc   1500
   tccctgggca ctgagctccc tctttgcggg gagaagacag taatgaaaaa tgacaagcat   1560
   gaggcagagg ggaagatcac gcttgggtgg tgcaggagca tggaggtgct cttaatgctc   1620
   tcaatgagaa agggttaacg gtcctggttg caggaatagc tgagtcagag gtggggcttc   1680
   ctccactccc ccaccccacc cctttcacca ttagggacct tcttgccttg ctcttgctac   1740
   tctgctctgg gtggtcattg tgaaaagccc gcaccaacca tgccagtggc agccagacga   1800
   ggacacagcc tggctctggg tcccagcagg aaaggcaatc ccagaaaggc agggtcaggg   1860
   actggagtcc tgtgggtgct ttttaagcaa agattatcac caggcaggct aaacttagca 1920
   accggctttt agctagaagg gcagggggct ggtgtcaggt tatgctgggc cagcaaagag 1980
   gcccgggatc cccctcccat gcacctgctg atgggccaag gccaccccac cccaccccct 2040
   tccttacaag tgttcagcac cctcccatcc cacactcaca aacctggccc tctgccctcc 2100
   taccagaaga atggatcccc tgtgggaggg ggcaggggac ctgttcccac cgtgtgccca 2160
   agacctcttt tcccactttt tccctcttct tgactcaccc tgccctcaat atcccccggc 2220
   gcagccagtg aaagggagtc cctggctcct ggctcgcctg cacgtcccag ggcggggagg 2280
   gacttccgcc ctcacgtccc gctcttcgcc ccaggctgga tggaatgaaa ggcacactgt 2340
   ctctctccct aggcagcaca gcccacaggt ttccaggagt gcctttgtgg gaggcctctg 2400
   ggcccccacc agccatcctg tcctccgcct ggggccccag cccggagaga gccgctggtg 2460
   cacacagggc cgggattgtc tgccctaatt atcaggtcca ggctacaggg ctgcaggaca 2520
   tcgtgacctt ccgtgcagaa acctccccct ccccctcaag ccgcctcccg agcctccttc 2580
   ctctccaggc ccccagtgcc cagtgcccag tgcccagccc aggcctcggt cccagagatg 2640
   ccaggagcca tcg 2653
<210> 11
   <211> 1966
   <212> DNA
   <213> Artificial
<220>
   <223> Fragment 3
<400> 11
   cgaagcttga gattgtccaa gcaggtagcc agagagcgcc atcagccaag aaaccatcca     60
   ctggtacgta aggcagcctg tgcgggcgag accagactgg gccctcccct cctgcagtga    120
   tttgtttctt cttctttttt aaatcacgtt ttcctgcctt ttctaggttc taggtaccag    180
   cctctggctt ctacagcctc agacaatgac tttgtcacac cagagccccg ccgtactacc    240
   cgtcggcatc caaacaccca gcagcgagct tccaaaaaga aacccaaagt tgtcttctca    300
   agtgatgagt ccagtgagga aggtatgatg ctcccgcctg ttcccggccg agaaggcaca    360
   cagctagggt gcagagggct ggtttccata ggacctgctg cgggggcctg agtgtagatg    420
   ctctgcccca ctgccgcaga agggcctctc ctgtacagct tggattttat ttcttctgtg    480
   cggtgtggga ttgtctcact tgttctctga tatctatttt ttcaccatct ttgtgactca    540
   gctttttctt attcctttaa ttctttgcat agatctttca gcagagatga cagaagacga    600
   gacacccaag aaaacaactc ccattctcag agcatcggct cgcaggcaca gatcctagga    660
   agtctgttcc tgtcctccct gtgcagggta tcctgtaggg tgacctggaa ttcgaattct    720
   gtttcccttg taaaatattt gtctgtctct tttttttaaa aaaaaaaaag gccgggcact    780
   gtggctcacg cctgtaatcc cagcactttg cgataccaag gcgggtggat aacctgaggt 840
   agggagttcg agaccagcct gaccaacatg gagaaacccc atctctacta aaaataaaaa 900
   attagccggg cgtattggcg tgcgcctgta atcccagcta ctcaagaggc tgaggcagga 960
   gaatcgcctg aacccagagg cggaggttgt agtgagccga aatcacacca ttgcactcca 1020
   gcttgggcaa caatagcgaa cctccatctc aaattaaaaa aaaaatgcct acacgctctt 1080
   taaaatgcaa ggctttctct taaattagcc taactgaact gcgttgagct gcttcaactt 1140
   tggaatatat gtttgccaat ctccttgttt tctaatgaat aaatgttttt atatactttt 1200
   agacattttt tcctaagctt gtctttgttt catctttcac attagcccag tttcatgcag 1260
   cagagagagg gttatcagtg cagagagaga tgagtgagcc cagagtccta gggcctgtcc 1320
   cgggatggca gatgagcttc ctgccccgtc actgccacct ttcccctctc aacctctgga 1380
   ccctgcacag tgaccagaca gcctctctgg ggagaattat gcagtgccta ggctccagat 1440
   cagtgcttct gaaccggggg caattttgtc tgccagagga catctgacaa cacctggggc 1500
   ctgttttgtt gtcatagcct ataggggaag aatgctacca gcatttgtgg gaagaggcca 1560
   gggatgtggc tcaacatcct gcagtgcaca ggatggcccc tcaacaaaga atcacacggc 1620
   ccacaatgtc aatagcgtca cagttgagaa aacctgctct agaccaaggg ttgctttctg 1680
   ccgtgtgcct caccccaccc ccactcgtgt tccctaatcc catctccaaa ggttggcagc 1740
   agaccggccc aggctcgtgg aagttcagat catgatcccc tccagctctg caggagacaa 1800
   gacctgtctc ccagcattcc tcattgttcc cgggtctgca gagggcgtga gctatgctgc 1860
   aggcgggctg ccccctgaag cctgcgcacc cctctccagc tcctcaagtc ttctctgctg 1920
   agtcaccttc gaaccggagg ctgtgagctg gctgtcgtga ccacac 1966
<210> 12
   <211> 1198
   <212> DNA
   <213> Artificial
<220>
   <223> Fragment 4
<400> 12
   tggtgcctct gctgtcatga caacagcaca ctacgtcagt agtgctccct gggcactgag      60
   ctccctcttt gcggggagaa gacagtaatg aaaaatgaca agcatgaggc agaggggaag     120
   atcacgcttg ggtggtgcag gagcatggag gtgctcttaa tgctctcaat gagaaagggt     180
   taacggtcct ggttgcagga atagctgagt cagaggtggg gcttcctcca ctcccccacc     240
   ccaccccttt caccattagg gaccttcttg ccttgctctt gctactctgc tctgggtggt     300
   cattgtgaaa agcccgcacc aaccatgcca gtggcagcca gacgaggaca cagcctggct     360
   ctgggtccca gcaggaaagg caatcccaga aaggcagggt cagggactgg agtcctgtgg     420
   gtgcttttta agcaaagatt atcaccaggc aggctaaact tagcaaccgg cttttagcta     480
   gaagggcagg gggctggtgt caggttatgc tgggccagca aagaggcccg ggatccccct     540
   cccatgcacc tgctgatggg ccaaggccac cccaccccac ccccttcctt acaagtgttc   600
   agcaccctcc catcccacac tcacaaacct ggccctctgc cctcctacca gaagaatgga     660
   tcccctgtgg gagggggcag gggacctgtt cccaccgtgt gcccaagacc tcttttccca     720
   ctttttccct cttcttgact caccctgccc tcaatatccc ccggcgcagc cagtgaaagg     780
   gagtccctgg ctcctggctc gcctgcacgt cccagggcgg ggagggactt ccgccctcac     840
   gtcccgctct tcgccccagg ctggatggaa tgaaaggcac actgtctctc tccctaggca     900
   gcacagccca caggtttcca ggagtgcctt tgtgggaggc ctctgggccc ccaccagcca     960
   tcctgtcctc cgcctggggc cccagcccgg agagagccgc tggtgcacac agggccggga    1020
   ttgtctgccc taattatcag gtccaggcta cagggctgca ggacatcgtg accttccgtg    1080
   cagaaacctc cccctccccc tcaagccgcc tcccgagcct ccttcctctc caggccccca    1140
   gtgcccagtg cccagtgccc agcccaggcc tcggtcccag agatgccagg agccatcg      1198
<210> 13
   <211> 259
   <212> DNA
   <213> Artificial
<220>
   <223> Fragment 8
<400> 13
   cctctgggcc cccaccagcc atcctgtcct ccgcctgggg ccccagcccg gagagagccg      60
   ctggtgcaca cagggccggg attgtctgcc ctaattatca ggtccaggct acagggctgc     120
   aggacatcgt gaccttccgt gcagaaacct ccccctcccc ctcaagccgc ctcccgagcc     180
   tccttcctct ccaggccccc agtgcccagt gcccagtgcc cagcccaggc ctcggtccca     240
   gagatgccag gagccatcg                                                  259
<210> 14
   <211> 1947
   <212> DNA
   <213> Artificial
<220>
   <223> Fragment 9
<400> 14
   cgaagcttga gattgtccaa gcaggtagcc agagagcgcc atcagccaag aaaccatcca      60
   ctggtacgta aggcagcctg tgcgggcgag accagactgg gccctcccct cctgcagtga     120
   tttgtttctt cttctttttt aaatcacgtt ttcctgcctt ttctaggttc taggtaccag     180
   cctctggctt ctacagcctc agacaatgac tttgtcacac cagagccccg ccgtactacc 240
   cgtcggcatc caaacaccca gcagcgagct tccaaaaaga aacccaaagt tgtcttctca 300
   agtgatgagt ccagtgagga aggtatgatg ctcccgcctg ttcccggccg agaaggcaca 360
   cagctagggt gcagagggct ggtttccata ggacctgctg cgggggcctg agtgtagatg 420
   ctctgcccca ctgccgcaga agggcctctc ctgtacagct tggattttat ttcttctgtg 480
   cggtgtggga ttgtctcact tgttctctga tatctatttt ttcaccatct ttgtgactca 540
   gctttttctt attcctttaa ttctttgcat agatctttca gcagagatga cagaagacga 600
   gacacccaag aaaacaactc ccattctcag agcatcggct cgcaggcaca gatcctagga 660
   agtctgttcc tgtcctccct gtgcagggta tcctgtaggg tgacctggaa ttcgaaccgg 720
   aggctgtgag ctggctgtcg tgaccacact ggtgcctctg ctgtcatgac aacagcacac 780
   tacgtcagta gtgctccctg ggcactgagc tccctctttg cggggagaag acagtaatga 840
   aaaatgacaa gcatgaggca gaggggaaga tcacgcttgg gtggtgcagg agcatggagg 900
   tgctcttaat gctctcaatg agaaagggtt aacggtcctg gttgcaggaa tagctgagtc 960
   agaggtgggg cttcctccac tcccccaccc cacccctttc accattaggg accttcttgc 1020
   cttgctcttg ctactctgct ctgggtggtc attgtgaaaa gcccgcacca accatgccag 1080
   tggcagccag acgaggacac agcctggctc tgggtcccag caggaaaggc aatcccagaa 1140
   aggcagggtc agggactgga gtcctgtggg tgctttttaa gcaaagatta tcaccaggca 1200
   ggctaaactt agcaaccggc ttttagctag aagggcaggg ggctggtgtc aggttatgct 1260
   gggccagcaa agaggcccgg gatccccctc ccatgcacct gctgatgggc caaggccacc 1320
   ccaccccacc cccttcctta caagtgttca gcaccctccc atcccacact cacaaacctg 1380
   gccctctgcc ctcctaccag aagaatggat cccctgtggg agggggcagg ggacctgttc 1440
   ccaccgtgtg cccaagacct cttttcccac tttttccctc ttcttgactc accctgccct 1500
   caatatcccc cggcgcagcc agtgaaaggg agtccctggc tcctggctcg cctgcacgtc 1560
   ccagggcggg gagggacttc cgccctcacg tcccgctctt cgccccaggc tggatggaat 1620
   gaaaggcaca ctgtctctct ccctaggcag cacagcccac aggtttccag gagtgccttt 1680
   gtgggaggcc tctgggcccc caccagccat cctgtcctcc gcctggggcc ccagcccgga 1740
   gagagccgct ggtgcacaca gggccgggat tgtctgccct aattatcagg tccaggctac 1800
   agggctgcag gacatcgtga ccttccgtgc agaaacctcc ccctccccct caagccgcct 1860
   cccgagcctc cttcctctcc aggcccccag tgcccagtgc ccagtgccca gcccaggcct 1920
   cggtcccaga gatgccagga gccatcg 1947
<210> 15
   <211> 1436
   <212> **DNA**
   <213> Artificial
<220>
   <223> Fragment 11
<400> 15
   atctattttt tcaccatctt tgtgactcag ctttttctta ttcctttaat tctttgcata      60
   gatctttcag cagagatgac agaagacgag acacccaaga aaacaactcc cattctcaga     120
   gcatcggctc gcaggcacag atcctaggaa gtctgttcct gtcctccctg tgcagggtat     180
   cctgtagggt gacctggaat tcgaaccgga ggctgtgagc tggctgtcgt gaccacactg     240
   gtgcctctgc tgtcatgaca acagcacact acgtcagtag tgctccctgg gcactgagct     300
   ccctctttgc ggggagaaga cagtaatgaa aaatgacaag catgaggcag aggggaagat     360
   cacgcttggg tggtgcagga gcatggaggt gctcttaatg ctctcaatga gaaagggtta     420
   acggtcctgg ttgcaggaat agctgagtca gaggtggggc ttcctccact cccccacccc     480
   acccctttca ccattaggga ccttcttgcc ttgctcttgc tactctgctc tgggtggtca      540
   ttgtgaaaag cccgcaccaa ccatgccagt ggcagccaga cgaggacaca gcctggctct     600
   gggtcccagc aggaaaggca atcccagaaa ggcagggtca gggactggag tcctgtgggt     660
   gctttttaag caaagattat caccaggcag gctaaactta gcaaccggct tttagctaga     720
   agggcagggg gctggtgtca ggttatgctg ggccagcaaa gaggcccggg atccccctcc     780
   catgcacctg ctgatgggcc aaggccaccc caccccaccc ccttccttac aagtgttcag     840
   caccctccca tcccacactc acaaacctgg ccctctgccc tcctaccaga agaatggatc     900
   ccctgtggga gggggcaggg gacctgttcc caccgtgtgc ccaagacctc ttttcccact     960
   ttttccctct tcttgactca ccctgccctc aatatccccc ggcgcagcca gtgaaaggga    1020
   gtccctggct cctggctcgc ctgcacgtcc cagggcgggg agggacttcc gccctcacgt    1080
   cccgctcttc gccccaggct ggatggaatg aaaggcacac tgtctctctc cctaggcagc    1140
   acagcccaca ggtttccagg agtgcctttg tgggaggcct ctgggccccc accagccatc    1200
   ctgtcctccg cctggggccc cagcccggag agagccgctg gtgcacacag ggccgggatt    1260
   gtctgcccta attatcaggt ccaggctaca gggctgcagg acatcgtgac cttccgtgca    1320
   gaaacctccc cctccccctc aagccgcctc ccgagcctcc ttcctctcca ggcccccagt    1380
   gcccagtgcc cagtgcccag cccaggcctc ggtcccagag atgccaggag ccatcg        1436
<210> 16
   <211> 1177
   <212> DNA
   <213> Artificial
<220>
   <223> Fragment 17
<400> 16
   atctattttt tcaccatctt tgtgactcag ctttttctta ttcctttaat tctttgcata      60
   gatctttcag cagagatgac agaagacgag acacccaaga aaacaactcc cattctcaga     120
   gcatcggctc gcaggcacag atcctaggaa gtctgttcct gtcctccctg tgcagggtat     180
   cctgtagggt gacctggaat tcgaaccgga ggctgtgagc tggctgtcgt gaccacactg     240
   gtgcctctgc tgtcatgaca acagcacact acgtcagtag tgctccctgg gcactgagct     300
   ccctctttgc ggggagaaga cagtaatgaa aaatgacaag catgaggcag aggggaagat     360
   cacgcttggg tggtgcagga gcatggaggt gctcttaatg ctctcaatga gaaagggtta     420
   acggtcctgg ttgcaggaat agctgagtca gaggtggggc ttcctccact cccccacccc     480
   acccctttca ccattaggga ccttcttgcc ttgctcttgc tactctgctc tgggtggtca      540
   ttgtgaaaag cccgcaccaa ccatgccagt ggcagccaga cgaggacaca gcctggctct     600
   gggtcccagc aggaaaggca atcccagaaa ggcagggtca gggactggag tcctgtgggt     660
   gctttttaag caaagattat caccaggcag gctaaactta gcaaccggct tttagctaga     720
   agggcagggg gctggtgtca ggttatgctg ggccagcaaa gaggcccggg atccccctcc     780
   catgcacctg ctgatgggcc aaggccaccc caccccaccc ccttccttac aagtgttcag     840
   caccctccca tcccacactc acaaacctgg ccctctgccc tcctaccaga agaatggatc     900
   ccctgtggga gggggcaggg gacctgttcc caccgtgtgc ccaagacctc ttttcccact     960
   ttttccctct tcttgactca ccctgccctc aatatccccc ggcgcagcca gtgaaaggga    1020
   gtccctggct cctggctcgc ctgcacgtcc cagggcgggg agggacttcc gccctcacgt    1080
   cccgctcttc gccccaggct ggatggaatg aaaggcacac tgtctctctc cctaggcagc    1140
   acagcccaca ggtttccagg agtgcctttg tgggagg                             1177
<210> 17
   <211> 18106
   <212> **DNA**
   <213> Homo sapiens
<400> 17
   gtgagcagca caggacactt caatgcctgt tgggttctgg gctggctaag acatctgccg 60
   gccctgggca gcatacggct cttgcagtca ccttcccgtc ctccttatcc ccagctgggt 120
   tgcaaccaaa ttgccagagt gacctaagac cagatctttg tctccagttc tttttttatt 180
   actccaaaaa cacaaccaaa gcagcatctc atccaattct tgtttgtttg tttttaatag 240
   tttttatttt tcagagcagt tttaggttca aagcaaaatt gagcagaaag tacagggagt 300
   tcccttctac cccttgcccc tacacatcac agccttcccc accttcaaca tcctgcacca 360
   gggtggcaca tttgttacag ctgaacctac acttacacat catctcctaa agtcatggtt 420
   taccttggag ttcactgcac gtaatgacat gtacccacca ttgcagtatc atacagaaga 480
   gtttcactgc cttacaaatc ccctgcactc cacctattta tccctctctc cccacaaccc 540
   ctgatctttt tactgttgcc atcactttgt cttttccaga atgtatcatt ggaatgatcc 600
   ggtatggagc cttctcacct tggcttctta gtaatgtgcg tttaaggcct ccatgtcttc 660
   catggccttg tttcttttta atcagaagta actgttttca ggcctgctct gaatctcctt 720
   ttctccctcc aggctataat agatgaattt gagcagaagc ttcgggcctg tcataccaga 780
   ggtttggatg gaatcaagga gcttgagatt ggccaagcag gtagccagag agcgccatca 840
   gccaagaaac catccactgg tacgtaaggc agcctgtgcg ggcgagacca gactgggccc 900
   tcccctcctg cagtgatttg tttcttcttc ttttttaaat cacgttttcc tgccttttct 960
   aggttctagg taccagcctc tggcttctac agcctcagac aatgactttg tcacaccaga 1020
   gccccgccgt actacccgtc ggcatccaaa cacccagcag cgagcttcca aaaagaaacc 1080
   caaagttgtc ttctcaagtg atgagtccag tgaggaaggt atgatgctcc cgcctgttcc 1140
   cggccgagaa ggcacacagc tagggtgcag agggctggtt tccataggac ctgctgcggg 1200
   ggcctgagtg tagatgctct gccccactgc cgcagaaggg cctctcctgt acagcttgga 1260
   ttttatttct tctgtgcggt gtgggattgt ctcacttgtt ctctgatatc tattttttca 1320
   ccatctttgt gactcagctt tttcttattc ctttaattct ttgcatagat ctttcagcag 1380
   agatgacaga agacgagaca cccaagaaaa caactcccat tctcagagca tcggctcgca 1440
   ggcacagatc ctaggaagtc tgttcctgtc ctccctgtgc agggtatcct gtagggtgac 1500
   ctggaattcg aattctgttt cccttgtaaa atatttgtct gtctcttttt tttaaaaaaa 1560
   aaaaaggccg ggcactgtgg ctcacgcctg taatcccagc actttgcgat accaaggcgg 1620
   gtggataacc tgaggtaggg agttcgagac cagcctgacc aacatggaga aaccccatct 1680
   ctactaaaaa taaaaaatta gccgggcgta ttggcgtgcg cctgtaatcc cagctactca 1740
   agaggctgag gcaggagaat cgcctgaacc cagaggcgga ggttgtagtg agccgaaatc 1800
   acaccattgc actccagctt gggcaacaat agcgaacctc catctcaaat taaaaaaaaa 1860
   atgcctacac gctctttaaa atgcaaggct ttctcttaaa ttagcctaac tgaactgcgt 1920
   tgagctgctt caactttgga atatatgttt gccaatctcc ttgttttcta atgaataaat 1980
   gtttttatat acttttagac attttttcct aagcttgtct ttgtttcatc tttcacatta 2040
   gcccagtttc atgcagcaga gagagggtta tcagtgcaga gagagatgag tgagcccaga 2100
   gtcctagggc ctgtcccggg atggcagatg agcttcctgc cccgtcactg ccacctttcc 2160
   cctctcaacc tctggaccct gcacagtgac cagacagcct ctctggggag aattatgcag 2220
   tgcctaggct ccagatcagt gcttctgaac cgggggcaat tttgtctgcc agaggacatc 2280
   tgacaacacc tggggcctgt tttgttgtca tagcctatag gggaagaatg ctaccagcat 2340
   ttgtgggaag aggccaggga tgtggctcaa catcctgcag tgcacaggat ggcccctcaa 2400
   caaagaatca cacggcccac aatgtcaata gcgtcacagt tgagaaaacc tgctctagac 2460
   caagggttgc tttctgccgt gtgcctcacc ccacccccac tcgtgttccc taatcccatc 2520
   tccaaaggtt ggcagcagac cggcccaggc tcgtggaagt tcagatcatg atcccctcca 2580
   gctctgcagg agacaagacc tgtctcccag cattcctcat tgttcccggg tctgcagagg 2640
   gcgtgagcta tgctgcaggc gggctgcccc ctgaagcctg cgcacccctc tccagctcct 2700
   caagtcttct ctgctgagtc accttcgaac cggaggctgt gagctggctg tcgtgaccac 2760
   actggtgcct ctgctgtcat gacaacagca cactacgtca gtagtgctcc ctgggcactg 2820
   agctccctct ttgcggggag aagacagtaa tgaaaaatga caagcatgag gcagagggga 2880
   agatcacgct tgggtggtgc aggagcatgg aggtgctctt aatgctctca atgagaaagg 2940
   gttaacggtc ctggttgcag gaatagctga gtcagaggtg gggcttcctc cactccccca 3000
   ccccacccct ttcaccatta gggaccttct tgccttgctc ttgctactct gctctgggtg 3060
   gtcattgtga aaagcccgca ccaaccatgc cagtggcagc cagacgagga cacagcctgg 3120
   ctctgggtcc cagcaggaaa ggcaatccca gaaaggcagg gtcagggact ggagtcctgt 3180
   gggtgctttt taagcaaaga ttatcaccag gcaggctaaa cttagcaacc ggcttttagc 3240
   tagaagggca gggggctggt gtcaggttat gctgggccag caaagaggcc cgggatcccc 3300
   ctcccatgca cctgctgatg ggccaaggcc accccacccc acccccttcc ttacaagtgt 3360
   tcagcaccct cccatcccac actcacaaac ctggccctct gccctcctac cagaagaatg 3420
   gatcccctgt gggagggggc aggggacctg ttcccaccgt gtgcccaaga cctcttttcc 3480
   cactttttcc ctcttcttga ctcaccctgc cctcaatatc ccccggcgca gccagtgaaa 3540
   gggagtccct ggctcctggc tcgcctgcac gtcccagggc ggggagggac ttccgccctc 3600
   acgtcccgct cttcgcccca ggctggatgg aatgaaaggc acactgtctc tctccctagg 3660
   cagcacagcc cacaggtttc caggagtgcc tttgtgggag gcctctgggc ccccaccagc 3720
   catcctgtcc tccgcctggg gccccagccc ggagagagcc gctggtgcac acagggccgg 3780
   gattgtctgc cctaattatc aggtccaggc tacagggctg caggacatcg tgaccttccg 3840
   tgcagaaacc tccccctccc cctcaagccg cctcccgagc ctccttcctc tccaggcccc 3900
   cagtgcccag tgcccagtgc ccagcccagg cctcggtccc agagatgcca ggagccagga 3960
   gatggggagg gggaagtggg ggctgggaag gaaccacggg cccccgcccg aggcccatgg 4020
   gcccctccta ggcctttgcc tgagcagtcc ggtgtcacta ccgcagagcc tcgaggagaa 4080
   gttccccaac tttcccgcct ctcagccttt gaaagaaaga aaggggaggg ggcaggccgc 4140
   gtgcagccgc gagcggtgct gggctccggc tccaattccc catctcagtc gttcccaaag 4200
   tcctcctgtt tcatccaagc gtgtaagggt ccccgtcctt gactccctag tgtcctgctg 4260
   cccacagtcc agtcctggga accagcaccg atcacctccc atcgggccaa tctcagtccc 4320
   ttccccccta cgtcggggcc cacacgctcg gtgcgtgccc agttgaacca ggcggctgcg 4380
   gaaaaaaaaa agcggggaga aagtagggcc cggctactag cggttttacg ggcgcacgta 4440
   gctcaggcct caagaccttg ggctgggact ggctgagcct ggcgggaggc ggggtccgag 4500
   tcaccgcctg ccgccgcgcc cccggtttct ataaattgag cccgcagcct cccgcttcgc 4560
   tctctgctcc tcctgttcga cagtcagccg catcttcttt tgcgtcgcca ggtgaagacg 4620
   ggcggagaga aacccgggag gctagggacg gcctgaaggc ggcaggggcg ggcgcaggcc 4680
   ggatgtgttc gcgccgctgc ggggtgggcc cgggcggcct ccgcattgca ggggcgggcg 4740
   gaggacgtga tgcggcgcgg gctgggcatg gaggcctggt gggggagggg aggggaggcg 4800
   tgtgtgtcgg ccggggccac taggcgctca ctgttctctc cctccgcgca gccgagccac 4860
   atcgctcaga caccatgggg aaggtgaagg tcggagtcaa cgggtgagtt cgcgggtggc 4920
   tggggggccc tgggctgcga ccgcccccga accgcgtcta cgagccttgc gggctccggg 4980
   tctttgcagt cgtatggggg cagggtagct gttccccgca aggagagctc aaggtcagcg 5040
   ctcggacctg gcggagcccc gcacccaggc tgtggcgccc tgtgcagctc cgcccttgcg 5100
   gcgccatctg cccggagcct ccttccccta gtccccagaa acaggaggtc cctactcccg 5160
   cccgagatcc cgacccggac ccctaggtgg gggacgcttt ctttcctttc gcgctctgcg 5220
   gggtcacgtg tcgcagagga gcccctcccc cacggcctcc ggcaccgcag gccccgggat 5280
   gctagtgcgc agcgggtgca tccctgtccg gatgctgcgc ctgcggtaga gcggccgcca 5340
   tgttgcaacc gggaaggaaa tgaatgggca gccgttagga aagcctgccg gtgactaacc 5400
   ctgcgctcct gcctcgatgg gtggagtcgc gtgtggcggg gaagtcaggt ggagcgaggc 5460
   tagctggccc gatttctcct ccgggtgatg cttttcctag attattctct ggtaaatcaa 5520
   agaagtgggt ttatggaggt cctcttgtgt cccctccccg cagaggtgtg gtggctgtgg 5580
   catggtgcca agccgggaga agctgagtca tgggtagttg gaaaaggaca tttccaccgc 5640
   aaaatggccc ctctggtggt ggccccttcc tgcagcgccg gctcacctca cggccccgcc 5700
   cttcccctgc cagcctagcg ttgacccgac cccaaaggcc aggctgtaaa tgtcaccggg 5760
   aggattgggt gtctgggcgc ctcggggaac ctgcccttct ccccattccg tcttccggaa 5820
   accagatctc ccaccgcacc ctggtctgag gttaaatata gctgctgacc tttctgtagc 5880
   tgggggcctg ggctggggct ctctcccatc ccttctcccc acacacatgc acttacctgt 5940
   gctcccactc ctgatttctg gaaaagagct aggaaggaca ggcaacttgg caaatcaaag 6000
   ccctgggact agggggttaa aatacagctt cccctcttcc cacccgcccc agtctctgtc 6060
   ccttttgtag gagggactta gagaaggggt gggcttgccc tgtccagtta atttctgacc 6120
   tttactcctg ccctttgagt ttgatgatgc tgagtgtaca agcgttttct ccctaaaggg 6180
   tgcagctgag ctaggcagca gcaagcattc ctggggtggc atagtggggt ggtgaatacc 6240
   atgtacaaag cttgtgccca gactgtgggt ggcagtgccc cacatggccg cttctcctgg 6300
   aagggcttcg tatgactggg ggtgttgggc agccctggag ccttcagttg cagccatgcc 6360
   ttaagccagg ccagcctggc agggaagctc aagggagata aaattcaacc tcttgggccc 6420
   tcctgggggt aaggagatgc tgcattcgcc ctcttaatgg ggaggtggcc tagggctgct 6480
   cacatattct ggaggagcct cccctcctca tgccttcttg cctcttgtct cttagatttg 6540
   gtcgtattgg gcgcctggtc accagggctg cttttaactc tggtaaagtg gatattgttg 6600
   ccatcaatga ccccttcatt gacctcaact acatggtgag tgctacatgg tgagccccaa 6660
   agctggtgtg ggaggagcca cctggctgat gggcagcccc ttcataccct cacgtattcc 6720
   cccaggttta catgttccaa tatgattcca cccatggcaa attccatggc accgtcaagg 6780
   ctgagaacgg gaagcttgtc atcaatggaa atcccatcac catcttccag gagtgagtgg 6840
   aagacagaat ggaagaaatg tgctttgggg aggcaactag gatggtgtgg ctcccttggg 6900
   tatatggtaa ccttgtgtcc ctcaatatgg tcctgtcccc atctcccccc cacccccata 6960
   ggcgagatcc ctccaaaatc aagtggggcg atgctggcgc tgagtacgtc gtggagtcca 7020
   ctggcgtctt caccaccatg gagaaggctg gggtgagtgc aggagggccc gcgggagggg 7080
   aagctgactc agccctgcaa aggcaggacc cgggttcata actgtctgct tctctgctgt 7140
   aggctcattt gcagggggga gccaaaaggg tcatcatctc tgccccctct gctgatgccc 7200
   ccatgttcgt catgggtgtg aaccatgaga agtatgacaa cagcctcaag atcatcaggt 7260
   gaggaaggca gggcccgtgg agaagcggcc agcctggcac cctatggaca cgctcccctg 7320
   acttgcgccc cgctccctct ttctttgcag caatgcctcc tgcaccacca actgcttagc 7380
   acccctggcc aaggtcatcc atgacaactt tggtatcgtg gaaggactca tggtatgaga 7440
   gctggggaat gggactgagg ctcccacctt tctcatccaa gactggctcc tccctgccgg 7500
   ggctgcgtgc aaccctgggg ttgggggttc tggggactgg ctttcccata atttcctttc 7560
   aaggtgggga gggaggtaga ggggtgatgt ggggagtacg ctgcagggcc tcactccttt 7620
   tgcagaccac agtccatgcc atcactgcca cccagaagac tgtggatggc ccctccggga 7680
   aactgtggcg tgatggccgc ggggctctcc agaacatcat ccctgcctct actggcgctg 7740
   ccaaggctgt gggcaaggtc atccctgagc tgaacgggaa gctcactggc atggccttcc 7800
   gtgtccccac tgccaacgtg tcagtggtgg acctgacctg ccgtctagaa aaacctgcca 7860
   aatatgatga catcaagaag gtggtgaagc aggcgtcgga gggccccctc aagggcatcc 7920
   tgggctacac tgagcaccag gtggtctcct ctgacttcaa cagcgacacc cactcctcca 7980
   cctttgacgc tggggctggc attgccctca acgaccactt tgtcaagctc atttcctggt 8040
   atgtggctgg ggccagagac tggctcttaa aaagtgcagg gtctggcgcc ctctggtggc 8100
   tggctcagaa aaagggccct gacaactctt ttcatcttct aggtatgaca acgaatttgg 8160
   ctacagcaac agggtggtgg acctcatggc ccacatggcc tccaaggagt aagacccctg 8220
   gaccaccagc cccagcaaga gcacaagagg aagagagaga ccctcactgc tggggagtcc 8280
   ctgccacact cagtccccca ccacactgaa tctcccctcc tcacagttgc catgtagacc 8340
   ccttgaagag gggaggggcc tagggagccg caccttgtca tgtaccatca ataaagtacc 8400
   ctgtgctcaa ccagttactt gtcctgtctt attctagggt ctggggcaga ggggagggaa 8460
   gctgggcttg tgtcaaggtg agacattctt gctggggagg gacctggtat gttctcctca 8520
   gactgagggt agggcctcca aacagccttg cttgcttcga gaaccatttg cttcccgctc 8580
   agacgtcttg agtgctacag gaagctggca ccactacttc agagaacaag gccttttcct 8640
   ctcctcgctc cagtcctagg ctatctgctg ttggccaaac atggaagaag ctattctgtg 8700
   ggcagcccca gggaggctga caggtggagg aagtcagggc tcgcactggg ctctgacgct 8760
   gactggttag tggagctcag cctggagctg agctgcagcg ggcaattcca gcttggcctc 8820
   cgcagctgtg aggtcttgag cacgtgctct attgctttct gtgccctcgt gtcttatctg 8880
   aggacatcgt ggccagcccc taaggtcttc aagcaggatt catctaggta aaccaagtac 8940
   ctaaaaccat gcccaaggcg gtaaggacta tataatgttt aaaaatcggt aaaaatgccc 9000
   acctcgcata gttttgagga agatgaactg agatgtgtca gggtgactta tttccatcat 9060
   cgtccttagg ggaacttggg taggggcaag gcgtgtagct gggacctagg tccagacccc 9120
   tggctctgcc actgaacggc tcagttgctt tgggcagtta ctcccgggcc tcactttgca 9180
   cgtgtgctta cctagtggag acaaaagtac atacctcggt agagcgcgca cgcctgtaac 9240
   cccagcactt tgggaggcca aggtgggtgt atcacctgag gtcaggagtt tgagaccagc 9300
   ctggccaaca tggtgaaact ccgtctctac taaaattaca aaaatcagcc aggcttcatg 9360
   gcacatgcct atagtcccag ctacaggcat gctgaagcag gagaatcgct tgcaccccgg 9420
   aggcagaggc tgcagtgagc tgagaccaca ccactgcact ccagcctagg caacagagta 9480
   tgagactcca tctcaaaaaa aaaaaaagta cctacctcag agttcaaact agtgaatatt 9540
   aggaagtgct tgagacagtg acaccaaagt gcacaataaa tactcgccag tttcattatt 9600
   attaaagaat ccatttgaat gtcagctcaa cacagcctcc tataccgagg cattgtgaac 9660
   cgcatctccc cagcttctcc aggcttttcc aagaatcagg gacactgtag cctgttggtc 9720
   tcagtgtatg acagacacgg aggaagcaca tctttagctg atacttaaac agagaccctg 9780
   agcgcacata cacccgcgca cacatgcatg gagcttcacc ttctctgtca ttctgcagtg 9840
   accaggagag caagagctcc cacctccctt caaaacactg tgcccatccc gggcactaag 9900
   gcctctttaa agcacggcac ctccacgagg gagggccaca gccacataca ctccacctgg 9960
   caggtggaca gcgtgagcac gtggaccata gcagggacaa ggtgccccgg ccagccccaa 10020
   cgccctctgc cgctgacagg gacagaagcc ctctccagct gcgtgtgctg cagaggccat 10080
   gcgtagcctc cagctgcatt ctattccact ccagtgcctg ggccagttag caccagtgtg 10140
   gaagacagtg agctggctcc ggacaacagg gatggaggaa aggtcccaca ttcacattcc 10200
   tgatacgtgg acaaggtgag gggccgcaat cgctctggca gcattttaaa gatggggaag 10260
   tagcagacac ccacgcgtga aggcaggaga gccccaactg tggtggaaat ggccccagaa 10320
   tggtagggcc aagcctagct ccagacaccc cagagccctg gagaagccaa gactgaggga 10380
   gaaagcctga gggaggagcg ccccagtccc cagggaccgg cctggtgcag agctgcagct 10440
   gatgttcccc tctgtgcagc cccaccctct gcctcgctga gctccctgct gcgagggcct 10500
   cgggtgcaag ggggaggcag gtctctatct catggagctg tcagatgaga catcgcgatc 10560
   ggagtcctca gcctcgcttg gcggcggcgg cgggtcgcta agcgggaccg cagtgaaagc 10620
   aggagacttt ctagaaaaaa acaccagttg tcaaccttgg ggcaggcagg aatcctgaag 10680
   acggacggca ctcctcctcc tgctgcctca ccctctggca gcccgtgaga agtaccggaa 10740
   gcgagggcgg ggccgcggga tggcgaggga gcggcaggga ctgaactctc tccaaaccca 10800
   ccctgacagg gaaatgggcc ccgcctgtgt cttgggaact cagaggctga ggtcaggcat 10860
   gatggctcac gcctgtaatt ccagcacttt gggaggcaga ggcgggtgga tcacgacgtc 10920
   aggagttcaa ggcaagcctg gccaacatgg tgaaacccca tctctactaa aaatgcaaaa 10980
   attagccagg tgtggtggcg ggcgccttgg aggctgaggc aagataatca cttgaacctg 11040
   ggaggcggag gttgtagtga gccaaaaaaa aaaaaaaaag aaatagctga agtcacagta 11100
   ggagagaagc tgctgagcct ccagcaccct gactctaggg ccttggcttt atgtctatct 11160
   gcagtatttt tgtgattttt aaaaattcac tttcttgttg cggtgtaact tacacagggt 11220
   caaatgcaca aatcatggcc ctgactttag ataaaaatct gcccccacaa cccttctgtt 11280
   ccttgccagt ttttaaactg cctctaacca ggggaaccac cagagctggt ggccttggga 11340
   ggtttcagcc ctcccgtcat gaatggacat agctcatcca actgccaagg gagagagctg 11400
   tgggtctggg ccagccccac caggtaactc ccaaagggca gccccacagc aagatgtgac 11460
   ccagtcattg cctgagggtc tctggggctg tgttccaacc tttctccccg ctgtgtcccc 11520
   ctggaaggcc ccatgcccag gggaggcgcc taccggtctc cagactgggt gatgagccgg 11580
   cggcaggtct ccatctgcac gtccaggccg cgcttcatgc tgcacatctc catgtactcg 11640
   tgcaggtgcc ggttcatgtc gttcttggcc gtggccaact ccagctgtgg tggggcaggc 11700
   agggccatcg gggttaacag gtggcgttca cagcgcctct gttgcccccg ccaggaggcc 11760
   aacacgccaa gagcagtggc tgggccgggg gcccaggcag ccattactga aggctcagat 11820
   tttaaaataa accagaccaa ggcagaaggc agcgaataca ccattgtttc ctctgctttt 11880
   cctggaaatc tttgcctatg gaggaatgca aaggtactgc tgcaggctgt gttcttttaa 11940
   gactgataag agtggtgagg aaagtcagtg tgggcgggat ggagcagatc cacaggctcc 12000
   cagggccaca caggttccta tggggcccgg ctcacccctc atcccctgta ctgcgctggt 12060
   cctgggctgt cccctcactg cactgtgagc tcccgtaagg gttagcgaca ggttttcctc 12120
   atcctggcag acccagacct cgaagggaat taactctctc tgtgacccgt ggcatacaag 12180
   cctggctaac tgctgaaagg ggcccaatcc tcttaccatt ctctccttat cactttattt 12240
   tctcacctaa aagccatttt cagacactaa aaggaagact cccatattgg aggtgccatt 12300
   gattctgaga cctgtcctga tttcagaaat gtcacacgtg gaaaacacgg ggctcagaat 12360
   caaagaaatc cggtctccct ccccgggccc attctgcagc ccacactctg aggcaggcgg 12420
   agaggcaggg cccaggcagc acaaactcag tgccagggac agtcgactgc ctctctttgt 12480
   tttgggtaca aagggcaggg gccgcaaggg gaagcaaaat gaaacttcct ggagatgacg 12540
   ttcgtgactg cctgagtgaa gagaacgggg aggataccag ctgccataat gtggattgga 12600
   cagagagggt gggaacttag agaaagaagg aaacaggtta cagggccaga cagcagagat 12660
   ggtcaggaca gtgacatttg gggtgagatg gcggctggct gggtgagaag ccctagtggg 12720
   gacagcttcc ctggcaggct tggatccaca gctggggccc cacagtagtg ggaggagcag 12780
   gacacggtga ctgttcaacc cagggggaaa atacagtgcc accagccctt gttcagatca 12840
   gctgcctaca ccccaatttc tccaccttgg aagaaccgga gtgcagggag ctgggttctg 12900
   gaaccatcac tcagggagac tgggaagaag gaaatgaacc aaatatattc tactgaaata 12960
   caaggctagt actgccacca gtagtctctg catatgtcta tgtccccact taattcacaa 13020
   atttacagca ggaacaaggt caggaggtga acgtgcatgt caggagatgt ccttgacaag 13080
   ccaccttgaa aactgcaggc tcgctgtgtg aacagacttg ggggtgggac gggaggagac 13140
   agagagaggt gaggagcggt ggggaagaac tgcctcccgc ccaccactct gggccttggc 13200
   tttaccttgc tccatcttag tgaccctgag agatcaaagg tcacaaagat aaaggcacct 13260
   tgggtactcc tgaggtcagc aagtcctcct aacggttccc ctaatgcaga agtccccaaa 13320
   ccccaggcca cggaccagta cctgtctgtg gcctgttagg aaccaggcca aatagcagga 13380
   ggtgagcagt gggcaagtga gtgaagcttc atctgtattt acagctgctc cccatcactg 13440
   gcattacagc ctgagctcca cctcctgtca gatccgcggc ggcattagat tctcacagga 13500
   tcctgaaccc taccttacgt gaactgtgca tgcgagggat ctaggctgtg tgctccttat 13560
   gagaatctaa tgcctgatga tctgtcacta tctcccatca cccccagatg ggaccgtcta 13620
   gttgcggaaa acaagttcat ggctcccact gattctacat tatggtgaga tgtataatta 13680
   tttcattata tattataatg taataataat agaaataaag tgcacaggcc gggtgcggtg 13740
   gctcatgcct gtaatcccag cactttggga ggccgaggtg gtggatcacc tgaggtcggg 13800
   agttcgagac cagcttgacc aacatggaga aaccccatct ctactaaaaa tacaaaatta 13860
   gccaggcgtg gtggcacatg cccgtaatcc cagctacttg ggaggctgag gcaggagaat 13920
   tgcttgagcc cgggaggcgg aggctgcagt gagccaagat tgcgccattg cactccagcc 13980
   tgggcaacaa gagcaaaact ctgtctctaa aaataataat aataattttt aaaaatagta 14040
   ataaagtaca caataaatgt aatgtttgaa tcatctcaaa atcatttccc ccgggtcagt 14100
   ggaaaaactg tcttccacaa accagtccct ggtaccaaaa aggttgggga ctgctgccta 14160
   aagtcattgc actgggagag aggaggagtc agatgatttg aaaatacttt tttttttttt 14220
   tttcctgaga tggaatcttg ctctgtcacc caggctgtgg tgcagtggca caatctcagc 14280
   tcactgcagc ctccacctcc cagattccag caattctcct gcctcagcct cccaagtagc 14340
   tgggattaca ggtgcccacc acaatgcctg gctacttttt gtatttttag tagagacggg 14400
   gtttcacatg ttggtggcca ggctggtctc acactcctga cctcaagtga tctgcccgcg 14460
   ttggcctccc aaagtgctgg gattacaggc gagagccacc acgcccagcc aaaaatagct 14520
   taaaaggcat gattccattt aagatattat tttcgttctt tcagatcatg ttttacactg 14580
   ggcacttgag gtccagggaa gactagggag agatggcagc ccgagatctc ttctcacgaa 14640
   gatggccgca caaaggcccc tagttggttg aaggcacagt gaaaacacaa accttaaatc 14700
   caccatccaa aagagggaga caagaatcag gtcaggcagg aggaggaaac agtgagccgc 14760
   ggtagacggg agggtgggga gtactggttt cccttgccat gtctttagct agcagcacaa 14820
   atccaggaaa cctcatctac ctggccaaga aaaccaggtt tgatgcagga gcaggacagt 14880
   ttgatgtgtg ttgggagaat gagtatatag gaaaataatt tctcgacttc cttctcaaac 14940
   acctccaatg tgacagcaat aagaataaag tcaaggccgg gcgcggtggc tcacgcctgt 15000
   aatcccagca ctttggaagg ccgaggcggg tggatcacga ggtcaggaga tcgagaccat 15060
   cctggctaac atggtgaaac cccgtctcta ctaaaaaata caaaaaatta gccgggcgta 15120
   gtggcaggcg cctgtagtcc cagctactct ggaggctgag gcaggagaat ggcgtgaacc 15180
   tgggaggtgg agcttgcagt gagctgagat cgcgccactg cactccagcc tgggcgacac 15240
   agcgagacta cgtctcaaaa aaataaataa ataaataaat aaatgaaaag aataaagtca 15300
   ctctctcctt atccactggt ttcactttcc acggcttcag gtacccaagg tgaaccttga 15360
   tctgaaaata ttaaatgaaa tattccagaa ataaacaatt cctaagtttt aaattgcacg 15420
   ctgttctgag tggcctgata aaatctcact ctctctgccg ggcgcggtgg ctcacgccta 15480
   taatcccagc actttgggag gccgaggtgg gcggatcatg aggtcaggag atcgagatca 15540
   tcctggctaa cacggtgaaa ccccatctct actaaaaaat acaaaagaaa ttagccgggc 15600
   gtgatggcgg gcgcctgtag tcccagctac tcaggaggct gaggcaggag aatggcgtga 15660
   acccaggagg tggagcttgc agtgagccga gttcaagcca ctgcactcca gcctgggtga 15720
   cacagcgaga ctccgtctcg gaaaaaaaaa aaaaaacctc actctatcca gaccaggaca 15780
   tgaaccctcc ctctgtccca aggatccaca ctgtctatac tgcctgccca ttagttactt 15840
   aggagccatc tcagtgatga gattgactgt cgtggtatgg cagtgcccgt gttcaaggaa 15900
   cccttatttt tacttcataa tggccccaaa gcacaagagt agtgatgctg gcaatttgga 15960
   tatgtgaaag agaagccaaa atatgctccc tttaagtgaa aaggtaaaag ttttcaactt 16020
   aataagaaaa agaatccgat gctgaggttg gtaagctcta cagtaagaat gaatcttcta 16080
   tccgtgaaat tgtaaagaag gaaaaagaaa tttgtgccag ttttgctgtc acacctcgaa 16140
   ctgcaaaagg taccgccaca gcgcacgcta agtgcttagt taagatagaa aaggcattga 16200
   atgtgtgggt gggagacatg aacagaaaca cgttccagtt gacagcaact ggacccaagc 16260
   tatcaggagg tcagtgctat ctttggtttc aggcatccac tgtgggtctt ggaacacatg 16320
   ccctgaagat aaggggggac tacgtatgaa tctcccatgt gggcctgaag agaggctgag 16380
   acgcagagaa ggctccatgc ctctcccaaa gtcagagcgt cgggaaagga ccacctcacc 16440
   aggccaggag agctcacggg ggtcccgtcc atccctcctc ccacagcaac agcaacctcc 16500
   attctgtggt tccacaaatc cttcttcctt tgtttgtatt tttgagacgg agtcttgctc 16560
   tgtcgcccag gctggagtgc agtggtgcga tctcggctca ctgcaagctc cgcctcccgg 16620
   gttcacgcca ttctcctgcc tcagcctccc gagtagctgg gactacaggt gcctgccacc 16680
   acggccagct aatttttttt ttttgtattt ttcgtagaga cggggtttca ccgtgttagc 16740
   caggatggtc tcgatctcct gacctcgtga tcttcccgcc tcggcctccc aaagtgctgg 16800
   gattacaggc gtgagccacc gcgcccggcc tccacaaatc cttcttgcag ctcctaaagc 16860
   ctagagtttg agctaaagca ccagctccac ttgcctctag gtacctctag aaggtacttc 16920
   aaggcttggg aaaaagcatg gagatttctc tgttatctca aaggctgagt ggaagaaaca 16980
   ggaaagtccc agtccatctc tgagtgccat ggcactgata gtgttaataa taataaaaca 17040
   gtccccacta tgctacactc gctatgagcc agacactgcc ctaagctcta tatatgcatt 17100
   atttcatctg atcttctaaa caaccctata ggtgaggtat tattaaattc ctcattttac 17160
   aaataaaaag gcccggcaca gtggctcatg cctgtaatcc caacactttg tggggccgag 17220
   ttaggaggac tgcttgaatc cagaagtttg agggcagcct ggaaaacata gtgacaccct 17280
   gttctctgta aaaaaaatca aaaaattagc cgggcgtggt agcacacgcc tgtggtccca 17340
   gctacttggg aggctgaggt aggaggattg cttggatcca gaagatcacg gctgcagtgg 17400
   gcctggagtg acagagcaag accctgtctc aaaaaaagaa agaaagaaag aaagaaagaa 17460
   aaagaaacag gtgtagggct ggacacatgg ctcatgtctc taattctagt actttgggag 17520
   gccgaggcag gaggatcact taagcccaag agtttaagac ctgcctggga aacacaggga 17580
   gacaccatct ctacaaaaaa atacaaataa aaattagctg ggtatggtgg cacacacctg 17640
   tgggcccagc tactcagaag gctggggtgg gaggatcact ccagcctagg caacagagtg 17700
   aaacagagtg agaccttgtc tcaaaaaaga aaggacagaa agacaaggga gagagggagg 17760
   gagggataga gggggagggg aagagagaga gagagagaaa ggaaggaagg aaggaaggaa 17820
   agaaaggaaa ggaaaggaaa ggaaaggagg ggctcagagg gaacaagtaa cttattacaa 17880
   gttcagggtg gttgtaagta acagagctga gatttgaacc gatatctgtc tgtttccaga 17940
   gcctgaggtc ttcagagccc accaggagga ctatagctga gagatcactc ctcctcccaa 18000
   gaccttggtc tctagggtcc tgcagcaggg atgaggccac tgcgcctgca gccccactca 18060
   aaaccctctg ggacaccacg cccctggctt ccgctcctgc ccttac 18106
<210> 18
   <211> 16312
   <212> DNA
   <213> Mus sp.
<400> 18
   gtgagtggtc ctgttaggtt ccggtgcagt aaacttcatc tgcctcccta agtgctctga 60
   gactcctgtc tgaactttct gccttgctag cctcactaca gctatggaac cagagtgact 120
   aagaccagat cttaattcct ggtccctttt ttaaaggcca ttcatttcct tttttgttaa 180
   catgcattgg tattttgcct gtgtatatgt ttgcatgaag gtgccaagtc cccttggaat 240
   tggagttaga ctgttgtggg ccctgagaac tgaacacagg tcttttgtaa gagcagccag 300
   tgctcctaac cactatgcca ccttcccagc cccagtgaag gccttgagat agggtcttgc 360
   tatgtagacc cagctgcctc agactaacag agattcccct gcctctgtct cctggtagta 420
   gaggtatgcc gcacatgttt gcactggact gcttctttag agtagtttca tgtttaaagc 480
   aaaagtcagt aagtagagct cccatatgct ctgtcccaca cccgcatggc ctccacccca 540
   tgaccttctt gtcctggtat acgtggtcct ggatgaagga acgtgattga cagatcagca 600
   tccaaagtcc actcactgga cacagtggca catacttacc acttgtgtca tactgggcat 660
   tttcaggact ttgaggtcac cttacccccc acccctgtgg tggcatcagt tagttggaat 720
   cacagtttca gattggtttc tttagttttc tctcttttca tggctatttc tttttaagca 780
   gggagataat gtttccagcc ctgtctttaa ctttctgtcc tccaggctat aatagatgaa 840
   tttgagcaga agcttcgtgc ctgtcacacc agaggcatgg atggaataga ggagtttgaa 900
   actggccagg gaggcagcca gcgagccctg tctgccaaga aaccctccgc tggtatgaga 960
   gggatgcaag cccacatggg gctcctatga gtggtcccca cccttttttt gttcctttgt 1020
   ggtttgtttt tggttttgaa atggtcccct tgcctttctt ttctagtctc aaggctacag 1080
   cctctgactt ctgtagactc agacaatgac tttgtcacac ctaagccgcg acgtaccaaa 1140
   cctggtcgtc cacaaactca gcagcgcaag aagtcccaga ggaaagccaa agttgtcttc 1200
   ttgagtgacg agtccagtga ggacggtatg atacaccctc ccaacagatc agctgcctgt 1260
   gtggggtctt ccagtggggc ttgcctagct gtggattcat tcttcctggc catgtcttcc 1320
   tcactatgca gctcggctga ctttcacagt gtgccgtgtg cgtgtgtgtt tgtgtgtgtg 1380
   tttgtgtgtg tgtgtgcaca cgtgtgcgcg tgcgcacgcg cgcatgtgtt tactttgaga 1440
   tccattttgc cccatcttta tgactcagct tttgttccta taattctttg aatagaactt 1500
   tcagcagaga tgacagaaga agagacaccc aagagaacca cccccatccg cagagcatct 1560
   gggcgaagac acaggtccta ggagctgcct ttgcctactc ccgtccctgc cgtgcagggt 1620
   atcttactgg atgatcttgg attcagtgcc ccccccccct tgtagaagtg cttgttgtct 1680
   gtggttttaa catggaaatg gcctttgcat tgaggtcttt ctcttagagc cctgctgcca 1740
   ctttcccttt ctccgagcct gtgccaattg ctgttttcta atgaataaat gtttttatag 1800
   acttttaagc atcctgtcct ggctggtctg tcccatcctt agccctgagc tgtgtctgga 1860
   tcttcatggc ccctgtggtg cctcatcctt gcacaactga aactctgctg tgcagaatta 1920
   tgcggtttct aggttcacgt gaacagttct cagcagtctg gggtactttg caccccagaa 1980
   aacattcggt aatacctaga gacttatttc tgtttgcctt gtggggcagg gggctgcagt 2040
   gaaagcctta agatgaaggc caccttcatg cacattgtgc cccataacac agcatgtcag 2100
   gagtacacaa cttgagaaat gctgctctag acctaaggtt tgtttcctct gtgcgcccca 2160
   ccccatcccc cgaagactgg caaatgactc agcctggcta atggcagaga ttatgaaccc 2220
   cactcagttc tataggtgac aaggtccatc gccccagagc cagtccttca ctacactaag 2280
   gggtctgcag gaggtccagc tgtaagccat gctgtgtgcc caccatcccc aagcccttaa 2340
   tctgctgagt cacttggagc aggaggcaca actgctgttt ctgctgccat gacaacagca 2400
   cagtgctgct gaagtgctcc ctacctcttg gtgaggggac agtaatgata gatgacaggc 2460
   gtgaggcaaa agggaaggtg cacctctgaa acactaggtt agggctgggc tatggaaggg 2520
   ctctttgggt aggaaaaggg ttaacgctgt gcatagagcc tcggtaggaa ggcagaaaag 2580
   ggactctgaa tctgccatgc ctctcctccc caccactgcg ctggggccac tcccaccctt 2640
   cccaccctgt tcatctggcc tgctccccgc tcatgggaag cctgggcacg ggccacacag 2700
   ctgtcagtct aacacagcct tggctctggg gccccaagga ggtagggcaa tctggaaggg 2760
   caaggagcca agactagatt tggggtgcag cccagcgtgc tccctgcctt ttaagcaaag 2820
   gttatcacca ggccagctaa acttagcaat aggctcttga gctagatgag cagggggctg 2880
   gtttcatgtt gcactggcct agcaaagagg cgccagagtc cccctgccct gcacctgcta 2940
   cagtgctccc aagcccttcc acccttccca gcctccctac ccttcggagt gaagaatccc 3000
   ggtctcgaaa agacaaaata aaaccaaaga atgccttttc tcccttccac ttgtggcaag 3060
   aggctagggg cttccctgtc ctggctcagg ggtataatat ttcctctcct gtgttctccc 3120
   ctcactgatc tcaccctgtg tccacgaggg cagccaaaaa aggagagtcc ctggctccag 3180
   ggccagtcag cacgtccctg gatggggagg gacttccgcc ctcacgtccc aactctccac 3240
   cctggggcta cagtgggtga aaggggcagt gtctcctagc ctgggcgggg cagctctcag 3300
   gttccgagga gggatactat aggaggcctc tgagcctcct ccaattcaac ccttaagagg 3360
   gatgctgccc ttaccccggg gtcccagctt aggttcatca ggtaaactca ggagagtgtt 3420
   tgtaagtctc aattatcagg tttccaggct gcagggcatc ctgacctatg gcgtagcaat 3480
   ctccttttca agcgtcttcc tggccacccc cttgccctca cagcgcggta cacaagccag 3540
   gcctctggcc aaagacagaa gccaggaggg gggggggggc agatagggaa atggggctcc 3600
   tgagggctcc cgcgggagga aaagtgcccc caccctggca ttttcttcca ctcttttttt 3660
   tttcgggggg gggggttgct gtgtcactac cgaagaacaa cgaggagaag atcctcaact 3720
   tttccgcagc cttttcaata atggggagag gttcgatgat gcagtggcag ggagacccac 3780
   acttctccat ttcccctgtt ctcccatttt actcgggaag cagcattcag gtctctgggt 3840
   cctggatgtc cttggtgcac actccaagga ctcctcgtcc ttaagttcat agtctgtatt 3900
   ccctgagtcc tatcctggga accatcaccc ggtcacctcc tgagcggggc aatctcagct 3960
   cccctccccc tatcagttcg gagcccacac gcttggtgcg tgcacatttc aaaaatgagg 4020
   cgggtccaaa gagagggagg aggggaaatg agagaggccc agctactcgc ggctttacgg 4080
   gtgcacgtag ctcaggcctc tgcgcccttg agctaggact ggataagcag ggcgggaggc 4140
   ggggcgcgcg tcatcagctc ccccccacca tccgggttcc tataaatacg gactgcagcc 4200
   ctccctggtg ctctctgctc ctccctgttc cagagacggc cgcatcttct tgtgcagtgc 4260
   caggtgaaaa tcgcggagtg ggccgcagga ggccggggac agtcggaaac tgggaagggg 4320
   agtgggcact gtacgggtct agggatgctg gtgcgaagtg tgcaagccgg acccaggctc 4380
   cgcattgcag gggggggatg atggaggacg tgatggggcg cacggcggga atggaggcgg 4440
   ggtgggggag gggactgcct ggtgtccttc gggccacgct aatctcattt tcttctcctg 4500
   cagcctcgtc ccgtagacaa aatggtgaag gtcggtgtga acgggtgagt tccagggcgg 4560
   ggccctgctc cgttgcctac gcaggtcttg ctgacccggg ggctctgcag tactgtgggg 4620
   aggtggatga ggtggccgaa gcgcccaagg agacctcaag gtcagcgctc ggacctggcg 4680
   atggctcgca cttgcggccc ccagctgctg cacctctggt aactccgcct ttgcggggat 4740
   gagcggcccg gagtcttaag tattaggaac aaccccacgc gcccgttcag acccatcccg 4800
   taatccccag tccggggctt ctttctttac tttcgcgccc tgaggagtca cgtgccagga 4860
   gggaagcccc tcccccatct cccccttcct cggggtcggt ggcatggcgt tgtgaggtgc 4920
   atacctttgc gcatcatctc ccaggcttgg gcttccttta gggtaactgg ccgccgccat 4980
   gttgcaaacg ggaaggaaat gaatgaaccg ccgttatgaa atcttgctta ggccttcctt 5040
   cttcctagct tgtgactaac ctcattcctc tcggctgggt ggagtgtcct ttatcctgta 5100
   ggccaggtga tgcaaggctt ccgtgctctc gagagagttc tacctcacaa tctgtctcac 5160
   cttattagcc ttaaaagccc ttgagcctta ttgtcctcgg gcataatgcg tattctagat 5220
   tattctctga aaatcaaagc ggacttacag aggtccgctt gacctcccaa ccccagaggt 5280
   agttatggcg tagtgcagag ccgtgggatg gggagctgag tcatggtggt tctgaaaaga 5340
   aattttccac cacaaaatgg ctcctgtagt agcagcccct tccatcccct gcacttccca 5400
   tcacagcctc gcactgaccc aggccctata ggccaggatg taaaggtcat taagaggatt 5460
   gggtgtccct gcgcctcaga atcctgccct tctccccgtt ccatcctcca gaaaccagat 5520
   ctctccactc cgccctgatc tgaggttaaa tttagccgtg tgacctttct ggatctgggg 5580
   tctgagcggg ctctccaccc tgctccccct acacacatct gttgctccgg ctctcatttt 5640
   tgcccgagaa gaacaggtgt ttcgcgaacg agccctggga ttagggttgg aaacccccca 5700
   catgttttct cagtctttcc ccttagttcg agggacttgg aggacacagg tgggcccgcc 5760
   ctgtgctgct cacgctgacc tttagccttg ccctttgagc ttgctgatga atgagttcac 5820
   aggtctgccc tgtccagggg gtgtagcctg aagtccagcc atgctggaac aaacttccca 5880
   gggcatgagt gatgggggtg atgtgccaag cttgtaccca gactggggca aactgccact 5940
   tcttaagaga cttagaatga cttggaggag gtttgctggg caagcaatca cctcttggac 6000
   aggaaagaaa cctccacttt ataaccgtgc tataaaagcc ctgccaggcc tcggctgctc 6060
   aaagaatata aaattagatc tctttggact ttctagggtg ggaacagtct atatttgggt 6120
   tgtacatcca agcattcaac tagctttatt aaagggaatt ctgaacaaaa catgaacttc 6180
   ctgatgcata catttctaat gtactgtgtc tgcataaagg cttgtacctt tgttgtggta 6240
   cgtgcatagc tgatggctgc aggttctcca cacctatggt gcaacagtat tccactctga 6300
   agaacatgag atagcctggg gctcactaca gacccatgag gagttctgat ctcagctccc 6360
   ctgtttcttg tctttcagat ttggccgtat tgggcgcctg gtcaccaggg ctgccatttg 6420
   cagtggcaaa gtggagattg ttgccatcaa cgaccccttc attgacctca actacatggt 6480
   ctacatgttc cagtatgact ccactcacgg caaattcaac ggcacagtca aggccgagaa 6540
   tgggaagctt gtcatcaacg ggaagcccat caccatcttc caggagcgag accccactaa 6600
   catcaaatgg ggtgaggccg gtgctgagta tgtcgtggag tctactggtg tcttcaccac 6660
   catggagaag gccggggtaa gtggccggaa agctgaaggt gacgggcacc cttgatatgg   6720
   tgcaacctga aaaccaagaa ctgagtctga aatcaacttc tttcccttaa acaggcccac   6780
   ttgaagggtg gagccaaaag ggtcatcatc tccgcccctt ctgccgatgc ccccatgttt   6840
   gtgatgggtg tgaaccacga gaaatatgac aactcactca agattgtcag caatgcatcc   6900
   tgcaccacca actgcttagc ccccctggcc aaggtcatcc atgacaactt tggcattgtg   6960
   gaagggctca tggtatgtag gcagtgggga gacagctcat gcatttctta tcttaccctg   7020
   ccatgagtgg acccttcttt gtaggtgtcc cttttgggta gaggggtgcc gtgcaggacc   7080
   tcactcattg cccccgtgtt ttctagacca cagtccatgc catcactgcc acccagaaga   7140
   ctgtggatgg cccctctgga aagctgtggc gtgatggccg tggggctgcc cagaacatca   7200
   tccctgcatc cactggtgct gccaaggctg tgggcaaggt catcccagag ctgaacggga   7260
   agctcactgg catggccttc cgtgttccta cccccaatgt gtccgtcgtg gatctgacgt   7320
   gccgcctgga gaaacctgta tgtatgggga gagctgggct tgtctctggt gtgacagtga   7380
   cttgggacaa ggatagtcat tttggggttt gttcttatca ggccaagtat gatgacatca   7440
   agaaggtggt gaagcaggca tctgagggcc cactgaaggg catcttgggc tacactgagg   7500
   accaggttgt ctcctgcgac ttcaacagca actcccactc ttccaccttc gatgccgggg   7560
   ctggcattgc tctcaatgac aactttgtca agctcatttc ctggtatgtg gggatgggaa   7620
   acctgacttt taagagcaac tgggggtttg gtgccctctg gtggctagct cagaaaagaa   7680
   acccaaacta acagttgtcc caatttgttc taggtatgac aatgaatacg gctacagcaa   7740
   cagggtggtg gacctcatgg cctacatggc ctccaaggag taagaaaccc tggaccaccc   7800
   accccagcaa ggacactgag caagagaggc cctatcccaa ctcggccccc aacactgagc   7860
   atctccctca caatttccat cccagacccc cataataaca ggaggggcct agggagccct   7920
   ccctactctc ttgaatacca tcaataaagt tcgctgcacc cacctctcct tggttttaat   7980
   gcggtgttgg gggagggagg gtgtgtatgg ggaggtgaag caggctcaat caacttttgt   8040
   gcatgtatct ttattggctc tgacatccag ctgggtgccg gaagtccagg gctacattct   8100
   atccaagtcc cagcccaggg cgcctgtcct gaacagctga tatgtctggc aatgcggagg   8160
   gtggggcttg acaatgggac aggaaatgca gtctgagact cacttgttgg acagcactga   8220
   cttccagagc tcactgctct tgagaaagat cggtgtcctt ttatctgcaa acaaaggtta   8280
   attgtaccca gccctcctgc agacagacgt aaaattagat aaaattggaa gggcgcccag   8340
   tacagtaaaa cctagtggta tccatgaaga ggggctgtag cactggtcga tgctttggtt   8400
   cgtgcgtcac acagtgactc agtgcttggg aagtattcca caccgtcatt gcttttaaag   8460
   gttccattcc aatagttcaa catagcgtct cctgggaggt cgtgactgac tccctaggtc   8520
   agggacatct taaccttttg gttttgtcag tgacagccat ggcggcatac cctcagctgc 8580
   tcactggaga aacaggcccc gagcaccagt aacctgcagg ccacacccat gtgcgggcag 8640
   cagcttcacc tcctcagccg cgacctgggc ccagcacagg acttcccaaa ggcgagcctg 8700
   cacggccatg ttcactgcaa gaggcaagtg aacagctggg agcatgtgga cagagcaccc 8760
   cacaccctcc aaagccttac tagggagcaa agccctatcc caaggtacat gatgaatctg 8820
   cttcacacga gtgcctaggc aagttagcac cagtacaaaa tccaagatac ccaaggaata 8880
   caggcaggtc ccgtgatcac attccttaca aagtggaaaa gggaggctca caagtgtcct 8940
   ggcagcattc cgaagatgga gaagtagcag atacccacat gtgaggaagg caggaaggag 9000
   tgtcccaatg tcatgggcgt tgtacaggga gggtggggct gagcctagct ccagacaccc 9060
   cagaacctgg ggagggacag cccagggcca ggcttcatgc agtggcacag aagacccaca 9120
   cccctgcaga cccacacccc tgccccagtg agctctgggg tgccagacac tggtttctat 9180
   ctcatggagc tatcagatga gacgtctcga tcggagtcct cagtctcact cggcggtggc 9240
   ggcgggtcgc taagcgggac cgcagtgaaa gcaggagact ttctagaaaa aaacaccagc 9300
   tgtcaccttg gaccaagcag gaaggctgca gacttctaag cttgctttct catggccttc 9360
   tgaggtacag ggggtgccag gcaagggctg gggagggaag aagacacttc ccttttcaac 9420
   ttgatgggga gcagtgcctc tcttatcttg gggggctctg agaggctcag agatcagaaa 9480
   agtgcgaagt cacagcacgg agatgccccc tgagccacgc tggggcctta gcttcttgtc 9540
   cagcagtaat ctggtatggg ggtgggggct gctggacccc aaaactcctg ggtgcaagca 9600
   atcctcttgc ctccaaagtg gctggggtgg agccacagat ctcttgtaag catgcacact 9660
   atactcagct tgatgtaatt tgggagattg ggttgggttt tttgttgtct ttgagacagg 9720
   gcctgtacgc attcctgtct ggcctggaac tctatacaga ggtgaggctg gcctggtact 9780
   tggagtacct gcctgttgct tctatgcatt tgaatgacag atatgcgcta gcacacctga 9840
   cttactgtag tttcttggat tttgtttgtt gttgctattt ggtttgtttg ggacaaattt 9900
   ttgctgctgt cgtccaggac agcctaaaac tcatgacaat ccttctgcct ctgccttcca 9960
   agactgaaac tgcagacatg taccactctg cctggtttgc agtaaatttt aaaaaaaatt 10020
   tttaatgtgt gtgtgtctgt gtatgtgtat ctgtctgtgt gtctgtgtgt gtatgtgtgt 10080
   gtctgtgtgt gtatctgtct gtgtgtgttt atctgtgtgc ctgtatctgt ctgtgtgtat 10140
   gtctgtgtgt atctgtgtgt ctgtgtctgt ctgtgtgtgt atctgtgtgt gtgtgtttgt 10200
   atctgtgtgt gtgtctgtgt gtctgactgt ctgcctgtct gtctctgtgt gtgtgtgtgt 10260
   gtgtacacag ggccccacac aggctaggca agtcctctac cactaagcta tactccgaaa 10320
   ccccagattt actcttacac aaggtcaaac acataaaccc gtgatttgga taagatcctg 10380
   tccccgtatc tttggtcctt gcctggttta ggttctaaga cactcagagt tgggacactg 10440
   gtcagtttca tctgatggcc aaggcaggga gaggaagctt cattctgggc cacacctaca 10500
   ggcgactccc aaggacagct ggacataaag atgtgacctg gtccctgccc acagaccatc 10560
   ggggcctatt ccagccttcc tccctgggag agagacccag cccgctcggg gcaggccctt 10620
   accggtcccc ggactgtgtg atgagccggc ggcaggtctc catctgcacg tccaggcccc 10680
   gcttcatgct gcacatctcc atgtactcat gcaggtgtcg gttcatgtcg ttcttggctg 10740
   tggccagctc cagctgcagt aaggtggggt cagggaggac cacggttagc aaacccaggc 10800
   aaggccctgg gggccaagca gctcaggaga caagtaagct acaactgcct gtttccccca 10860
   ggatatggag gaggggtggg gcaactggcg atctgacttc aggccctcac tgtggctagg 10920
   caagggctca ggacagtgct ccccgctcgg ccctgtcctg ccaatccact cctactgcca 10980
   cccaagaatc aatcagctga tgtctgagtt ctctgtaaga acagcaagag cacacaacac 11040
   agtaaggatg gtgcggtgcg gtgcggtgcg gggagcacgc atccctccag cgcttactcg 11100
   gctcaggcag cagggtcccg aaggcaaacc taaatataaa acccggtctc aaaaaggggt 11160
   aaggaggcca caccaggcgg ctcagtggat aaggctcctg cgcatctggc aatgtgaatt 11220
   tgttctctaa cctccacaca tgcaccgtgg catgtggaga gagagagaca cacacacact 11280
   aataacaata ataataaaat ttagtatttt gggggctggt gagatggctc agtgggtaag 11340
   agcacccgac tgctcttcca aaggtccaga gttcaaatcc cagcaaccac atggtggctc 11400
   acaaccatcc gtaacgagat ctgacgccct cttctggtgt gtctgaagac agctacagtg 11460
   tacttacata taataaataa ataaatctta aaaaaaaaaa aaaaaattta gtattttgtt 11520
   tgtttgtttt tcgaaacagg gtctctccat gtagccctgg ctgtcctgga actcactcta 11580
   tagaccaggc tggcctggaa ctcagaaatg cgcctgcctc tgcctcccga gtgttgggat 11640
   taaaggtgtg caccaccact cccggcaagg cagcagttct taacctgtga gtctcaatcc 11700
   ccttgggggt tgactatcag acactctgca tgtctgtatt tatgttataa ttcgtaacag 11760
   tagcaaaatt agttatgaag cagcaatgaa atagtttatg cctggggcca ccacagcgtg 11820
   aagcactgtg ttaagggtca cagcgtctgg aaggctgagc accactgctt taaggacaga 11880
   cagggccact catctgtcca tctttgccac tgagcctccc ctcaactgaa gctaattcac 11940
   tgtgaaatat tccaaagcct cgctgcaatg gagggaacct ggctgcttct gtttatagca 12000
   tttcattcca catcatcttc tcccttatat cctgctcgcc cacataagag agagaccttc 12060
   ctcctgagtg attacagggt aaatccccaa caaaagaaat gggggggggg agtctctgta 12120
   tcaagaaaag tctcccttcc agagctgact ctgcagccag gtttgaaggc agcaaaaaaa 12180
   gtaggctgag gcggaaaagt atctaggcca gagacaaact gcggcctctc tttgtttgtg 12240
   atacaaaggc caggggccag atagggagaa tcagactgcg gtggggagat ttgcttggct 12300
   gcctgagggc aaagacaagg aggccgagtg gccttgaagc atgtggatca gctgtgggaa 12360
   gtcacccgaa acaaatgtga actggaccgg gtgggagtgg gccccaggag cagacaaagg 12420
   agacagccag gacactggca ttttgggtca tctgctgaac agctgggtgg taagccacag 12480
   aggagatggc ttgaaaggca tgcttgttcc cacagcagac tgcaccattt gtgagaaaaa 12540
   catgacactg aggatcagag ctcagctgcc accccccaca gacaaatgca cacagcattc 12600
   caggtcagag atcagctgcc acccatgtac atcaacacgc acacacacac acactattac 12660
   aggtcagaga gcagctgtca cccatacata ccaacacaca cacacactat tacaggtcag 12720
   agagcagctg tcacccatac ataccaacac acacacacac acacacacac acacactatt 12780
   acaggtcaga gagcagctgt cacccattca taccaacaca cacacacaca cacacacact 12840
   attacaggtc gagagcagct gtcacccata aatacaagca tacccctcac tccctcccct 12900
   accccacctc cattatagtc tctcaaagca taaggcagta ctaccagcca tgtctctaag 12960
   tgtcatatgt caccctgatt tacacactga aggggaagat gcacattcta gaagatgcct 13020
   tcaacaaatc acccccaaat ttcagactca gtgtgggaga ggcaggggta gaggtagata 13080
   ggagcgatga aggggcgggg ccatggaaga aaactccacc caccactctg catcaccatt 13140
   caataatggg ggctggtcta tctctatgcc aataaccctg agatcaaggg tcatggagat 13200
   aaaggtccta cagatgctcc agggagaccc aggagctact ctgctaccaa cgctaccaag 13260
   tgagcatcct ttggctttct cctcaactca ttctcccagg agagaacaga agtcatacaa 13320
   cttaggaaca gcttcaaaca tgttcccaat ttttttggga gggcaggggg agagtagagg 13380
   tttgaagcag ggcttctctg tgcagccctg gccatcctgg aagtagttct gttccccagg 13440
   ctgtcctcga actcacagag atcctcctgc ctgttttccc actaccagga ctaaaggtgg 13500
   caccaccaca ctccatttta taggtactgg tatcaaacct agggcttcac gcatgaaggt 13560
   aggcaggcat tctaccatga gagcacagtc ccagcccagg gtgctcttct ctgtcctacc 13620
   acatcatgtt tacacttagt tatttgtggc ctaagggatg aaccacagaa gggcagcagc 13680
   tcctgatcac tgtgctaaga ctcctacctg gctgacggta gggaaaaaag gaccaagcct 13740
   taaatctacc gtctgtaaga ggtggacaca acccagggag ggggacagga aggaggaagg 13800
   agcagtaaga ggagcggaca ggaggagatg ggagatcatt tgtccccagg caaagcttaa 13860
   ctggtggcag aacactggct gggtgggaat tctgggttaa aaaccaaaag cactggtttt 13920
   cctcagagta gcacggccat ctgacatgtg ctggccactg cagaagagtt acttccaaag 13980
   ttccctttca aaatgcaaac agagtgaccc ccatgggcac gggaatgcac cacagggctc 14040
   gcagggagcc caatggcatt agtctcctgc ctcatccctg catcagtgtc taagacatag 14100
   agccctgttc actgtccaat ggcgaccccc ttcctgcagt ccctgcctgc agtccagcag 14160
   gacaatctaa accccagccc caccaccatt catacccgat gccccaaggc tcaaaaaaca 14220
   aaagagggag acacctccag gagctccaag gctgagccaa ggagacagga gtccctctgg 14280
   gtgctacggc acttagggtg ttcccagtaa taaatgcgca cactaccgca tgtcaacctg 14340
   ccagatcctg tcctaagagc cacgcgcgcc tcactctatc tgatcatctt aataaccctg 14400
   cctgcctacc agcctgtcgg gagagattat gaacatctct attggacaaa taaagagaca 14460
   ggcccaccct tagcagggta gaaaccatct ctaaccctaa caggctgagg caggaggatt 14520
   gcctcaagtt tgactggcta cacagaaatc tggtctgggt gggggcttag gatggctcag 14580
   tagttaagag catgccctgt tcttgcagag gacccaagtt tattttccag cgcccacatc 14640
   aggttcacaa acacctgtga gcgccagctc ctggaaacct gatgcccacc tctgacctcc 14700
   aaggagagag ggggagggag ggagggaagg aaggagggag ggagggaggg agggagggag 14760
   ggagggaggg agagagagag aaagagagag agagagaaag agagaggaga gatgggggcg 14820
   tggatctgcg aggaaggcag gcaggcaaga tgaatgtgtg tgagagccta caacaaaaga 14880
   ataaaaagga atgaaggagc tagctgagga acacctcatc agtgggaggc ttactgcaca 14940
   gcatgaagac ctgagtttga tcttagaacc catctagaaa ataagctagg gctgaagaga 15000
   tggctcagca gttaagagca ctgtgtttgc tcttgcaatt cccagcaacc acatggtagc 15060
   tcacaacctg ggacctgatg ccctcttctg gcatgcaggt gtacatacag atagaacact 15120
   catatattaa ataaataaat aaataaataa atcttaaaaa aataaaataa actagccata 15180
   gtagaacaca cttgcaatcc caaagctaag aatgtacaga cagacagaca gacagacaga 15240
   cagacaaaag atgcctaggg ctccctggcc agctcggcct actcagcaag tttcagacca 15300
   gtgaaagacc ctgtctcaat taaaaaaaaa aaacaaaaaa cccttaacca cactcaaaga 15360
   acactaccca ggtttgtctt ctggcctcca cacttgtatg tgtacacatg tgcgcgtgca 15420
   cgcacacaca cacacacaca cacacacaca cacacacaca cacagaggaa aggaggaaga 15480
   cccaaggaag gataaaaagc tggtcagtca aagctggctt aaagctcctg ccgtaatcct 15540
   atcgcacagg aggcgtgggc agaagcacca ggagcttgag gccaacctag actacaagac 15600
   aagatcttga aaagaaacag agaggcaggg gcaggaagga cactcacaag tgcgagagag 15660
   cttgcagttg tgatgcacac ttttaatccc aggagaggca cagtaacttc tccagagtga 15720
   gttccaggac agccagggct acagagaagc cctgtctgtc agggggaaaa gaaaaaaaaa 15780
   agaaaggggg ggacagaaaa gaaaaagata gtctggtggg aaatgattca ccgggtaaag 15840
   gcatgagcca ccaagtccaa agacctaaac ttgattgctg tcacccccat ggtaaaagag 15900
   aacttcctcc cacacgttgt tctctgacct ctacacatgt gcatggactg cgcatgctct 15960
   ctgacactcc atccatccat ccatccatcc atccatccat ccatccagtg tgtgtgtgtg 16020
   tgtgtatgtg tgtgtgtgtg tgtgtgtaaa ataaataaaa aggaaggagg gagggacagc 16080
   aaaagatttg ttccatgtgg tgacagagcc gggatttgaa cccacatcta tgtgcctcca 16140
   gatcctgaag tctcaaggac ctgctacagc taccacaact gagaggatca ctactgacaa 16200
   cagatggaag ctcgggccct ggggttcagc gaggaggagg ccagcaacac acctacagcg 16260
   tggctcctga gccctggacc tacagcgtgg ctcctgaggc cggcaccctt ac 16312
<210> 19
   <211> 11058
   <212> DNA
   <213> Rattus sp.
<400> 19
   gtgagtggac ccggatcact ggccctgttg ggttccggtg taggaaacgg aatccgctct     60
   ccctaagtgc tcagagatcc ctgtctggac ttgctagcct cactacagcc gtgcaaccga    120
   agtgacttaa ggccagacct tttttttttt ttttttctga gctggggacc gaacccaggg    180
   ccttgcgctt gctaggcaag tgctctacca ctgagctaaa tccccaaccc caggccagac    240
   cttttttaaa ggccattcgt ttcctctttt atttattttt tagtgtgcac tggtattttg    300
   cctatgttta tgtctgtgaa ggagcccagt gccctggagt tggagccaca gacagttgtg    360
   ggtgctgaga actaaacaca ggtcttaacc actatgccgc cccccccccc cccagcctca    420
   gtaaaggcca ttttctactt gcgatagggt cttgctatgt agacccagct ggcctcaaac    480
   taacagagat tcccctgcct ctgtctccag tgtgctggta gtaaaggtgt acccccatgt    540
   tcggcactag actgcttctt tagagtggtc tcatgtttaa agtaacactg agttcccatc    600
   tgcctgtccc acaccctcat ggcctctacc ccatcacctt atcctcggta cgtgatccta    660
   caatctacat tgactgggat gaaggaatga gattgacaga tcagcatcca aagtccactc    720
   actgaatcca gtggcacatt cttgccacta gtgtcatact gggcattttc aggactgtga    780
   ggtcacctca ctccccaccc tgtggtggta tcagtcagtt ggaatcacag tttcagagtt    840
   tgtttcttta acttgctgta tctttcatgg ctgcttttta aatagggaga taatgcttcc    900
   gaccccgtct ttaccttttt ctccaggctg taatagatga atttgagcag aagcttcgtg    960
   cctgtcacac cagaggcatg gatggaatag aggagcttga aactggccaa ggaggcagcc   1020
   agcgggccct gtctgccaag aaaccctccg ccggtgtgag aggcaagccc acgctgggcc   1080
   ccacaggcag tcccctcttt tctttttttt cctttgtggt ttgtttttgg ttttgaaatc   1140
   atgtccctct gcatattcca gtctcaaggc aacagcctct gacttctgta gactcagaca   1200
   atgactttgt cacacctaag cctcgacgta ccaaaccccg tcgtccacaa actcagcaac   1260
   gcaagtccca gaggaaagcc aaagttgtct tctcaagtga cgagtccagt gaagatggta   1320
   agatacaccc tcccaacagg acaactgctt gtgtggggtc tccagccggg cttgcctggc   1380
   tgtggattca ttctccctgg cagtgtcttc cccactgtgc agctgctggg ctgactttca   1440
   ctctgtgtgt gtgtttgtgg tgtgtgtcca gatccgtttt gccccatctt tatgactcag 1500
   cgtttgttcc tataattctt tgaatagaac tttcagcgga gatgaccgaa gaagagacac 1560
   ccaagaaaac cacccccatc cgcagagcat ctgcgcaaag acacaggtcc taggagctgc 1620
   ctttgcctat tcccatccct gctgtgcagg gtatcttact gaatgatcga gaattcaacg 1680
   cccccccccc ccttggaaaa tacttgttgc ctccttgttt ttaacatgta aatggtcttt 1740
   tcgagcaagg cctttctctt agagcccctg actgctactt tccaagatcc aatcaccatg 1800
   ttttctagtg aataaatgtt tttatatact tttaagcatt ctggccggct ggtctgttcc 1860
   attattaggc agcagatagc agtccagaga gagaacccca ggcatcctga gccatctctg 1920
   gaccttcatg gaccctgtgg tgcttcatct ttgcacaact gctgagattt gctgtagaga 1980
   gttctgtggt ttctaggttc acataagcac ttctcagcct gggggtactt tgcaccccag 2040
   aacacgtttg gcaatatcta gagaccaggg gtgggagaga tggctcagcg attaggagca 2100
   cttactgccc ttccagaggt cctgagttca attcccagca accacatggt ggctcacagc 2160
   catctgtaat gagatctggt gccctcttcc ggtgtgtcta aagacagcta tataataaat 2220
   aaataaatct ttaaaaaaaa aatctagaga cctatttctt ctgcccacct tgtggggcag 2280
   gggctgctgt aggcacccag tcagtcagag cctaaggagg aagactcctt catccatagc 2340
   actcccaaca cagcatgtcg ggactgtgca actagaggtg ctgctctaga cctgcggttt 2400
   gtttcctgtg tgccacaccc catcccagaa gactggcaaa tgagtcagcc tggctggtgg 2460
   cagagatcat gccattagct ctgttggtga caaggtccac ctcccaagag tcagtcctcc 2520
   actatgctaa ggggtctctc gctcagctgc aggcccaggc tgtgctatgt gctgcccacc 2580
   ttccccaagc ccttaatctg ctgagtcact tggagcagga ggcaggactg ttgtttccgc 2640
   tgtcatgaca acagcacagt gcagctgagc ctctccctac ctcttggtat ggaaagagta 2700
   atgacaggcg tgaggcagaa gaagatgcac ctcagaaaca ctaggttagg ggtggactgt 2760
   ggaagggctc ttgagacctc tgggtaggaa aagggttaac tcggtggata gagcctcagt 2820
   aggaagacag aaaagggaca gctgagtctg agatgcctgt ctccccagca ctgcactggg 2880
   gccactccta ccctttccac cctgtttttg gcctgctccc tgctcatgaa aagcctaggc 2940
   atggaccaca cagctgtcag tctaaacaca gctttggctc tggggcccca aggaggtagg 3000
   gcaatcccag aagggcaagg agccaggact agatttgggg tgcagcccag cgtgctccct 3060
   gccttttaag caaaggttat caccaggcca gctaaactta gcaataggct cttcagctag 3120
   aagtgcaggg ggctggtctc aagttgcact gggctagcaa agaagcccca gagtccccct 3180
   ggcccgcacc tgctgatggc ccgcacctcc caatcaaccc cttccaccct ttctagcctc 3240
   cctaccctgc aaagcaaaca agtccttttc tccctccact gtgcaaagag gctaggggtt 3300
   gcctgttctg gctcgggata cttcccttct gctttcttcc ctttctgatc tcaccctgct 3360
   tccacgaggg cagccaagga aagagagtcc ttggctcctg ggccagtcag caggtcgcct 3420
   ggatggggag ggacttccgc cctcacgtcc cagctctcca ccctggggct gcggtgggtg 3480
   aaagggacag tgtctccaat cctgggcggt acaactcagg ttccggggag ggaaggcctc 3540
   tgagcctcct ccaacccaac cctcaacagg gatgcttacc ccggggcccc ggtataggtt 3600
   cagcaggtaa actcagggaa gtgttgtctc aatcaggttt ccagactgca ggggatcccc 3660
   gacctatggc gtaggaatct ccttttcaag cctctagtca cttccttgct ttcacatcgc 3720
   ggtacacaag ccaggactct ggcctaagag atagaagcca ggcggtgggg agagaggaaa 3780
   atggggctgc gaagggcccc caccggagga aaagtgcccc ccccgtccct cccccagcct 3840
   tttcttctgc tccttttttc ggggcggggg gggcgtgctg tgtcactacc gaagaacaac 3900
   gagaagatcc tcaacttctc ctaagccttt tcactaatag ggagaagttc gatggggcag 3960
   ccttgggcag acccacactt ctgctccatt tccctggttc ctgcagctct cagattctcc 4020
   cattttattc gggaagcagc tttctggttt ctgggtcctg gatgtccttg gtgcacactc 4080
   caaggactcc tcgtccttaa tccatagtct gtattccctg agtcctatcc tgggaaccct 4140
   catccggtca cttcctcggc gggacaatct cagctcccct ccccctctca ggtcggagcc 4200
   cacacgcttg gtgcgtgcac atttcaaaaa cgaggcgggt ccaaaaagag ggaggggggg 4260
   aatgagagag gcccagctac tcgcggcttt acgggtgcac gtagctcagg cctctgcgcc 4320
   cttgagctgg gactggatga gccgagcggg aggcggggcg cgcgtcatca gctcccccca 4380
   ccatccagtt cctataaata cggactgcag ccctccctgg tgctctctgc tcctccctgt 4440
   tctagagaca gccgcatctt cttgtgcagt gccaggtgaa aaagaaaaga aaaaaaagga 4500
   ctgggccgca ggaggccgga gacgaatgga aattaggaat ggggggaagg acgctgtacg 4560
   ggtttagggg cgctggtgcg aggtccggaa gccgagccca ggctccgcat tgcagaggat 4620
   ggtagaggac gtgatggggc atgcggcggg aatggaggcg ggtgggggga ggggactggc 4680
   cacgctaatc tgactttctt ctcccgcagc ctcgtctcat agacaagatg gtgaaggtcg 4740
   gtgtgaacgg gtgagttcca gggcggggcc ccgctccgtt gcgcatgccg gtctggctaa 4800
   attcggggct gtgctgtagc gtgcggggag gtggataggg tggccgaagt acccaaggag 4860
   acctcaaggt cagcgctcgg acctggggaa ggctcgcact taacgggacc ccagctgttg 4920
   ctccccttgt aactccgcct ttgctgggga gcggcccgga gtctaaagta gcaggagcac 4980
   ccccgacact ggcgcgcacc cgctcaggat ccgacccgta atccccaggc tggggcttct 5040
   ttctttactt tcgcgcccag aggagtcacg tgccagaagg ggagcccctc ccccatcgtc 5100
   cccttcctca gggtcggtgg cacgggggtg tgaggtgcat acctttgcgc atcttctaag 5160
   ggcttcactt atggtaactg gccgccgcca tgttgcaaac gggaaggaaa tgaatgaacc 5220
   accgttaaga aatcttcctt cggccttcct cattcttagc ttgtgactaa ctttctcatt 5280
   cctctcagct gggtggagtg tcctttattc tgtaggccag gtgatggatg gcagggtttc 5340
   cgtgctctca ttagagctct actatacaat ccgtctctcg tcagccttta taggcctcca 5400
   gccttatttc cctcgggcat aacttatatt ctagattgtt ctctggaagt cgaagtgcat 5460
   ttacagaggt ctacttggcc tcccagcccc ggaggtgcgg tagcaatggc gtagtgccga 5520
   gcccgggggt ggggtgggga gctgagtcat gatggtcctg aaaagaaatt ttctgccaca 5580
   aaatggctcc ggtggtagca gccccctccc tccagggcct ccacttccat cccagctcag 5640
   cactgaccca aaccctatag gccaggatgt aaaggtcact aagaggattg ggtgtctctg 5700
   agcctcagga tcctaccctt ttccccaacc catcctccag aaaccagatc tccccattcc 5760
   gccctgatct ggggttaaat ttagctgtct gacctttctg tatctggggt ctgagctggg 5820
   ctctctgccc tgttcatccc tccacacatc tgttgctcct gctccgattt tttttttttt 5880
   ttttttttgg ccgggaaaga caggtgtttt gcaaatgagt cctgggatta gggctggaaa 5940
   atcactggtt ttcttgatct ttcccccaga aagggacttg gaggaagcag gtgggctggc 6000
   cctgtcctgc tcactctgac ctttagcctt gccctttgag ctgctgatga atgagttctc 6060
   tcctgtccgg gagtgtagcc tgaagtccag ccatgctgga accggcttcc caggcctgtg 6120
   tggtgggggt gatgaatacc atgtgccaag cttgtaccca gaccggggca aaccgccact 6180
   tcttaagaga cttaaaatga ctttggggtg ccgggcaagg ctgtgggcct aatcacctct 6240
   tggacaggaa aggaacctcc actttatagc tgctataaaa gtcctgccag ccctgggtgg 6300
   ctcaaggaat ataaaattag atctctttgg actttctagg gagggaacat tctatatttg 6360
   ggttgtacat ccaagcattc aaccggcttt attaaaggaa attctggaca aaacatgaac 6420
   ttcctgctgc ttacgtttct gatgctgtac tgaatcagcg taagaacttg cacctttttg 6480
   tgatgcgtgt gtagcgggct gctgtaggat ctccacgccc atggtgcagc gatgctttac 6540
   tttctgaaga acatgcgttg gcccagggct gactacaaac ccaggagggg ttcactgatc 6600
   ccaactaact cgcctatttc ttgcctcaga tttggccgta tcggacgcct ggttaccagg 6660
   gctgccttct cttgtgacaa agtggacatt gttgccatca acgacccctt cattgacctc 6720
   aactacatgg tctacatgtt ccagtatgac tctacccacg gcaagttcaa cggcacagtc 6780
   aaggctgaga atgggaagct ggtcatcaac gggaaaccca tcaccatctt ccaggagtac 6840
   gtatggaaac atgcacaggg tacttcgagg agatggtaag gggcggcatt aactctgtcc 6900
   tgttccccgc ctgtaggcga gatcccgcta acatcaaatg gggtgatgct ggtgctgagt 6960
   atgtcgtgga gtctactggc gtcttcacca ccatggagaa ggctggggta agtggccagg 7020
   aagctggagg taaggggaac ccttgatatg ggtgcaacct ccaaactgaa gagctgagtc 7080
   tgaaatcaac ttctcctgca caggctcacc tgaagggtgg ggccaaaagg gtcatcatct 7140
   ccgccccttc cgctgatgcc cccatgtttg tgatgggtgt gaaccacgag aaatatgaca   7200
   actccctcaa gattgtcagc aatgcatcct gcaccaccaa ctgcttagcc cccctggcca   7260
   aggtcatcca tgacaacttt ggcatcgtgg aagggctcat ggtatgtagg caatggagac   7320
   agctcatgca tgagtggacc tttctttgaa gatgtccctt tgggtaggag ggctgccctg   7380
   caagacctca cccattgcct ctatgctttc tagaccacag tccatgccat cactgccact   7440
   cagaagactg tggatggccc ctctggaaag ctgtggcgtg atggccgtgg ggcagcccag   7500
   aacatcatcc ctgcatccac tggtgctgcc aaggctgtgg gcaaggtcat cccagagctg   7560
   aacgggaagc tcactggcat ggccttccgt gttcctaccc ccaatgtatc cgttgtggat   7620
   ctgacatgcc gcctggagaa acctgtacgt agggggagag ctgggtttgt tttgtctatg   7680
   gtatggtggt gacttggggc aagagcagtc atctttggtt ttgcccttac aaggccaagt   7740
   atgatgacat caagaaggtg gtgaagcagg cggccgaggg cccactaaag ggcatcctgg   7800
   gctacactga ggaccaggtt gtctcctgtg acttcaacag caactcccat tcttccacct   7860
   ttgatgctgg ggctggcatt gctctcaatg acaactttgt gaagctcatt tcctggtatg   7920
   tggggaccgc aagcctggct cttgagagta actgaaggtt tggtgccctc tggtggccag   7980
   cttagaaaag aagcccaaac taaccgttgt cccaatctgt tctaggtatg acaatgaata   8040
   tggctacagc aacagggtgg tggacctcat ggcctacatg gcctccaagg agtaagaaac   8100
   cctggaccac ccagcccagc aaggatactg agagcaagag agaggccctc agttgctgag   8160
   gagtccccat cccaactcag cccccaacac tgagcatctc cctcacaatt ccatcccaga   8220
   ccccataaca acaggagggg cctggggagc cctcccttct ctcgaatacc atcaataaag   8280
   ttcgctgcac cctcttcttg tgtgtgaatg cagtgttgtg ggggttatgg gcaggtgaag   8340
   gaggctccat tcggttttgt gcacatatct ttattggctc tgccatccag ctgggtgcta   8400
   gaggtccagg gctacattct aaccgagtgc cagcccaggc cgcctgtcca gacacctgat   8460
   gtgtctggca gtgtggaggg tggggcttga cactggaaca gaaggtgcag tctttgaaca   8520
   gcactgactt tcagagcttg ctgctcttga gaaagatctg tgtcctttat ctgcaaacaa   8580
   aggttaattg tacccagctc tcaaagtctc cagatgttaa attaggtaaa gttggaaggg   8640
   tgcccagtac agtaggacta attgtgtcca tgaagaggga aggtccctgc tgagctgttc   8700
   acctgtcttc aggtgtgggc acttgaaggt gctttggttc tggggcagtg actagccagc   8760
   gctcactgtt ggttgggaat gaaatgaagg tgcattacac ggtgactcga ctcaacgcct   8820
   tcattgcttt caaaggtccc attcaaatgt cagctctgca tagcctctcc tgggagatcc   8880
   tagctcccta ggaccaggga cattgtaacc ttttggtttt gtcagtgaca gccatggagg   8940
   cattccctca gctggtcact gaggagacac cctgagcacc cctaaccttg tgcaggccac   9000
   acccaagtgt ggcagcagct tcatctcagt ggcgacctgg cccaggcact ggacttccca 9060
   aaggcacagc catgttcact gcagtgggca ggtgggcagc tagctgtgag catgtgcagt 9120
   gtccacgccc tcaaggcctt cctagggagc aaggtcctat ccaaaggtac atgcggaatc 9180
   tgtttcacat gagtgcctag gccagttagc accagtacaa aacccaagga aaacaggcag 9240
   gtcccataac cacattcctt acaaatggaa aagggaggct ccaagtgtgc tggcagcatt 9300
   ccaaagatgg agaagtagca gatacccaca tgtgaggaag gcaggagtgc cccaatgtgt 9360
   tgggagttgt acagggggtg gggccgagcc tagctccaga caccccagaa ccctggagag 9420
   gacaagccag ggccaggctt catgcggtgg cacagaagat ggtcctctgc agacccacac 9480
   ccctgcccca gagagctctg gggcaccagg ccctggttcc tatctcatgg agctgtcaga 9540
   tgagacatct cgatcggagt cctcagtctc gcttggcggc ggcggcgggt cgctaagcgg 9600
   gaccgcagtg aaagcaggag actttctaga aagaaatacc agttgtcacc ttggggcaaa 9660
   gcaggaaagc tcctgagact tccaaacttg ctttctctgg ccttatcttt tgaggtacag 9720
   ggggatgaca ggcaaggacg ggggagggaa ggaaaacttg ttttcaactt gatgggagga 9780
   acagtttcgc tctttgggac tcagagatca gaaaagtctg aagtcacagc atggagatgt 9840
   tccctgagcc acactaaggc cctagcttct tatccagcaa taattcggtg tgcggggggc 9900
   tgtggggact gtaggaggtg agactggggg agtatggggg tttagggggc tgtttcactc 9960
   tgttgtcact gctgacccca aattcctggg tgcaagcaat cctcttgcct tcagttgatt 10020
   ttcttttttt ttttaattta ttttatttat atgagtacac tgtaactgtt ttcagacata 10080
   ccagaagagg gcatcagatc ccattacaga tggttgtgag ccaccatgtg gttgctggga 10140
   attgaactca ggacctctgg aagagcagtc agtgctctta accgctgagc catctctcca 10200
   gcccctcttg ccttcaaagt ggctggggtg gccacaaacc cagttagcgt gctcaccata 10260
   caatgcaatt tggggggtcg ggttgggttg ggttttttgt tctctttgag atgagtcagt 10320
   ttgcattcct gcctggcctt gaactcctca cagaggcgag gctggtctag tacttgtggc 10380
   aattcacctg cccgttgctt ctgtgcatct gaatgatagg tatgcgctca taagcctgac 10440
   ttaactgcca ttccttggat ttttgtttgt tgttgttgct gtttggtttg tgtgggacaa 10500
   ggttttactg ctgtccagaa cagcctaaaa ctcatgacaa tccttctgcc tccgccttcc 10560
   aagactgaga ctgcagacat gtaccgctag gcctggttta gagatttcta aaaaaatctt 10620
   agtgtgtgtg tgtgtgtctg tgtgtgtgtg tgtgtgtgtc tgtgtgtgtg tgtgtgtgtg 10680
   tctgtgtgtg tctgtgtgtg tctgtgtgtg tgctagacag gctagacaag gactctacca 10740
   ctgagctata ttccaaaacc ccagatttac tcttaaacaa ggtcaaacag ctaaactgtg 10800
   atttggattt ggataatcct gtccctctat ttctttgctc cttgccaggt ttgggctctc 10860
   agaccctcag agttgggcac tagtcggttt catctgacgg ccaaggcagg gagaggaagc 10920
   ttcattctag gccacaccta cagggactcc caaggacagc tggacataaa gatgtgacct  10980
   ggtcactccc acaggccact ggggcctgtc cagccttctt ccctgggaga cagccacgcc  11040
   actcagggcg gcccttac                                                 11058
<210> 20
   <211> 3158
   <212> DNA
   <213> Homo sapiens
<400> 20
   agcttgagat tgtccaagca ggtagccaga gagcgccatc agccaagaaa ccatccactg 60
   gtacgtaagg cagcctgtgc gggcgagacc agactgggcc ctcccctcct gcagtgattt 120
   gtttcttctt cttttttaaa tcacgttttc ctgccttttc taggttctag gtaccagcct 180
   ctggcttcta cagcctcaga caatgacttt gtcacaccag agccccgccg tactacccgt 240
   cggcatccaa acacccagca gcgagcttcc aaaaagaaac ccaaagttgt cttctcaagt 300
   gatgagtcca gtgaggaagg tatgatgctc ccgcctgttc ccggccgaga aggcacacag 360
   ctagggtgca gagggctggt ttccatagga cctgctgcgg gggcctgagt gtagatgctc 420
   tgccccactg ccgcagaagg gcctctcctg tacagcttgg attttatttc ttctgtgcgg 480
   tgtgggattg tctcacttgt tctctgatat ctattttttc accatctttg tgactcagct 540
   ttttcttatt cctttaattc tttgcataga tctttcagca gagatgacag aagacgagac 600
   acccaagaaa acaactccca ttctcagagc atcggctcgc aggcacagat cctaggaagt 660
   ctgttcctgt cctccctgtg cagggtatcc tgtagggtga cctggaattc gaattctgtt 720
   tcccttgtaa aatatttgtc tgtctctttt ttttaaaaaa aaaaaaggcc gggcactgtg 780
   gctcacgcct gtaatcccag cactttgcga taccaaggcg ggtggataac ctgaggtagg 840
   gagttcgaga ccagcctgac caacatggag aaaccccatc tctactaaaa ataaaaaatt 900
   agccgggcgt attggcgtgc gcctgtaatc ccagctactc aagaggctga ggcaggagaa 960
   tcgcctgaac ccagaggcgg aggttgtagt gagccgaaat cacaccattg cactccagct 1020
   tgggcaacaa tagcgaacct ccatctcaaa ttaaaaaaaa aatgcctaca cgctctttaa 1080
   aatgcaaggc tttctcttaa attagcctaa ctgaactgcg ttgagctgct tcaactttgg 1140
   aatatatgtt tgccaatctc cttgttttct aatgaataaa tgtttttata tacttttaga 1200
   cattttttcc taagcttgtc tttgtttcat ctttcacatt agcccagttt catgcagcag 1260
   agagagggtt atcagtgcag agagagatga gtgagcccag agtcctaggg cctgtcccgg 1320
   gatggcagat gagcttcctg ccccgtcact gccacctttc ccctctcaac ctctggaccc 1380
   tgcacagtga ccagacagcc tctctgggga gaattatgca gtgcctaggc tccagatcag 1440
   tgcttctgaa ccgggggcaa ttttgtctgc cagaggacat ctgacaacac ctggggcctg 1500
   ttttgttgtc atagcctata ggggaagaat gctaccagca tttgtgggaa gaggccaggg 1560
   atgtggctca acatcctgca gtgcacagga tggcccctca acaaagaatc acacggccca 1620
   caatgtcaat agcgtcacag ttgagaaaac ctgctctaga ccaagggttg ctttctgccg 1680
   tgtgcctcac cccaccccca ctcgtgttcc ctaatcccat ctccaaaggt tggcagcaga 1740
   ccggcccagg ctcgtggaag ttcagatcat gatcccctcc agctctgcag gagacaagac 1800
   ctgtctccca gcattcctca ttgttcccgg gtctgcagag ggcgtgagct atgctgcagg 1860
   cgggctgccc cctgaagcct gcgcacccct ctccagctcc tcaagtcttc tctgctgagt 1920
   caccttcgaa ccggaggctg tgagctggct gtcgtgacca cactggtgcc tctgctgtca 1980
   tgacaacagc acactacgtc agtagtgctc cctgggcact gagctccctc tttgcgggga 2040
   gaagacagta atgaaaaatg acaagcatga ggcagagggg aagatcacgc ttgggtggtg 2100
   caggagcatg gaggtgctct taatgctctc aatgagaaag ggttaacggt cctggttgca 2160
   ggaatagctg agtcagaggt ggggcttcct ccactccccc accccacccc tttcaccatt 2220
   agggaccttc ttgccttgct cttgctactc tgctctgggt ggtcattgtg aaaagcccgc 2280
   accaaccatg ccagtggcag ccagacgagg acacagcctg gctctgggtc ccagcaggaa 2340
   aggcaatccc agaaaggcag ggtcagggac tggagtcctg tgggtgcttt ttaagcaaag 2400
   attatcacca ggcaggctaa acttagcaac cggcttttag ctagaagggc agggggctgg 2460
   tgtcaggtta tgctgggcca gcaaagaggc ccgggatccc cctcccatgc acctgctgat 2520
   gggccaaggc caccccaccc cacccccttc cttacaagtg ttcagcaccc tcccatccca 2580
   cactcacaaa cctggccctc tgccctccta ccagaagaat ggatcccctg tgggaggggg 2640
   caggggacct gttcccaccg tgtgcccaag acctcttttc ccactttttc cctcttcttg 2700
   actcaccctg ccctcaatat cccccggcgc agccagtgaa agggagtccc tggctcctgg 2760
   ctcgcctgca cgtcccaggg cggggaggga cttccgccct cacgtcccgc tcttcgcccc 2820
   aggctggatg gaatgaaagg cacactgtct ctctccctag gcagcacagc ccacaggttt 2880
   ccaggagtgc ctttgtggga ggcctctggg cccccaccag ccatcctgtc ctccgcctgg 2940
   ggccccagcc cggagagagc cgctggtgca cacagggccg ggattgtctg ccctaattat 3000
   caggtccagg ctacagggct gcaggacatc gtgaccttcc gtgcagaaac ctccccctcc 3060
   ccctcaagcc gcctcccgag cctccttcct ctccaggccc ccagtgccca gtgcccagtg 3120
   cccagcccag gcctcggtcc cagagatgcc aggagcca 3158
<210> 21
   <211> 3218
   <212> DNA
   <213> Homo sapiens
<400> 21
   aagctggcac cactacttca gagaacaagg ccttttcctc tcctcgctcc agtcctaggc 60
   tatctgctgt tggccaaaca tggaagaagc tattctgtgg gcagccccag ggaggctgac 120
   aggtggagga agtcagggct cgcactgggc tctgacgctg actggttagt ggagctcagc 180
   ctggagctga gctgcagcgg gcaattccag cttggcctcc gcagctgtga ggtcttgagc 240
   acgtgctcta ttgctttctg tgccctcgtg tcttatctga ggacatcgtg gccagcccct 300
   aaggtcttca agcaggattc atctaggtaa accaagtacc taaaaccatg cccaaggcgg 360
   taaggactat ataatgttta aaaatcggta aaaatgccca cctcgcatag ttttgaggaa 420
   gatgaactga gatgtgtcag ggtgacttat ttccatcatc gtccttaggg gaacttgggt 480
   aggggcaagg cgtgtagctg ggacctaggt ccagacccct ggctctgcca ctgaacggct 540
   cagttgcttt gggcagttac tcccgggcct cactttgcac gtgtgcttac ctagtggaga 600
   caaaagtaca tacctcggta gagcgcgcac gcctgtaacc ccagcacttt gggaggccaa 660
   ggtgggtgta tcacctgagg tcaggagttt gagaccagcc tggccaacat ggtgaaactc 720
   cgtctctact aaaattacaa aaatcagcca ggcttcatgg cacatgccta tagtcccagc 780
   tacaggcatg ctgaagcagg agaatcgctt gcaccccgga ggcagaggct gcagtgagct 840
   gagaccacac cactgcactc cagcctaggc aacagagtat gagactccat ctcaaaaaaa 900
   aaaaaagtac ctacctcaga gttcaaacta gtgaatatta ggaagtgctt gagacagtga 960
   caccaaagtg cacaataaat actcgccagt ttcattatta ttaaagaatc catttgaatg 1020
   tcagctcaac acagcctcct ataccgaggc attgtgaacc gcatctcccc agcttctcca 1080
   ggcttttcca agaatcaggg acactgtagc ctgttggtct cagtgtatga cagacacgga 1140
   ggaagcacat ctttagctga tacttaaaca gagaccctga gcgcacatac acccgcgcac 1200
   acatgcatgg agcttcacct tctctgtcat tctgcagtga ccaggagagc aagagctccc 1260
   acctcccttc aaaacactgt gcccatcccg ggcactaagg cctctttaaa gcacggcacc 1320
   tccacgaggg agggccacag ccacatacac tccacctggc aggtggacag cgtgagcacg 1380
   tggaccatag cagggacaag gtgccccggc cagccccaac gccctctgcc gctgacaggg 1440
   acagaagccc tctccagctg cgtgtgctgc agaggccatg cgtagcctcc agctgcattc 1500
   tattccactc cagtgcctgg gccagttagc accagtgtgg aagacagtga gctggctccg 1560
   gacaacaggg atggaggaaa ggtcccacat tcacattcct gatacgtgga caaggtgagg 1620
   ggccgcaatc gctctggcag cattttaaag atggggaagt agcagacacc cacgcgtgaa 1680
   ggcaggagag ccccaactgt ggtggaaatg gccccagaat ggtagggcca agcctagctc 1740
   cagacacccc agagccctgg agaagccaag actgagggag aaagcctgag ggaggagcgc 1800
   cccagtcccc agggaccggc ctggtgcaga gctgcagctg atgttcccct ctgtgcagcc 1860
   ccaccctctg cctcgctgag ctccctgctg cgagggcctc gggtgcaagg gggaggcagg 1920
   tctctatctc atggagctgt cagatgagac atcgcgatcg gagtcctcag cctcgcttgg 1980
   cggcggcggc gggtcgctaa gcgggaccgc agtgaaagca ggagactttc tagaaaaaaa 2040
   caccagttgt caaccttggg gcaggcagga atcctgaaga cggacggcac tcctcctcct 2100
   gctgcctcac cctctggcag cccgtgagaa gtaccggaag cgagggcggg gccgcgggat 2160
   ggcgagggag cggcagggac tgaactctct ccaaacccac cctgacaggg aaatgggccc 2220
   cgcctgtgtc ttgggaactc agaggctgag gtcaggcatg atggctcacg cctgtaattc 2280
   cagcactttg ggaggcagag gcgggtggat cacgacgtca ggagttcaag gcaagcctgg 2340
   ccaacatggt gaaaccccat ctctactaaa aatgcaaaaa ttagccaggt gtggtggcgg 2400
   gcgccttgga ggctgaggca agataatcac ttgaacctgg gaggcggagg ttgtagtgag 2460
   ccaaaaaaaa aaaaaaaaga aatagctgaa gtcacagtag gagagaagct gctgagcctc 2520
   cagcaccctg actctagggc cttggcttta tgtctatctg cagtattttt gtgattttta 2580
   aaaattcact ttcttgttgc ggtgtaactt acacagggtc aaatgcacaa atcatggccc 2640
   tgactttaga taaaaatctg cccccacaac ccttctgttc cttgccagtt tttaaactgc 2700
   ctctaaccag gggaaccacc agagctggtg gccttgggag gtttcagccc tcccgtcatg 2760
   aatggacata gctcatccaa ctgccaaggg agagagctgt gggtctgggc cagccccacc 2820
   aggtaactcc caaagggcag ccccacagca agatgtgacc cagtcattgc ctgagggtct 2880
   ctggggctgt gttccaacct ttctccccgc tgtgtccccc tggaaggccc catgcccagg 2940
   ggaggcgcct accggtctcc agactgggtg atgagccggc ggcaggtctc catctgcacg 3000
   tccaggccgc gcttcatgct gcacatctcc atgtactcgt gcaggtgccg gttcatgtcg 3060
   ttcttggccg tggccaactc cagctgtggt ggggcaggca gggccatcgg ggttaacagg 3120
   tggcgttcac agcgcctctg ttgcccccgc caggaggcca acacgccaag agcagtggct 3180
   gggccggggg cccaggcagc cattactgaa ggctcaga 3218
<210> 22
   <211> 508
   <212> DNA
   <213> Homo sapiens
<400> 22
   agcttgagat tgtccaagca ggtagccaga gagcgccatc agccaagaaa ccatccactg      60
   gtacgtaagg cagcctgtgc gggcgagacc agactgggcc ctcccctcct gcagtgattt     120
   gtttcttctt cttttttaaa tcacgttttc ctgccttttc taggttctag gtaccagcct     180
   ctggcttcta cagcctcaga caatgacttt gtcacaccag agccccgccg tactacccgt     240
   cggcatccaa acacccagca gcgagcttcc aaaaagaaac ccaaagttgt cttctcaagt     300
   gatgagtcca gtgaggaagg tatgatgctc ccgcctgttc ccggccgaga aggcacacag     360
   ctagggtgca gagggctggt ttccatagga cctgctgcgg gggcctgagt gtagatgctc     420
   tgccccactg ccgcagaagg gcctctcctg tacagcttgg attttatttc ttctgtgcgg     480
   tgtgggattg tctcacttgt tctctgat                                        508
<210> 23
   <211> 2650
   <212> DNA
   <213> Homo sapiens
<400> 23
   atctattttt tcaccatctt tgtgactcag ctttttctta ttcctttaat tctttgcata 60
   gatctttcag cagagatgac agaagacgag acacccaaga aaacaactcc cattctcaga 120
   gcatcggctc gcaggcacag atcctaggaa gtctgttcct gtcctccctg tgcagggtat 180
   cctgtagggt gacctggaat tcgaattctg tttcccttgt aaaatatttg tctgtctctt 240
   ttttttaaaa aaaaaaaagg ccgggcactg tggctcacgc ctgtaatccc agcactttgc 300
   gataccaagg cgggtggata acctgaggta gggagttcga gaccagcctg accaacatgg 360
   agaaacccca tctctactaa aaataaaaaa ttagccgggc gtattggcgt gcgcctgtaa 420
   tcccagctac tcaagaggct gaggcaggag aatcgcctga acccagaggc ggaggttgta 480
   gtgagccgaa atcacaccat tgcactccag cttgggcaac aatagcgaac ctccatctca 540
   aattaaaaaa aaaatgccta cacgctcttt aaaatgcaag gctttctctt aaattagcct 600
   aactgaactg cgttgagctg cttcaacttt ggaatatatg tttgccaatc tccttgtttt 660
   ctaatgaata aatgttttta tatactttta gacatttttt cctaagcttg tctttgtttc 720
   atctttcaca ttagcccagt ttcatgcagc agagagaggg ttatcagtgc agagagagat 780
   gagtgagccc agagtcctag ggcctgtccc gggatggcag atgagcttcc tgccccgtca 840
   ctgccacctt tcccctctca acctctggac cctgcacagt gaccagacag cctctctggg 900
   gagaattatg cagtgcctag gctccagatc agtgcttctg aaccgggggc aattttgtct 960
   gccagaggac atctgacaac acctggggcc tgttttgttg tcatagccta taggggaaga 1020
   atgctaccag catttgtggg aagaggccag ggatgtggct caacatcctg cagtgcacag 1080
   gatggcccct caacaaagaa tcacacggcc cacaatgtca atagcgtcac agttgagaaa 1140
   acctgctcta gaccaagggt tgctttctgc cgtgtgcctc accccacccc cactcgtgtt 1200
   ccctaatccc atctccaaag gttggcagca gaccggccca ggctcgtgga agttcagatc 1260
   atgatcccct ccagctctgc aggagacaag acctgtctcc cagcattcct cattgttccc 1320
   gggtctgcag agggcgtgag ctatgctgca ggcgggctgc cccctgaagc ctgcgcaccc 1380
   ctctccagct cctcaagtct tctctgctga gtcaccttcg aaccggaggc tgtgagctgg 1440
   ctgtcgtgac cacactggtg cctctgctgt catgacaaca gcacactacg tcagtagtgc    1500
   tccctgggca ctgagctccc tctttgcggg gagaagacag taatgaaaaa tgacaagcat    1560
   gaggcagagg ggaagatcac gcttgggtgg tgcaggagca tggaggtgct cttaatgctc    1620
   tcaatgagaa agggttaacg gtcctggttg caggaatagc tgagtcagag gtggggcttc    1680
   ctccactccc ccaccccacc cctttcacca ttagggacct tcttgccttg ctcttgctac    1740
   tctgctctgg gtggtcattg tgaaaagccc gcaccaacca tgccagtggc agccagacga    1800
   ggacacagcc tggctctggg tcccagcagg aaaggcaatc ccagaaaggc agggtcaggg    1860
   actggagtcc tgtgggtgct ttttaagcaa agattatcac caggcaggct aaacttagca    1920
   accggctttt agctagaagg gcagggggct ggtgtcaggt tatgctgggc cagcaaagag    1980
   gcccgggatc cccctcccat gcacctgctg atgggccaag gccaccccac cccaccccct     2040
   tccttacaag tgttcagcac cctcccatcc cacactcaca aacctggccc tctgccctcc    2100
   taccagaaga atggatcccc tgtgggaggg ggcaggggac ctgttcccac cgtgtgccca    2160
   agacctcttt tcccactttt tccctcttct tgactcaccc tgccctcaat atcccccggc    2220
   gcagccagtg aaagggagtc cctggctcct ggctcgcctg cacgtcccag ggcggggagg    2280
   gacttccgcc ctcacgtccc gctcttcgcc ccaggctgga tggaatgaaa ggcacactgt    2340
   ctctctccct aggcagcaca gcccacaggt ttccaggagt gcctttgtgg gaggcctctg    2400
   ggcccccacc agccatcctg tcctccgcct ggggccccag cccggagaga gccgctggtg    2460
   cacacagggc cgggattgtc tgccctaatt atcaggtcca ggctacaggg ctgcaggaca    2520
   tcgtgacctt ccgtgcagaa acctccccct ccccctcaag ccgcctcccg agcctccttc   2580
   ctctccaggc ccccagtgcc cagtgcccag tgcccagccc aggcctcggt cccagagatg    2640
   ccaggagcca                                                           2650
<210> 24
   <211> 1963
   <212> DNA
   <213> Homo sapiens
<400> 24
   agcttgagat tgtccaagca ggtagccaga gagcgccatc agccaagaaa ccatccactg      60
   gtacgtaagg cagcctgtgc gggcgagacc agactgggcc ctcccctcct gcagtgattt     120
   gtttcttctt cttttttaaa tcacgttttc ctgccttttc taggttctag gtaccagcct    180
   ctggcttcta cagcctcaga caatgacttt gtcacaccag agccccgccg tactacccgt     240
   cggcatccaa acacccagca gcgagcttcc aaaaagaaac ccaaagttgt cttctcaagt     300
   gatgagtcca gtgaggaagg tatgatgctc ccgcctgttc ccggccgaga aggcacacag     360
   ctagggtgca gagggctggt ttccatagga cctgctgcgg gggcctgagt gtagatgctc     420
   tgccccactg ccgcagaagg gcctctcctg tacagcttgg attttatttc ttctgtgcgg 480
   tgtgggattg tctcacttgt tctctgatat ctattttttc accatctttg tgactcagct 540
   ttttcttatt cctttaattc tttgcataga tctttcagca gagatgacag aagacgagac 600
   acccaagaaa acaactccca ttctcagagc atcggctcgc aggcacagat cctaggaagt 660
   ctgttcctgt cctccctgtg cagggtatcc tgtagggtga cctggaattc gaattctgtt 720
   tcccttgtaa aatatttgtc tgtctctttt ttttaaaaaa aaaaaaggcc gggcactgtg 780
   gctcacgcct gtaatcccag cactttgcga taccaaggcg ggtggataac ctgaggtagg 840
   gagttcgaga ccagcctgac caacatggag aaaccccatc tctactaaaa ataaaaaatt 900
   agccgggcgt attggcgtgc gcctgtaatc ccagctactc aagaggctga ggcaggagaa 960
   tcgcctgaac ccagaggcgg aggttgtagt gagccgaaat cacaccattg cactccagct 1020
   tgggcaacaa tagcgaacct ccatctcaaa ttaaaaaaaa aatgcctaca cgctctttaa 1080
   aatgcaaggc tttctcttaa attagcctaa ctgaactgcg ttgagctgct tcaactttgg 1140
   aatatatgtt tgccaatctc cttgttttct aatgaataaa tgtttttata tacttttaga 1200
   cattttttcc taagcttgtc tttgtttcat ctttcacatt agcccagttt catgcagcag 1260
   agagagggtt atcagtgcag agagagatga gtgagcccag agtcctaggg cctgtcccgg 1320
   gatggcagat gagcttcctg ccccgtcact gccacctttc ccctctcaac ctctggaccc 1380
   tgcacagtga ccagacagcc tctctgggga gaattatgca gtgcctaggc tccagatcag 1440
   tgcttctgaa ccgggggcaa ttttgtctgc cagaggacat ctgacaacac ctggggcctg 1500
   ttttgttgtc atagcctata ggggaagaat gctaccagca tttgtgggaa gaggccaggg 1560
   atgtggctca acatcctgca gtgcacagga tggcccctca acaaagaatc acacggccca 1620
   caatgtcaat agcgtcacag ttgagaaaac ctgctctaga ccaagggttg ctttctgccg 1680
   tgtgcctcac cccaccccca ctcgtgttcc ctaatcccat ctccaaaggt tggcagcaga 1740
   ccggcccagg ctcgtggaag ttcagatcat gatcccctcc agctctgcag gagacaagac 1800
   ctgtctccca gcattcctca ttgttcccgg gtctgcagag ggcgtgagct atgctgcagg 1860
   cgggctgccc cctgaagcct gcgcacccct ctccagctcc tcaagtcttc tctgctgagt 1920
   caccttcgaa ccggaggctg tgagctggct gtcgtgacca cac 1963
<210> 25
   <211> 1195
   <212> DNA
   <213> Homo sapiens
<400> 25
   tggtgcctct gctgtcatga caacagcaca ctacgtcagt agtgctccct gggcactgag      60
   ctccctcttt gcggggagaa gacagtaatg aaaaatgaca agcatgaggc agaggggaag     120
   atcacgcttg ggtggtgcag gagcatggag gtgctcttaa tgctctcaat gagaaagggt     180
   taacggtcct ggttgcagga atagctgagt cagaggtggg gcttcctcca ctcccccacc     240
   ccaccccttt caccattagg gaccttcttg ccttgctctt gctactctgc tctgggtggt     300
   cattgtgaaa agcccgcacc aaccatgcca gtggcagcca gacgaggaca cagcctggct     360
   ctgggtccca gcaggaaagg caatcccaga aaggcagggt cagggactgg agtcctgtgg     420
   gtgcttttta agcaaagatt atcaccaggc aggctaaact tagcaaccgg cttttagcta     480
   gaagggcagg gggctggtgt caggttatgc tgggccagca aagaggcccg ggatccccct     540
   cccatgcacc tgctgatggg ccaaggccac cccaccccac ccccttcctt acaagtgttc     600
   agcaccctcc catcccacac tcacaaacct ggccctctgc cctcctacca gaagaatgga     660
   tcccctgtgg gagggggcag gggacctgtt cccaccgtgt gcccaagacc tcttttccca     720
   ctttttccct cttcttgact caccctgccc tcaatatccc ccggcgcagc cagtgaaagg     780
   gagtccctgg ctcctggctc gcctgcacgt cccagggcgg ggagggactt ccgccctcac     840
   gtcccgctct tcgccccagg ctggatggaa tgaaaggcac actgtctctc tccctaggca     900
   gcacagccca caggtttcca ggagtgcctt tgtgggaggc ctctgggccc ccaccagcca     960
   tcctgtcctc cgcctggggc cccagcccgg agagagccgc tggtgcacac agggccggga    1020
   ttgtctgccc taattatcag gtccaggcta cagggctgca ggacatcgtg accttccgtg    1080
   cagaaacctc cccctccccc tcaagccgcc tcccgagcct ccttcctctc caggccccca    1140
   gtgcccagtg cccagtgccc agcccaggcc tcggtcccag agatgccagg agcca         1195
<210> 26
   <211> 256
   <212> DNA
   <213> Homo sapiens
<400> 26
   cctctgggcc cccaccagcc atcctgtcct ccgcctgggg ccccagcccg gagagagccg      60
   ctggtgcaca cagggccggg attgtctgcc ctaattatca ggtccaggct acagggctgc     120
   aggacatcgt gaccttccgt gcagaaacct ccccctcccc ctcaagccgc ctcccgagcc     180
   tccttcctct ccaggccccc agtgcccagt gcccagtgcc cagcccaggc ctcggtccca     240
   gagatgccag gagcca                                                     256
<210> 27
   <211> 1941
   <212> DNA
   <213> Homo sapiens
<400> 27
   agcttgagat tgtccaagca ggtagccaga gagcgccatc agccaagaaa ccatccactg     60
   gtacgtaagg cagcctgtgc gggcgagacc agactgggcc ctcccctcct gcagtgattt    120
   gtttcttctt cttttttaaa tcacgttttc ctgccttttc taggttctag gtaccagcct     180
   ctggcttcta cagcctcaga caatgacttt gtcacaccag agccccgccg tactacccgt     240
   cggcatccaa acacccagca gcgagcttcc aaaaagaaac ccaaagttgt cttctcaagt     300
   gatgagtcca gtgaggaagg tatgatgctc ccgcctgttc ccggccgaga aggcacacag     360
   ctagggtgca gagggctggt ttccatagga cctgctgcgg gggcctgagt gtagatgctc     420
   tgccccactg ccgcagaagg gcctctcctg tacagcttgg attttatttc ttctgtgcgg     480
   tgtgggattg tctcacttgt tctctgatat ctattttttc accatctttg tgactcagct     540
   ttttcttatt cctttaattc tttgcataga tctttcagca gagatgacag aagacgagac     600
   acccaagaaa acaactccca ttctcagagc atcggctcgc aggcacagat cctaggaagt     660
   ctgttcctgt cctccctgtg cagggtatcc tgtagggtga cctggaattc gaaccggagg     720
   ctgtgagctg gctgtcgtga ccacactggt gcctctgctg tcatgacaac agcacactac     780
   gtcagtagtg ctccctgggc actgagctcc ctctttgcgg ggagaagaca gtaatgaaaa     840
   atgacaagca tgaggcagag gggaagatca cgcttgggtg gtgcaggagc atggaggtgc     900
   tcttaatgct ctcaatgaga aagggttaac ggtcctggtt gcaggaatag ctgagtcaga     960
   ggtggggctt cctccactcc cccaccccac ccctttcacc attagggacc ttcttgcctt    1020
   gctcttgcta ctctgctctg ggtggtcatt gtgaaaagcc cgcaccaacc atgccagtgg    1080
   cagccagacg aggacacagc ctggctctgg gtcccagcag gaaaggcaat cccagaaagg    1140
   cagggtcagg gactggagtc ctgtgggtgc tttttaagca aagattatca ccaggcaggc    1200
   taaacttagc aaccggcttt tagctagaag ggcagggggc tggtgtcagg ttatgctggg    1260
   ccagcaaaga ggcccgggat ccccctccca tgcacctgct gatgggccaa ggccacccca    1320
   ccccaccccc ttccttacaa gtgttcagca ccctcccatc ccacactcac aaacctggcc    1380
   ctctgccctc ctaccagaag aatggatccc ctgtgggagg gggcagggga cctgttccca    1440
   ccgtgtgccc aagacctctt ttcccacttt ttccctcttc ttgactcacc ctgccctcaa    1500
   tatcccccgg cgcagccagt gaaagggagt ccctggctcc tggctcgcct gcacgtccca    1560
   gggcggggag ggacttccgc cctcacgtcc cgctcttcgc cccaggctgg atggaatgaa    1620
   aggcacactg tctctctccc taggcagcac agcccacagg tttccaggag tgcctttgtg    1680
   ggaggcctct gggcccccac cagccatcct gtcctccgcc tggggcccca gcccggagag    1740
   agccgctggt gcacacaggg ccgggattgt ctgccctaat tatcaggtcc aggctacagg    1800
   gctgcaggac atcgtgacct tccgtgcaga aacctccccc tccccctcaa gccgcctccc    1860
   gagcctcctt cctctccagg cccccagtgc ccagtgccca gtgcccagcc caggcctcgg    1920
   tcccagagat gccaggagcc a                                               1941
<210> 28
   <211> 1433
   <212> DNA
   <213> Homo sapiens
<400> 28
   atctattttt tcaccatctt tgtgactcag ctttttctta ttcctttaat tctttgcata      60
   gatctttcag cagagatgac agaagacgag acacccaaga aaacaactcc cattctcaga     120
   gcatcggctc gcaggcacag atcctaggaa gtctgttcct gtcctccctg tgcagggtat     180
   cctgtagggt gacctggaat tcgaaccgga ggctgtgagc tggctgtcgt gaccacactg     240
   gtgcctctgc tgtcatgaca acagcacact acgtcagtag tgctccctgg gcactgagct     300
   ccctctttgc ggggagaaga cagtaatgaa aaatgacaag catgaggcag aggggaagat     360
   cacgcttggg tggtgcagga gcatggaggt gctcttaatg ctctcaatga gaaagggtta     420
   acggtcctgg ttgcaggaat agctgagtca gaggtggggc ttcctccact cccccacccc     480
   acccctttca ccattaggga ccttcttgcc ttgctcttgc tactctgctc tgggtggtca     540
   ttgtgaaaag cccgcaccaa ccatgccagt ggcagccaga cgaggacaca gcctggctct     600
   gggtcccagc aggaaaggca atcccagaaa ggcagggtca gggactggag tcctgtgggt     660
   gctttttaag caaagattat caccaggcag gctaaactta gcaaccggct tttagctaga     720
   agggcagggg gctggtgtca ggttatgctg ggccagcaaa gaggcccggg atccccctcc     780
   catgcacctg ctgatgggcc aaggccaccc caccccaccc ccttccttac aagtgttcag     840
   caccctccca tcccacactc acaaacctgg ccctctgccc tcctaccaga agaatggatc     900
   ccctgtggga gggggcaggg gacctgttcc caccgtgtgc ccaagacctc ttttcccact     960
   ttttccctct tcttgactca ccctgccctc aatatccccc ggcgcagcca gtgaaaggga    1020
   gtccctggct cctggctcgc ctgcacgtcc cagggcgggg agggacttcc gccctcacgt    1080
   cccgctcttc gccccaggct ggatggaatg aaaggcacac tgtctctctc cctaggcagc    1140
   acagcccaca ggtttccagg agtgcctttg tgggaggcct ctgggccccc accagccatc    1200
   ctgtcctccg cctggggccc cagcccggag agagccgctg gtgcacacag ggccgggatt    1260
   gtctgcccta attatcaggt ccaggctaca gggctgcagg acatcgtgac cttccgtgca    1320
   gaaacctccc cctccccctc aagccgcctc ccgagcctcc ttcctctcca ggcccccagt    1380
   gcccagtgcc cagtgcccag cccaggcctc ggtcccagag atgccaggag cca          1433
<210> 29
   <211> 17130
   <212> DNA
   <213> Cricetulus griseus
<220>
   <221> misc_feature
   <222> (4674)..(4912)
   <223> n is a, c, g, or t
<220>
   <221> misc_feature
   <222> (11347)..(13938)
   <223> n is a, c, g, or t
<220>
   <221> misc_feature
   <222> (14757)..(14870)
   <223> n is a, c, g, or t
<400> 29
   gtgagtggtg cagggtgact ggccctattg ggttcctgtt cagtgaacac agtctgttct 60
   tcgagtgctg tgggactctt aacttactgc cgtgctggcc tcactacagc catttaacta 120
   gagtgacttg agatcacttt ctatgtccac ttcccggtcc taaaaaaaaa atttattttg 180
   tatacattag tattttgcct gtgtgtatgt ccatgtgaag gtgccagatc tggaacttga 240
   gttacaggca gttgtgagct gccacatggg tgctgggaac tgaacccagg tcttctgcag 300
   gagcagcaag tgccatctct ctagccccca gtaaaagccg atttctagtt gagacatggt 360
   ctttctgtgt agctctagct ggcttagaat ttgctaccta gaccaggctg gccccaaatt 420
   cagattcccc tgcctctgcc tctcatgtgc tggtaggaaa ggcgtgagtc acatgcttgg 480
   cactagacta gctttgtgtt tagagcaaaa ctaagtaagt agagttcccg tttgtccctg     540
   tcctctgccc catcatcttc atacccttgg gtactgtgat cttacaatct acagtgacct     600
   ggatgaatga gtgtgagtga caatgaatga cagagcagta cccagagtcc acagttcaca 660
   ctgatactgg gttcactgta cagtggcaca tacttgtcac cacagtgtca tactggacat 720
   tttcatgacc ctaaggccac atcacccctg tggtgtcact cagttgtaat ttcaccctat 780
   atagtgattt caggttggct ttttacttag aatataacat ttaaattatg tttctttttt 840
   ggttttgagt tttgtttttc gagacagggt ttctctgtat tgctttggag gttgtgctgg 900
   aacttgctct ggagaccagg ctggcttcga actcacagag atcctcccac ctctgcctcc 960
   cgagtgctgg gattaaaggc gtgcgccacc aatgccccag catctttctg tttctctgtt 1020
   tttaaagagg gagataatgt ttccagtgct gtttttaact tttttcctgt aggctaccat 1080
   agatgaattt gagcagaagc ttcgggcatg tcataccaga ggcatggatg gaatagagga 1140
   gcttgaaact ggccaagaag gcagccagca agccctgtct gccaagaaac cctctgctgg 1200
   tatgagagac aagcccacac ggactcccca caaatcatcc ctgtcattta ttggtttttg 1260
   tttttaaaat cacatctccc tgccatttct agtctcaagg cgacagcctc tgacttctat 1320
   agattcagac aatgactttg tcacacctaa gcctcgacgc accaaccgtc atcgtccaaa 1380
   cactcagcaa cgaaagtcca agaagaaagc caaagttgtc ttttcaagtg atgagtccag 1440
   tgaggatggt atgactcgcc ctcccggcag ctgcttgctt gccttggggt ctccttgggg 1500
   cttgcctggt tgtagagtca ttcttcctgg taatgtgttc cccactgtgc acgttggctc 1560
   acctttgcag ctctatgttt taagatccac tttacaccat ctttgtgact cagcttttgt 1620
   tcctataatt ctttgaacag aactttcagc ggaaatgacg gaagaagaga ctcccaagag 1680
   aaccaccccc atccgcagat catctgcccg aagacacagg tcttaggagt tgcctcacct 1740
   attcccatcc ctgctgtgca gggtatctta attgattttt aattcatttc ctcccttgta 1800
   aaaatgttgc ctccttgttt ttaatatgta aatggtcttt gcattcaagg cctttctcct 1860
   agagcccatc tgactgctgc tttcactttc caacaaacac ttaaccaatc accatgtttt 1920
   ctaagaataa atgtttttat atacttttat tctttgctgg tctgtttcat cttccacctt 1980
   agcccagatt caggcagcaa tagagggtta tcaaaccaga gagaagatac accagcccag 2040
   gcatcctgag gctgtctctg ggtcttcatg tttcctcatt gattcatctt gcacaactga 2100
   tgagactcca ctgtagagaa ttctcacttc tcagcctggg ggtactttct acccagagac 2160
   ctatttctgt catagccttt tgggacacat aaagcttcct tcctgcatag aacaccccac 2220
   aacagtgtat caggagtgta caagttgaca aacaatgctc tagacctacg gttttcttcc 2280
   tctgtgtgcc tcatcccaga agagatcatg actcccagga gtcagccttt actatggggt 2340
   ctgcaggggc gtccagcccc tcagcggcaa gccatgccca ccctccccaa gtccttaatc 2400
   tgctgagtca cttggaacag gagacactga ttctgctgtc atgacaacag cacattgcca 2460
   tagaaatgct ccctacctct tacgtgtgtg gtgggggaac agtaatgaca aaccataggc 2520
   aggaggcaaa agggaagacg gcacctcaga aacatgtgtt aggttagggc agaactatgg 2580
   aggggctcct gagactcttt gatgggaaag ggttaatgct gctcctgaaa cctctgttgg 2640
   aaggcagaaa agggacaggg ctgagtcccc gcactgggac catttccatc ctctgcatcc 2700
   tgcccccggc tcatggaaag cctgggcatg ggccacacag ctgtcagtct tggctctggg 2760
   gccccaagga ggtagggcaa tcccagaatg gcaaggagcc aggactggat ttggggtgca 2820
   gcccagcctg ctccctgcct tttaagcaaa ggttatcacc aggccagcta aacttagcaa 2880
   ttaggctctt cagctaaaag agcagggggc tggtctcaag ttgcactgac ctagcaaaga 2940
   ggccccagga tccccctgcc cagcacctgt ggctgagctc ccaagccctt cccgagagct 3000
   caggatccac cctttccacc ctccctactc ttcagaggag gaaccccctt tctccttccc 3060
   acttgttgga gggggctggg gccaggctgt tctggcttgg ggtataatac cccctacccc 3120
   ttctactttc ccctcctctc agacctcacc ctgcctccac gagggcagcc aagaaaggag 3180
   agtccctggc tgcagggcca gtaggcacgt cccaggacgg ggagggactt ccgccctcac 3240
   gtccagctct ccgccctggg gctgcagtgg gtgaaagggg cagtgtctcc tagcctgggc 3300
   ggtgcaaccc tcaggttccg aggaggaacg ctctgggagg cttctttgcc tcctccaacc 3360
   caacccacaa ccaggacatt gtcctcaccc cggggcccca acctagacct taactgagga 3420
   acacagaggc cagtttgtaa gtctcaatta tgcagggcat cccgacctgt ggcgtaggga 3480
   gcgcccctcc aggccgcttc cctagcctcc tcctggccct cacagcccag gcctctggcc 3540
   caagaaatgg aagtgggggt gggggatgga actgcgaatg cgaagggccc ccgcaggagg 3600
   caaagtgacc cctcccgggc cttttctgct ccgagacttg tttttgcctg tgtcactacc 3660
   gaagaaccac gagaagatcc tcaacttttc cacagccttt gcataaaggg gagagggtcg 3720
   gcggtgcagc tgtggcacac acgcacttct gctcaacccg cccccccccg cccccgttcc 3780
   tgttccttcc caggttctcc ccattttatc ggggcggcaa cttttaggtc cctgggtcct 3840
   ggaagtcctt agtacacact cttcgtcctt aagtccatag tctgtattcc ctcggtccta 3900
   tcctgtcccc catcaccggg tcacctcccc agcgaagcaa tctcagttcc cctccccctc 3960
   tcagccccga gcccacacgt ttggtgcgtg cacatttcaa aaacgaggcg ggtccaaaga 4020
   gagggggtgg ggaggtgccg agtggcccag ctactcgcgg ctttacgggt gcacgtagct 4080
   caggcctcag cgcccttgag ctgtgactgg atggatgagc ggggcgggag gcggggcgag 4140
   cgtcctcggc gctccccacc accccagttc ctataaatac ggactgcagc cctccccggt 4200
   gctctctgct cctccctgtt ctagagacag ccgcatcttt ccgtgcagtg ccaggtgaaa 4260
   accgcagagt gggccgcagg tggccgggga cggtcggaaa cggggaaggg gggcgctcag 4320
   cccgggactg cgggcgctgg ggcgagctcc actgcccgag cccgggctcc gcattgcaga 4380
   ggctggaggg ggaagggatg gggggcgcgg cggggggcac gctgacctct ctttcttctc 4440
   tctccctccg cagcctcgct ccggagacgc aatggtgaag gtcggcgtga acgggtgagt 4500
   tcgtggctgg gctagggtgg ggctccgggt cccgctccgt cgcgtatgca ggtctacccc 4560
   accccggggc tctgcgggag cgtggggtgg ccggtgggtg gccgcagcac ccaaggagac 4620
   ctcaaggtca gcgagccacc tcctcccttg cggggatgag cagcgcggag tccnnnnnnn 4680
   nnnnnnnnnn nnnnnnnnnn nnnnnnnnnn nnnnnnnnnn nnnnnnnnnn nnnnnnnnnn 4740
   nnnnnnnnnn nnnnnnnnnn nnnnnnnnnn nnnnnnnnnn nnnnnnnnnn nnnnnnnnnn 4800
   nnnnnnnnnn nnnnnnnnnn nnnnnnnnnn nnnnnnnnnn nnnnnnnnnn nnnnnnnnnn 4860
   nnnnnnnnnn nnnnnnnnnn nnnnnnnnnn nnnnnnnnnn nnnnnnnnnn nnaggtcggt 4920
   ggcaccgggc cgtgcggtgc ccgctttagc gcatccatca tctcccaagg gcttccttta 4980
   gggtggctgg ccgccgccat gttgcaaacg ggaaggaaat gaatgaacca ccgttaggaa 5040
   acctcccttc ggccttcctc cttcctagcc cgtgactaac ctccccactc cctccccggg 5100
   tggagtcgcc tctgtactgt aagccaggtg atgcaaggct tccgtgctct cgagagagct 5160
   ctacctcgcc agctgtctca tattattagc ctcaaagcag ccctcaagcc tcatttacct 5220
   tgagcatatg atatattttg tagattctct gagaatcgaa gcggacttgg agaggtctgc 5280
   ttgtccttct cccagcccaa aggtggtagc tatggcgtag cgccggaggg gggagtgggg 5340
   ggggagctga gtcatggtgg ttctgaaaag aaaatttcca ccacaaaatg gctccggtgc 5400
   tagcatcccc ttccccccat aacctctgct tcccatcaca ccctgaccca aaccctgtag 5460
   gccagactgt aaaggtcact aagaggattg agtgtctgag cctcggaacc ctgcccttct 5520
   ccccatccca tcctctggaa accagatctc ccccgctcca ccctaatctg aggttatatt 5580
   tagccggctg acctttcagt atttggggtc tgggccccta cacacatctg ttgctcctgc 5640
   tcctgatttt tagctagcaa attcaagtgc tttgcaaatt agagcccagg gattaggggt 5700
   tggaaagctc agtggttttc tcagtctttc cctttagggg gagggacttg gaggaagcag 5760
   gtgggccgac ccctgtccta ctcattctga cctttaacct tgccctttga gcttgatgat 5820
   gctgagtgca cgagttcttc ctgtccaggg ggtgtagcct gaagccaggc caggctagaa 5880
   caaacttccc agggggtggg ggtagtgaat gccttgtgcc cacacagggg cacactgcca 5940
   cctcttggag acttgaaatg actggtgggg gggttggaca aggctttgag cccaatcacc 6000
   tcttggacag gaaagtaacc cccactttat ggccctgctg taaaagccca gtcaaacctc 6060
   atttgtccaa ggaagataga cctcttgggg cttcctaagg ataggggtgt tctatatttg 6120
   ggccctgctt ctaagcattc agccagcttt attaaaggaa attcataaca aaacttgaat 6180
   ttcctgcttc ttaaatacta atagtgtgct ggatctccat taaaaatgct gtcttgcaca 6240
   gtaggctatg gtttctgtgg gctctctaca gctatgggac aactggattc tgttttctga 6300
   agggcatgtg tcagctcagt actgactata gacctatgag ttctctgacc ccctaactca 6360
   cctttttttt tcttgcctca gatttggccg tattggacgc ctggttacca gggctgcctt 6420
   cacttctggc aaagtggaag ttgttgccat caatgacccc ttcattgacc tcaactacat 6480
   ggtctacatg ttccagtatg actctaccca tggcaagttc aaaggcacag tcaaggctga 6540
   gaatggaaag cttgtcatca acgggaaggc catcaccatc ttccaggagc gagatcccgc 6600
   caacatcaaa tggggtgatg ctggcgccga gtatgttgtg gaatctactg gcgtcttcac 6660
   caccatggag aaggctgggg cccacttgaa gggcggggcc aagagggtca tcatctccgc 6720
   cccttctgct gatgccccca tgtttgtgat gggtgtgaac caagacaagt atgacaactc 6780
   cctcaagatt gtcaggtgag gatggcagag ggctgtggca aagtgggcaa gcaggggcaa 6840
   ggttacaggt gggcgagcct cctaacctgt ctcttctctt cagcaatgcg tcctgcacca 6900
   ccaactgctt agcccccctg gccaaggtca tccatgacaa ctttggcatt gtggaaggac 6960
   tcatggtatg tagttcatct gtttcatcct gccagcagtg ggcgctgtgg tgggggccct 7020
   gcaagacctc actccctgcc tctgtgtctt tcagaccacg gtccatgcca tcactgccac 7080
   ccagaagact gtggatggcc cctccgggaa gctgtggcgt gatggccgtg ggctgcccca 7140
   gaacatcacc cctgcatcca ctggcgctgc caaggctgtg ggcaaagtca gcccagagct 7200
   gaacgggaag ctgactggca tggccttccg tgttcctacc cccaacgtgt ccgttgtgga 7260
   tctgacatgt cgcctggaga aacctgtatg tctggggtgg gctgagggtt gtctctagtg 7320
   gtgaggttgg ggcttgagta gtcaccttga tttttgccct taataggcca agtatgagga 7380
   catcaagaag gtggtgaagc aggcatctga gggcccactg aagggcatcc tgggctacac 7440
   cgaggaccag gttgtctcct gcgacttcaa cagtgactcc cactcttcca cctttgatgc 7500
   tggggctggc attgctctca atgacaactt tgtaaagctc atttcctggt atgtgggatc 7560
   agaaacagtc ttttaaaagt aacttggggt ttgtcacccc ttggtgtcta actcaaaaag 7620
   taaacccttc ctaactgccc tcccaatgtg ttctaggtat gacaatgaat ttggctacag 7680
   caacagagtg gtggacctca tggcctacat ggcctccaag gagtaagaag cccaccctgg 7740
   accatccacc ccagcaagga ctcgagcaag agggaggccc tggctgctga gcagtccctg 7800
   tccaataacc cccacaccga tcatctccct cacagtgtcc atcccagacc cccagaataa 7860
   ggaggggctt agggagccct actctcttga ataccatcaa taaagttcac tgcacccatc 7920
   ttccttggcc tttcaatgta aggttggggg agggaggctg tgacctagca aggttgggaa 7980
   tcctctgtgt cacttttcaa acagggcact agccacatgc cagcccaggt ttcctgtcct 8040
   gaacagatga aaattcacct aaggtgtctt ggtgctggga ggagtggggg ttgacaatgg 8100
   gaccagtagt atggtcttag aggcttgggc tggactgcat caagttccag ggctgtgtgt 8160
   gtgttatctg caaacaaagg tcaattgtgt ctggaggcct taggtaaaat tggaaggatg 8220
   cccaacatag taaaaatgta tcagccaggg gaagtgacta cactgtatct aacctgaaac 8280
   agctgagctg taagccagca gctgtcacta tgttcaggtg tggtccgctg gttctggggt 8340
   ggtcacttgt atccagtttg ttaggaagtg ttgtcattgc ttgttaggaa gacaacacat 8400
   ctcaggctgg gcagtggtag tgcgtgcctc taatgccagc attcccagca cggaagtggc 8460
   agaggcagga ggacagcctg gaataacaac ccagggcaga gcccccatct cggaaaagac 8520
   aaaaaccaga aagtgcttaa aacattgaca caaaggtgct caaatattcc ttcattgctt 8580
   ttagagattc cactgtcagc ttggcatggc ctctagtgag acatcctgac ttggtcccct 8640
   gctttccaag gtcaggagaa tgatagccac agaacgttcc ctcagctgat ggctggagaa 8700
   ccggggtccc tgagccccca ccctcacacc catgtgcagg agggagcttc acctttccct 8760
   ccgagcagtg tctgccttca ggacaccgct ctcagcccag acactgagtc ttctttgtgt 8820
   gcatttcccc ggggaaaagc tacacattcc ctgtacagca ggcaggaggg cagctgtgag 8880
   ctcatggaca cgggacagaa ggccaggtac cctgcctccc tgcggtaccc cgcctccctt 8940
   cgaggcctta ctaggaagca aagccttctc caggtgtacg tgatgcggaa agtctgcaga 9000
   aagtcgggga ctcccagatg agtctgctgc acagtgcctg ggccagttag caccagaaca 9060
   aaagtccaag atagccaagg agaacaggca ggtcccacaa tcacattcct tacaaacgga 9120
   gaagggagga tcacaagtgt tctggcagcg tttcaaagat agagaagtag caaacagtca 9180
   gagtgaagaa ggcaggagtg tcccaatgtc atggggatat ccaggaaggg tggggccaag 9240
   cctagctcca gacacccagg gagcccgggc gaggagacag ccccagggcc aggcttcatg 9300
   cagtggcaca aaagaccccc accccggcct cggtgagctc cagggagcca ggcactggtc 9360
   cctatctcat ggagctatca gatgagacat ggcaatcgga gtcctcagtt tcgcttggcg 9420
   ctggcggcgg gtcgctaagc gggaccgcag tgaaagcagg agactttcta gaaaaaaaca 9480
   ccagatgtca ccttggggca agcaggaagc ccaagcaggc ctgcatgctc gctctccttt 9540
   ccatggcctt atcttctggg gtgcagctgg atgccaggca gggaagtggg tgggaagact 9600
   tgtccccatt cacctctatg aggaacagtt cccctcttgg aggctctgag aggctcagag 9660
   atcaaaaatg tctcaagtca cagcatggag atgcttcctg agcccacagt gtgctgacac 9720
   cagggccagc ttcttgtcca gctgtaaagg gggtggggct gtctcactat gttcccaagg 9780
   ctgaccccca acacttggta caaagcaatc ctcttgctcc agagtagctg ggatgacaag 9840
   caaaggccac taaacctagt gaacatgctg cacactgtgc ctagcttaat gtaattgggg 9900
   gagtgggttg gggttttgtt ttgagacagg gtctcagtat gcattcctgg ctggcctgga 9960
   attcacagag gagagaccgg cctggtactt gtggcaatct gtagggagac accgtaacca 10020
   cgcctcctaa gagctggctc atcagggtgt ggtcaaggtg atgtcagggt gacatgggag 10080
   aggtgtaaag ggataaggaa cgcacatggt agctcttttc cctgctgctt tggcttgctc 10140
   tgctttcctg ccggttggct acctaataaa tatatcctga ccatcacggg ctatgtatta 10200
   gtaattaatc ctaatagccg gtgtctccct acaacaatcc acctgcctgt gacacctatg 10260
   tgcttggatg acagatgtac accaccacac ctggcttact gtggttcctt agattttacc 10320
   tgttgttgct ggttgtttta ttttaagaca agtttttgct attgtagtcc aggatagcct 10380
   caaactcacg gcagtctttt gcttcagcct cccaaggctg ggcttgcaga catgtaccat 10440
   catgcctggg ttacacattt ttaatatata atttattttt attctgtgtt aattggtatt 10500
   tgcctgcata tatgtctgtg taaaggtatc agaagccctg gaactggagt tacagacact 10560
   cgtgtgggtg ctgggaattg aacccaggcc ctctggaaga gcatccagtg ttcttaacta 10620
   atgagccatc tctcagcccc caggtttttt tttttttttt tttactctgt gtgtatgtgt 10680
   gtgtgtgcac acgcacgcgc atgctcgaac atacaccagc acacagggtc tcacacaggc 10740
   tagacaagta ctctaccact gagctatatt ccccaaaccc aaatttactc ttacacaagg 10800
   tcaaatactt aaatcatgac ttagactttg gataaaaacc tgcccctgta tctcttcatt 10860
   cctagccagg tttcggctct cagacactgg ggcactggta ggagtcgtct gaccaccagg 10920
   gcagggagag gaagctatca atctgggcca cacctatagg tgactccaaa ggacagctcc 10980
   acttgcagat gtgacctggt caccggccac tgggctggga cgagggccaa tcagagcagg 11040
   tccttaccgg tcccccgact gcgtgatcag ggcaggtcct taccggtccc ccgactgtgt 11100
   gatgaggcgg cggcaggtct ccatctgcac gtccaagccc cgtttcatgc tgcacatctc 11160
   catgtactcg tgcaggtggc ggttcatgtc attcttggct gtggccagct ccagctgcag 11220
   aaaagtaggg cagggcagga tgaggaggtt agcaagccca aggcagacag gggattagag 11280
   cccagctggg ccccggaggc caggcagcca tggttaaaag ctcggccttc aaaagaaagc 11340
   aagcagnnnn nnnnnnnnnn nnnnnnnnnn nnnnnnnnnn nnnnnnnnnn nnnnnnnnnn 11400
   nnnnnnnnnn nnnnnnnnnn nnnnnnnnnn nnnnnnnnnn nnnnnnnnnn nnnnnnnnnn 11460
   nnnnnnnnnn nnnnnnnnnn nnnnnnnnnn nnnnnnnnnn nnnnnnnnnn nnnnnnnnnn 11520
   nnnnnnnnnn nnnnnnnnnn nnnnnnnnnn nnnnnnnnnn nnnnnnnnnn nnnnnnnnnn 11580
   nnnnnnnnnn nnnnnnnnnn nnnnnnnnnn nnnnnnnnnn nnnnnnnnnn nnnnnnnnnn 11640
   nnnnnnnnnn nnnnnnnnnn nnnnnnnnnn nnnnnnnnnn nnnnnnnnnn nnnnnnnnnn 11700
   nnnnnnnnnn nnnnnnnnnn nnnnnnnnnn nnnnnnnnnn nnnnnnnnnn nnnnnnnnnn 11760
   nnnnnnnnnn nnnnnnnnnn nnnnnnnnnn nnnnnnnnnn nnnnnnnnnn nnnnnnnnnn 11820
   nnnnnnnnnn nnnnnnnnnn nnnnnnnnnn nnnnnnnnnn nnnnnnnnnn nnnnnnnnnn 11880
   nnnnnnnnnn nnnnnnnnnn nnnnnnnnnn nnnnnnnnnn nnnnnnnnnn nnnnnnnnnn 11940
   nnnnnnnnnn nnnnnnnnnn nnnnnnnnnn nnnnnnnnnn nnnnnnnnnn nnnnnnnnnn 12000
   nnnnnnnnnn nnnnnnnnnn nnnnnnnnnn nnnnnnnnnn nnnnnnnnnn nnnnnnnnnn 12060
   nnnnnnnnnn nnnnnnnnnn nnnnnnnnnn nnnnnnnnnn nnnnnnnnnn nnnnnnnnnn 12120
   nnnnnnnnnn nnnnnnnnnn nnnnnnnnnn nnnnnnnnnn nnnnnnnnnn nnnnnnnnnn 12180
   nnnnnnnnnn nnnnnnnnnn nnnnnnnnnn nnnnnnnnnn nnnnnnnnnn nnnnnnnnnn 12240
   nnnnnnnnnn nnnnnnnnnn nnnnnnnnnn nnnnnnnnnn nnnnnnnnnn nnnnnnnnnn 12300
   nnnnnnnnnn nnnnnnnnnn nnnnnnnnnn nnnnnnnnnn nnnnnnnnnn nnnnnnnnnn 12360
   nnnnnnnnnn nnnnnnnnnn nnnnnnnnnn nnnnnnnnnn nnnnnnnnnn nnnnnnnnnn 12420
   nnnnnnnnnn nnnnnnnnnn nnnnnnnnnn nnnnnnnnnn nnnnnnnnnn nnnnnnnnnn 12480
   nnnnnnnnnn nnnnnnnnnn nnnnnnnnnn nnnnnnnnnn nnnnnnnnnn nnnnnnnnnn 12540
   nnnnnnnnnn nnnnnnnnnn nnnnnnnnnn nnnnnnnnnn nnnnnnnnnn nnnnnnnnnn 12600
   nnnnnnnnnn nnnnnnnnnn nnnnnnnnnn nnnnnnnnnn nnnnnnnnnn nnnnnnnnnn 12660
   nnnnnnnnnn nnnnnnnnnn nnnnnnnnnn nnnnnnnnnn nnnnnnnnnn nnnnnnnnnn 12720
   nnnnnnnnnn nnnnnnnnnn nnnnnnnnnn nnnnnnnnnn nnnnnnnnnn nnnnnnnnnn 12780
   nnnnnnnnnn nnnnnnnnnn nnnnnnnnnn nnnnnnnnnn nnnnnnnnnn nnnnnnnnnn 12840
   nnnnnnnnnn nnnnnnnnnn nnnnnnnnnn nnnnnnnnnn nnnnnnnnnn nnnnnnnnnn 12900
   nnnnnnnnnn nnnnnnnnnn nnnnnnnnnn nnnnnnnnnn nnnnnnnnnn nnnnnnnnnn 12960
   nnnnnnnnnn nnnnnnnnnn nnnnnnnnnn nnnnnnnnnn nnnnnnnnnn nnnnnnnnnn 13020
   nnnnnnnnnn nnnnnnnnnn nnnnnnnnnn nnnnnnnnnn nnnnnnnnnn nnnnnnnnnn 13080
   nnnnnnnnnn nnnnnnnnnn nnnnnnnnnn nnnnnnnnnn nnnnnnnnnn nnnnnnnnnn 13140
   nnnnnnnnnn nnnnnnnnnn nnnnnnnnnn nnnnnnnnnn nnnnnnnnnn nnnnnnnnnn 13200
   nnnnnnnnnn nnnnnnnnnn nnnnnnnnnn nnnnnnnnnn nnnnnnnnnn nnnnnnnnnn 13260
   nnnnnnnnnn nnnnnnnnnn nnnnnnnnnn nnnnnnnnnn nnnnnnnnnn nnnnnnnnnn 13320
   nnnnnnnnnn nnnnnnnnnn nnnnnnnnnn nnnnnnnnnn nnnnnnnnnn nnnnnnnnnn 13380
   nnnnnnnnnn nnnnnnnnnn nnnnnnnnnn nnnnnnnnnn nnnnnnnnnn nnnnnnnnnn 13440
   nnnnnnnnnn nnnnnnnnnn nnnnnnnnnn nnnnnnnnnn nnnnnnnnnn nnnnnnnnnn 13500
   nnnnnnnnnn nnnnnnnnnn nnnnnnnnnn nnnnnnnnnn nnnnnnnnnn nnnnnnnnnn 13560
   nnnnnnnnnn nnnnnnnnnn nnnnnnnnnn nnnnnnnnnn nnnnnnnnnn nnnnnnnnnn 13620
   nnnnnnnnnn nnnnnnnnnn nnnnnnnnnn nnnnnnnnnn nnnnnnnnnn nnnnnnnnnn 13680
   nnnnnnnnnn nnnnnnnnnn nnnnnnnnnn nnnnnnnnnn nnnnnnnnnn nnnnnnnnnn 13740
   nnnnnnnnnn nnnnnnnnnn nnnnnnnnnn nnnnnnnnnn nnnnnnnnnn nnnnnnnnnn 13800
   nnnnnnnnnn nnnnnnnnnn nnnnnnnnnn nnnnnnnnnn nnnnnnnnnn nnnnnnnnnn 13860
   nnnnnnnnnn nnnnnnnnnn nnnnnnnnnn nnnnnnnnnn nnnnnnnnnn nnnnnnnnnn 13920
   nnnnnnnnnn nnnnnnnnac acacacacac acacacacac acacacttac ttcctgagga 13980
   agactgcagg gaacaaggct agatagaacc cattattcca ggagacaaaa gtgaagcaaa 14040
   aatggtctct taaagcacaa agcagcacta ccaacagtgg tctcttccag cctatatatc 14100
   accctagttt acacattgaa gggaaagatg tacattctag aacattttca acaagtcagc 14160
   ccccaaattc cagactcagc atgggagatc agagcagtag aggtagatgg aggagataga 14220
   ggggccaggg ccatggagga aaactcccac ctgtcatcag ctttaccatc caataatggg 14280
   cagctctacc tcagtaacac tgagagatta agggccatac agacaaaagg aacttttgcc 14340
   acaaagctac caagtgaact ttctttacct ttctcctcaa gtcattcttc caggaaggac 14400
   agaagtcaca caacttagaa acaactttcc atccatgtgc agtatacata tttttatgtg 14460
   tgtggataca gatgtgcatg tgtgtggtta catggggaag cctgatgtcg atgcagggaa 14520
   tcttcctagg tggttcttat actctgagtc tgtgtaacgg cattggttgt cctggaactt 14580
   gatctgtaga ccagactggc ctcaaactca cagagatcta cctgtctctg cctcccgaat 14640
   gctgggatta aaagcgtgtg cctcagccgg acgttggtgg cgcatgcctt taatcccagc 14700
   actcgggagg cagaggcagg aggatctctg tgagttctag accagcctgg tctacannnn 14760
   nnnnnnnnnn nnnnnnnnnn nnnnnnnnnn nnnnnnnnnn nnnnnnnnnn nnnnnnnnnn 14820
   nnnnnnnnnn nnnnnnnnnn nnnnnnnnnn nnnnnnnnnn nnnnnnnnnn ctagttccag 14880
   ggcagcctcc aaagccacag agaaaccctg tctcaaaaaa caaaacaaaa ataaataaat 14940
   aaataaataa aatcctgcgc ctccactgcc cggctcagaa tatttttaaa gacaaggatt 15000
   atgtagccca ggctggcctc aaacttgtta tgtagctaag atgatggtgg cctctgatcc 15060
   tcctgcctct acctcccaag tgttaggact ataggctggc actaccacac ccggtttcat 15120
   gtgggctagg atcacactca ggcagggctt catgcacatt gggcaggcat tctaccaact 15180
   gagttacaca gtcccagccc aggatgtctc accagatcac ggttacagct aggcacctgt 15240
   ggtctgaggt ttgcaccaca tggcagcccc agtcactgag ctaaggtgaa actcatactt 15300
   gactaaaggc acggaatctg aaaggggtgg gtacaaccca ggaggaaaat cggcggaagc 15360
   aataagaggc gtcaacaggc aatggtcact tttccccaga caagtctcag ctggaagcag 15420
   cacacgggct gggtatctga aactcagaag tttcctgggt taaaaacaaa agccctggtt 15480
   tttcttagga tagtatgttc atttgatgtg tgctgggaca ctgagaacac aggaaaataa 15540
   cttccaaagt ttgttctcaa aatgcagaca gggtgaacct ttacagggct tgcagggagg 15600
   ctgatacaag aggtctcctg cctcattcac ccatcagagt ccaagacaca gtcctcacac 15660
   agccccaccc aaccaactgt cctccgacgg tgacctcctc cctacaaacc tgcctgcagc 15720
   ccagaaggac agtcagagcc ccagctccat agccatttgt aaaggactcc ccaggctcaa 15780
   aaacaaaagg gagactcctc caggagctca caggctgagc caaggaggca ggagtgctcc 15840
   ggcacttagg gtgtccgggt gataatgcac aatatgtatg atgtgtcaac ctgccagatc 15900
   ctgtcctaag cgccacgtgt ctatctgatc atcctaataa cccagcctgc ggggagagac 15960
   atgcacatct ccttctgaca aataaagagg caggcccaag cttagcatgg cagtacacat 16020
   ctgtaaccca gcactcaaca ggcagagcag gaggattgac tgcctcctat tccaggccag 16080
   tttggctaca cagaaagacc ctgtactgat gctaagagag atggctcaga gttaagagca 16140
   cttcctgctc ctggagagga cccaggtttg ctttccagca cccatgtcag gctcacagtc 16200
   gcctgtgaac accagctcca ggggatctga tgccctcttc tggcctccag gggcacgcgc 16260
   gtgtgcgctc acacacacac acacacacac acacacacac acacacacac acacacacac 16320
   acagagtgca aagctgaagt gcagctcaca cccataatcc tagcaaatgg agacaaaagg 16380
   accaggagtt cgaggccaac ctagactaca agacaagtac ttgtaaataa atacatatat 16440
   acgaaaataa acaaaccaaa aataatgaaa caggcactca gatcaaggga gccagtattg 16500
   gtggcacacg cctttaatcc cagcactcag gaggcagagg caggtggatc tctgagtttg 16560
   aggccagcct agtctacaga atgagatcca ggactacaca gagaaaccct gtctcaccct     16620
   cctctcccct aaaaaaaatt aagaaagaaa agaaagagcc aggggctggt aagcaatgga     16680
   tcagcaggta aaggcacaag ccaccaagcc caacaaccta aatttgatcc ctgggaccta     16740
   catggtagaa ggagagaact cactgcaagc tgtcctctga cctccacaca cacaccatgg     16800
   caggcacatg ctctccccgc cacatacata catacataca tacatacata catacataca     16860
   tacatacatg taaacagaaa agaaagtagg aagacaaaag gttcattcta agtggtgaca     16920
   gagccaagat ttgaacccac atctatctgc gtctagatcc tgaggtctca agggcccatt     16980
   agagctacca aaactggggt ggggtgcact cctcctgtca acaggtggaa ggtcctggtc     17040
   ttgggctctg gctgggagga ggccagccag cagcacacct ccagcctggt ttgtgagccc    17100
   tgggaacaga gcccctgcca aaccccttac                                     17130
<210> 30
   <211> 3020
   <212> DNA
   <213> Artificial
<220>
   <223> amplicon
<400> 30
   tacggccggc ttcactgtac agtggcacat acttgtcacc acagtgtcat actggacatt      60
   ttcatgaccc taaggccaca tcacccctgt ggtgtcactc agttgtaatt tcaccctata     120
   tagtgatttc aggttggctt tttacttaga atataacatt taaattatgt ttcttttttg     180
   gttttgagtt ttgtttttcg agacagggtt tctctgtatt gctttggagg ttgtgctgga     240
   acttgctctg gagaccaggc tggcttcgaa ctcacagaga tcctcccacc tctgcctccc     300
   gagtgctggg attaaaggcg tgcgccacca atgccccagc atctttctgt ttctctgttt     360
   ttaaagaggg agataatgtt tccagtgctg tttttaactt ttttcctgta ggctaccata     420
   gatgaatttg agcagaagct tcgggcatgt cataccagag gcatggatgg aatagaggag     480
   cttgaaactg gccaagaagg cagccagcaa gccctgtctg ccaagaaacc ctctgctggt     540
   atgagagaca agcccacacg gactccccac aaatcatccc tgtcatttat tggtttttgt     600
   ttttaaaatc acatctccct gccatttcta gtctcaaggc gacagcctct gacttctata     660
   gattcagaca atgactttgt cacacctaag cctcgacgca ccaaccgtca tcgtccaaac     720
   actcagcaac gaaagtccaa gaagaaagcc aaagttgtct tttcaagtga tgagtccagt     780
   gaggatggta tgactcgccc tcccggcagc tgcttgcttg ccttggggtc tccttggggc     840
   ttgcctggtt gtagagtcat tcttcctggt aatgtgttcc ccactgtgca cgttggctca     900
   cctttgcagc tctatgtttt aagatccact ttacaccatc tttgtgactc agcttttgtt     960
   cctataattc tttgaacaga actttcagcg gaaatgacgg aagaagagac tcccaagaga    1020
   accaccccca tccgcagatc atctgcccga agacacaggt cttaggagtt gcctcaccta 1080
   ttcccatccc tgctgtgcag ggtatcttaa ttgattttta attcatttcc tcccttgtaa 1140
   aaatgttgcc tccttgtttt taatatgtaa atggtctttg cattcaaggc ctttctccta 1200
   gagcccatct gactgctgct ttcactttcc aacaaacact taaccaatca ccatgttttc 1260
   taagaataaa tgtttttata tacttttatt ctttgctggt ctgtttcatc ttccacctta 1320
   gcccagattc aggcagcaat agagggttat caaaccagag agaagataca ccagcccagg 1380
   catcctgagg ctgtctctgg gtcttcatgt ttcctcattg attcatcttg cacaactgat 1440
   gagactccac tgtagagaat tctcacttct cagcctgggg gtactttcta cccagagacc 1500
   tatttctgtc atagcctttt gggacacata aagcttcctt cctgcataga acaccccaca 1560
   acagtgtatc aggagtgtac aagttgacaa acaatgctct agacctacgg ttttcttcct 1620
   ctgtgtgcct catcccagaa gagatcatga ctcccaggag tcagccttta ctatggggtc 1680
   tgcaggggcg tccagcccct cagcggcaag ccatgcccac cctccccaag tccttaatct 1740
   gctgagtcac ttggaacagg agacactgat tctgctgtca tgacaacagc acattgccat 1800
   agaaatgctc cctacctctt acgtgtgtgg tgggggaaca gtaatgacaa accataggca 1860
   ggaggcaaaa gggaagacgg cacctcagaa acatgtgtta ggttagggca gaactatgga 1920
   ggggctcctg agactctttg atgggaaagg gttaatgctg ctcctgaaac ctctgttgga 1980
   aggcagaaaa gggacagggc tgagtccccg cactgggacc atttccatcc tctgcatcct 2040
   gcccccggct catggaaagc ctgggcatgg gccacacagc tgtcagtctt ggctctgggg 2100
   ccccaaggag gtagggcaat cccagaatgg caaggagcca ggactggatt tggggtgcag 2160
   cccagcctgc tccctgcctt ttaagcaaag gttatcacca ggccagctaa acttagcaat 2220
   taggctcttc agctaaaaga gcagggggct ggtctcaagt tgcactgacc tagcaaagag 2280
   gccccaggat ccccctgccc agcacctgtg gctgagctcc caagcccttc ccgagagctc 2340
   aggatccacc ctttccaccc tccctactct tcagaggagg aacccccttt ctccttccca 2400
   cttgttggag ggggctgggg ccaggctgtt ctggcttggg gtataatacc ccctacccct 2460
   tctactttcc cctcctctca gacctcaccc tgcctccacg agggcagcca agaaaggaga 2520
   gtccctggct gcagggccag taggcacgtc ccaggacggg gagggacttc cgccctcacg 2580
   tccagctctc cgccctgggg ctgcagtggg tgaaaggggc agtgtctcct agcctgggcg 2640
   gtgcaaccct caggttccga ggaggaacgc tctgggaggc ttctttgcct cctccaaccc 2700
   aacccacaac caggacattg tcctcacccc ggggccccaa cctagacctt aactgaggaa 2760
   cacagaggcc agtttgtaag tctcaattat gcagggcatc ccgacctgtg gcgtagggag 2820
   cgcccctcca ggccgcttcc ctagcctcct cctggccctc acagcccagg cctctggccc 2880
   aagaaatgga agtgggggtg ggggatggaa ctgcgaatgc gaagggcccc cgcaggaggc 2940
   aaagtgaccc ctcccgggcc ttttctgctc cgagacttgt ttttgcctgt gtcactaccg 3000
   aagaaccacg gccggcctga 3020
<210> 31
   <211> 3537
   <212> DNA
   <213> Artificial
<220>
   <223> amplicon
<400> 31
   tactcgcgat tcactgtaca gtggcacata cttgtcacca cagtgtcata ctggacattt 60
   tcatgaccct aaggccacat cacccctgtg gtgtcactca gttgtaattt caccctatat 120
   agtgatttca ggttggcttt ttacttagaa tataacattt aaattatgtt tcttttttgg 180
   ttttgagttt tgtttttcga gacagggttt ctctgtattg ctttggaggt tgtgctggaa 240
   cttgctctgg agaccaggct ggcttcgaac tcacagagat cctcccacct ctgcctcccg 300
   agtgctggga ttaaaggcgt gcgccaccaa tgccccagca tctttctgtt tctctgtttt 360
   taaagaggga gataatgttt ccagtgctgt ttttaacttt tttcctgtag gctaccatag 420
   atgaatttga gcagaagctt cgggcatgtc ataccagagg catggatgga atagaggagc 480
   ttgaaactgg ccaagaaggc agccagcaag ccctgtctgc caagaaaccc tctgctggta 540
   tgagagacaa gcccacacgg actccccaca aatcatccct gtcatttatt ggtttttgtt 600
   tttaaaatca catctccctg ccatttctag tctcaaggcg acagcctctg acttctatag 660
   attcagacaa tgactttgtc acacctaagc ctcgacgcac caaccgtcat cgtccaaaca 720
   ctcagcaacg aaagtccaag aagaaagcca aagttgtctt ttcaagtgat gagtccagtg 780
   aggatggtat gactcgccct cccggcagct gcttgcttgc cttggggtct ccttggggct 840
   tgcctggttg tagagtcatt cttcctggta atgtgttccc cactgtgcac gttggctcac 900
   ctttgcagct ctatgtttta agatccactt tacaccatct ttgtgactca gcttttgttc 960
   ctataattct ttgaacagaa ctttcagcgg aaatgacgga agaagagact cccaagagaa 1020
   ccacccccat ccgcagatca tctgcccgaa gacacaggtc ttaggagttg cctcacctat 1080
   tcccatccct gctgtgcagg gtatcttaat tgatttttaa ttcatttcct cccttgtaaa 1140
   aatgttgcct ccttgttttt aatatgtaaa tggtctttgc attcaaggcc tttctcctag 1200
   agcccatctg actgctgctt tcactttcca acaaacactt aaccaatcac catgttttct 1260
   aagaataaat gtttttatat acttttattc tttgctggtc tgtttcatct tccaccttag 1320
   cccagattca ggcagcaata gagggttatc aaaccagaga gaagatacac cagcccaggc 1380
   atcctgaggc tgtctctggg tcttcatgtt tcctcattga ttcatcttgc acaactgatg 1440
   agactccact gtagagaatt ctcacttctc agcctggggg tactttctac ccagagacct 1500
   atttctgtca tagccttttg ggacacataa agcttccttc ctgcatagaa caccccacaa 1560
   cagtgtatca ggagtgtaca agttgacaaa caatgctcta gacctacggt tttcttcctc 1620
   tgtgtgcctc atcccagaag agatcatgac tcccaggagt cagcctttac tatggggtct 1680
   gcaggggcgt ccagcccctc agcggcaagc catgcccacc ctccccaagt ccttaatctg 1740
   ctgagtcact tggaacagga gacactgatt ctgctgtcat gacaacagca cattgccata 1800
   gaaatgctcc ctacctctta cgtgtgtggt gggggaacag taatgacaaa ccataggcag 1860
   gaggcaaaag ggaagacggc acctcagaaa catgtgttag gttagggcag aactatggag 1920
   gggctcctga gactctttga tgggaaaggg ttaatgctgc tcctgaaacc tctgttggaa 1980
   ggcagaaaag ggacagggct gagtccccgc actgggacca tttccatcct ctgcatcctg 2040
   cccccggctc atggaaagcc tgggcatggg ccacacagct gtcagtcttg gctctggggc 2100
   cccaaggagg tagggcaatc ccagaatggc aaggagccag gactggattt ggggtgcagc 2160
   ccagcctgct ccctgccttt taagcaaagg ttatcaccag gccagctaaa cttagcaatt 2220
   aggctcttca gctaaaagag cagggggctg gtctcaagtt gcactgacct agcaaagagg 2280
   ccccaggatc cccctgccca gcacctgtgg ctgagctccc aagcccttcc cgagagctca 2340
   ggatccaccc tttccaccct ccctactctt cagaggagga accccctttc tccttcccac 2400
   ttgttggagg gggctggggc caggctgttc tggcttgggg tataataccc cctacccctt 2460
   ctactttccc ctcctctcag acctcaccct gcctccacga gggcagccaa gaaaggagag 2520
   tccctggctg cagggccagt aggcacgtcc caggacgggg agggacttcc gccctcacgt 2580
   ccagctctcc gccctggggc tgcagtgggt gaaaggggca gtgtctccta gcctgggcgg 2640
   tgcaaccctc aggttccgag gaggaacgct ctgggaggct tctttgcctc ctccaaccca 2700
   acccacaacc aggacattgt cctcaccccg gggccccaac ctagacctta actgaggaac 2760
   acagaggcca gtttgtaagt ctcaattatg cagggcatcc cgacctgtgg cgtagggagc 2820
   gcccctccag gccgcttccc tagcctcctc ctggccctca cagcccaggc ctctggccca 2880
   agaaatggaa gtgggggtgg gggatggaac tgcgaatgcg aagggccccc gcaggaggca 2940
   aagtgacccc tcccgggcct tttctgctcc gagacttgtt tttgcctgtg tcactaccga 3000
   agaaccacga gaagatcctc aacttttcca cagcctttgc ataaagggga gagggtcggc 3060
   ggtgcagctg tggcacacac gcacttctgc tcaacccgcc cccccccgcc cccgttcctg 3120
   ttccttccca ggttctcccc attttatcgg ggcggcaact tttaggtccc tgggtcctgg 3180
   aagtccttag tacacactct tcgtccttaa gtccatagtc tgtattccct cggtcctatc 3240
   ctgtccccca tcaccgggtc acctccccag cgaagcaatc tcagttcccc tccccctctc 3300
   agccccgagc ccacacgttt ggtgcgtgca catttcaaaa acgaggcggg tccaaagaga 3360
   gggggtgggg aggtgccgag tggcccagct actcgcggct ttacgggtgc acgtagctca 3420
   ggcctcagcg cccttgagct gtgactggat ggatgagcgg ggcgggaggc ggggcgagcg 3480
   tcctcggcgc tccccaccac cccagttcct ataaatagca gagctcgttt agtgaac 3537
<210> 32
   <211> 537
   <212> DNA
   <213> Artificial
<220>
   <223> amplicon
<400> 32
   tactcgcgaa gaagatcctc aacttttcca cagcctttgc ataaagggga gagggtcggc 60
   ggtgcagctg tggcacacac gcacttctgc tcaacccgcc cccccccgcc cccgttcctg 120
   ttccttccca ggttctcccc attttatcgg ggcggcaact tttaggtccc tgggtcctgg 180
   aagtccttag tacacactct tcgtccttaa gtccatagtc tgtattccct cggtcctatc 240
   ctgtccccca tcaccgggtc acctccccag cgaagcaatc tcagttcccc tccccctctc 300
   agccccgagc ccacacgttt ggtgcgtgca catttcaaaa acgaggcggg tccaaagaga 360
   gggggtgggg aggtgccgag tggcccagct actcgcggct ttacgggtgc acgtagctca 420
   ggcctcagcg cccttgagct gtgactggat ggatgagcgg ggcgggaggc ggggcgagcg 480
   tcctcggcgc tccccaccac cccagttcct ataaatagca gagctcgttt agtgaac 537
<210> 33
   <211> 30
   <212> DNA
   <213> Artificial
<220>
   <223> Primer GlnPr1896
<400> 33
   tacggccggc ttcactgtac agtggcacat 30
<210> 34
   <211> 30
   <212> DNA
   <213> Artificial
<220>
   <223> Primer GlnPr1897
<400> 34
   tcaggccggc cgtggttctt cggtagtgac 30
<210> 35
   <211> 35
   <212> DNA
   <213> Artificial
<220>
   <223> Primer GlnPr1901
<400> 35
   tactcgcgaa gaagatcctc aacttttcca cagcc 35
<210> 36
   <211> 40
   <212> DNA
   <213> Artificial
<220>
   <223> Primer GlnPr1902
<400> 36
   gttcactaaa cgagctctgc tatttatagg aactggggtg 40
<210> 37
   <211> 40
   <212> DNA
   <213> Artificial
<220>
   <223> Primer GLnPr1903
<400> 37
   caccccagtt cctataaata gcagagctcg tttagtgaac 40
<210> 38
   <211> 20
   <212> DNA
   <213> Artificial
<220>
   <223> Primer GlnPr1904
<400> 38
   cgctagcacc ggtcgatcga 20
<210> 39
   <211> 31
   <212> DNA
   <213> Artificial
<220>
   <223> Primer GlnPr1905
<400> 39
   tactcgcgat tcactgtaca gtggcacata c 31

## Description

### Field of the invention

The present invention relates to an expression cassette useful for the expression of a polynucleotide sequence encoding a polypeptide. The present invention is also directed to vectors and host cells which comprise the expression cassette and uses of the expression cassette for the production of a polypeptide from a host cell.

### Background of the invention

Expression systems for the production of recombinant polypeptides are well-known in the state of the art and are described by, e.g., Marino MH (1989) Biopharm, 2: 18-33; Goeddel DV et al., (1990) Methods Enzymol 185: 3-7; Wurm F & Bernard A (1999) Curr Opin Biotechnol 10: 156-159. Polypeptides for use in pharmaceutical applications are preferably produced in mammalian cells such as CHO cells, NSO cells, SP2/0 cells, COS cells, HEK cells, BHK cells, or the like. The essential elements of an expression vector used for this purpose are normally selected from a prokaryotic plasmid propagation unit, for example *E.coli,* comprising a prokaryotic origin of replication and a prokaryotic selection marker, optionally a eukaryotic selection marker, and one or more expression cassettes for the expression of the structural gene(s) of interest each comprising a promoter, a polynucleotide sequence encoding a polypeptide, and optionally a transcription terminator including a polyadenylation signal. For transient expression in mammalian cells a mammalian origin of replication, such as the SV40 Ori or OriP, can be included. As promoter a constitutive or inducible promoter can be selected. For optimized transcription a Kozak sequence may be included in the 5' untranslated region. For mRNA processing, in particular mRNA splicing and transcription termination, mRNA splicing signals, depending on the organization of the structural gene (exon/intron organization), may be included as well as a polyadenylation signal. Expression of a gene is performed either in transient or using a stable cell line. The level of stable and high expression of a polypeptide in a production cell line is crucial to the overall process of the production of recombinant polypeptides. The demand for biologic molecules such as proteins and specifically antibodies or antibody fragments has increased significantly over the last few years. High cost and poor yield have been limiting factors in the availability of biologic molecules and it has been a major challenge to develop robust processes that increase the yield of desirable biological molecules on an industrial scale.

EP1308510 teaches the use of the entire GAPDH promoter for screening apoptosis-inducing agents. In all embodiments disclosed in the cited application, the full promoter is used. Even where untranslated sequences are included in the constructs, they are used in conjunction with the full promoter.

Thus there is still a need for improving the efficiency of expression vectors to obtain high expression in recombinant polypeptide production.

### Summary of the invention

The present invention relates generally to expression systems such as expression cassettes and expression vectors which can be used to obtain increased expression in recombinant polypeptide production. In one aspect, the present disclosure provides an expression cassette which comprises a promoter, a polynucleotide sequence encoding a polypeptide, and a non-translated genomic DNA sequence downstream of a eukaryotic Glyceraldehyde 3-phosphate dehydrogenase (GAPDH) promoter, wherein the polypeptide encoded by the polynucleotide sequence is not GAPDH, and wherein the non-translated genomic DNA sequence downstream of the eukaryotic GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the eukaryotic GAPDH promoter is from around 200 to around 2800 nucleotides.

In a further aspect, the present disclosure provides an expression cassette which comprises a promoter, a polynucleotide sequence encoding a polypeptide, and a non-translated genomic DNA sequence upstream of a eukaryotic GAPDH promoter, wherein the polypeptide encoded by the polynucleotide sequence is not GAPDH, and wherein the non-translated genomic DNA sequence upstream of the eukaryotic GAPDH promoter starts within a region spanning from around the 5' end of the eukaryotic GAPDH promoter to nucleotide position around -3500, wherein the nucleotide position is relative to the transcription start of the GAPDH mRNA, wherein the length of the non-translated genomic DNA sequence upstream of the eukaryotic GAPDH promoter is from 200 to around 2800 nucleotides, with the proviso that the expression cassette does not comprise a eukaryotic GAPDH promoter or fragments thereof.
In a further aspect, the present disclosure provides an expression vector comprising an expression cassette and a host cell comprising an expression cassette or an expression vector comprising an expression cassette.

In still further aspects, the present disclosure provides an *in vitro* method for the expression of a polypeptide, comprising transfecting a host cell with an expression cassette or an expression vector and recovering the polypeptide and the use of an expression cassette or an expression vector for the expression of a heterologous polypeptide from a mammalian host cell.

### Brief description of the figures

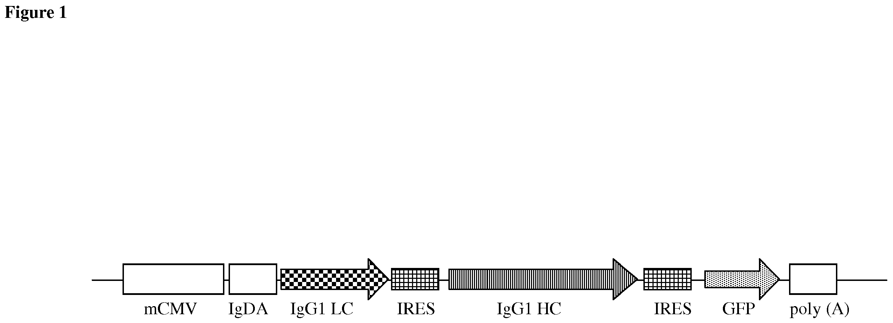

**Figure 1** shows reporter expression construct (REP) consisting of mouse cytomegalovirus promoter (mCMV), Ig donor acceptor fragment (IgDA) containing the first intron, IgG1 antibody light chain (IgG1 LC), Internal Ribosomal Entry Sites derived from Encephalomyocarditis virus (IRES), IgG1 antibody heavy chain (IgG1 HC), green fluorescent protein (GFP) and simian virus 40 polyadenylation signal (poly (A)).

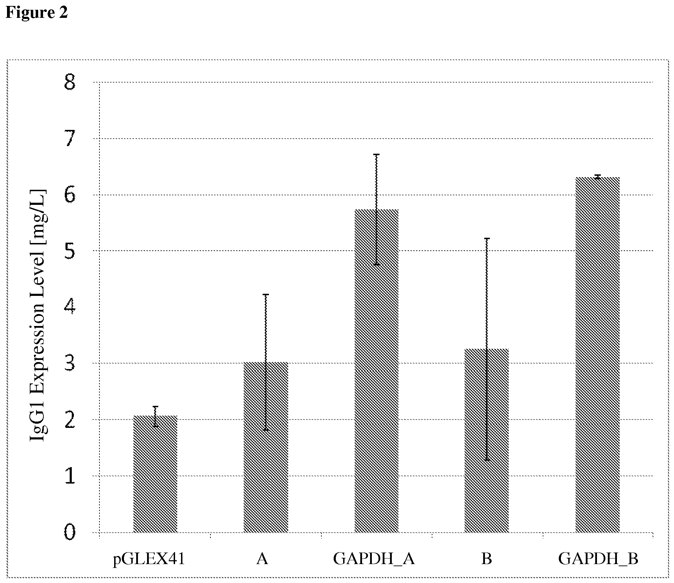

**Figure 2** shows transient expression of IgG1 antibody in CHO-S cells on day 5 post-transfection (Mean of IgG titers are plotted for two independent transfections). Cells were transfected using the GAPDH_A and GAPDH_B vectors (GAPDH_A and GAPDH_B), the same vectors without GAPDH upstream and downstream elements (A and B) and the pGLEX41 vector as a control (pGLEX41). The concentration of the accumulated IgG1 antibody in the supernatant was determined using the Octet instrument (Fortebio, Menlo, CA, USA).

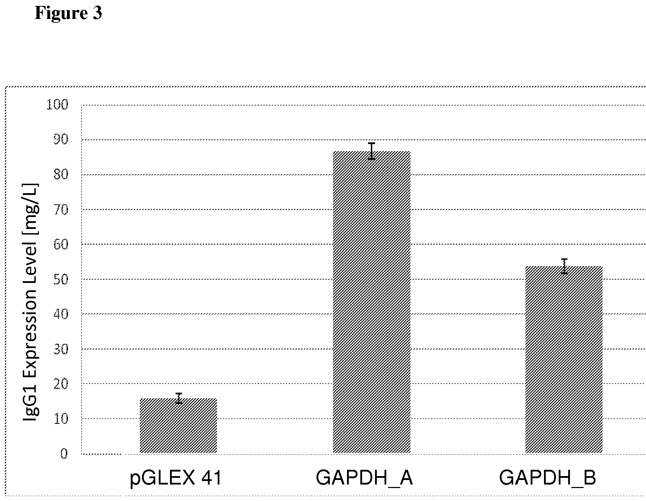

**Figure 3** shows expression of IgG1 antibody in HEK293 EBNA cells. Cells were transfected using the GAPDH_A and GAPDH_B vectors (GAPDH_A and GAPDH_B) and the pGLEX41 vector as a control (pGLEX41). The supernatant was harvested and analysed on day 10 after transfection using the Octet instrument. The data represent N = 3 independent transfections in tubespins per vector.

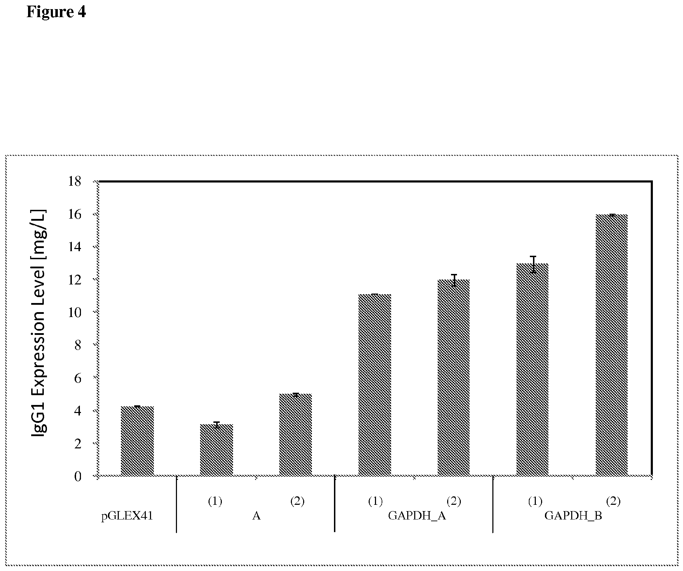

**Figure 4** shows an expression level study on a batch production using cellular pools. Cells were transfected and pools of stable cells were created using GAPDH_A and GAPDH_B vectors (GAPDH_A(1), GAPDH_A(2), GAPDH_B(1) and GAPDH_B(2)), the same vectors without the GAPDH upstream and downstream elements (A(1) and A(2)) and the pGLEX41 vector as a control (pGLEX41). After 7 days of culture the supernatant was analyzed using the Octet instrument for accumulated antibody in the supernatant. Mean of IgG titers are given (µg/ml) for each pool. The data represent N= 2 batches per pool.

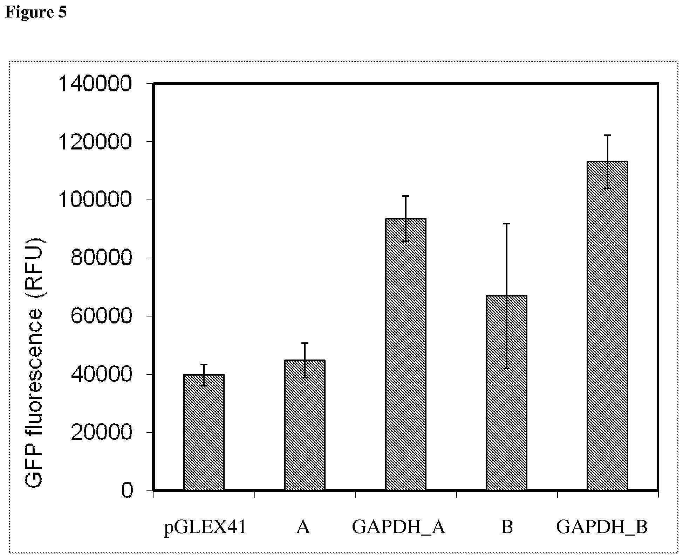

**Figure 5** shows an expression level study on populations generated by stable transfection and limiting dilution. Cells were transfected using the GAPDH_A and GAPDH_B vectors (GAPDH_A and GAPDH_B), the same vectors without the GAPDH upstream and downstream elements (A and B) and the pGLEX41 vector as a control (pGLEX41). The mean value of GFP fluorescence expressed by clones and minipools from stable transfections was read 14 days after transfection. Cells were cultivated under selection pressure in 96-well plates. The data represent N= 48 clones or minipools per vector.

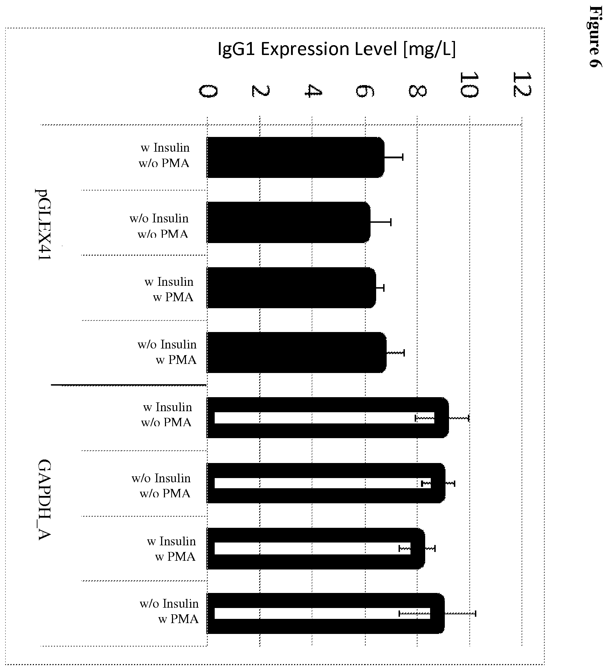

**Figure 6** shows the effect of medium additives insulin and PMA (phorbol 12-myristate 13-acetate, a phorbol ester) on expression of IgG1 antibody in the supernatant. After transfection with the GAPDH_A vector (GAPDH_A) and the pGLEX41 vector as a control (pGLEX41) the cells were either diluted in PowerCHO2 medium, 4mM Gln, +/- insulin and PowerCHO2, 4mM Gln, PMA +/- insulin. No difference in expression could be observed compared to the standard medium for pGLEX41 (filled bars) or GAPDH_A (open bars).

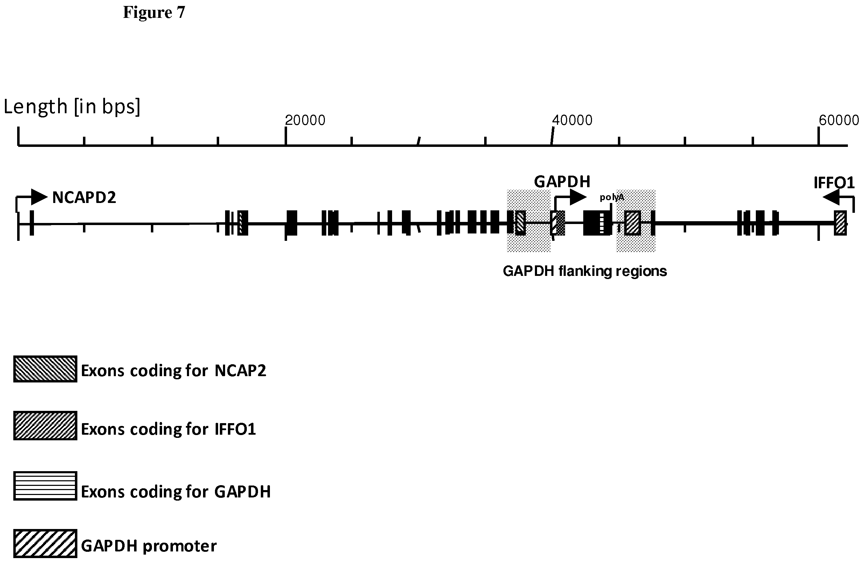

**Figure 7** shows an overview of the human GAPDH locus. The GAPDH gene is flanked by the genes NCAPD2 and IFFO1.

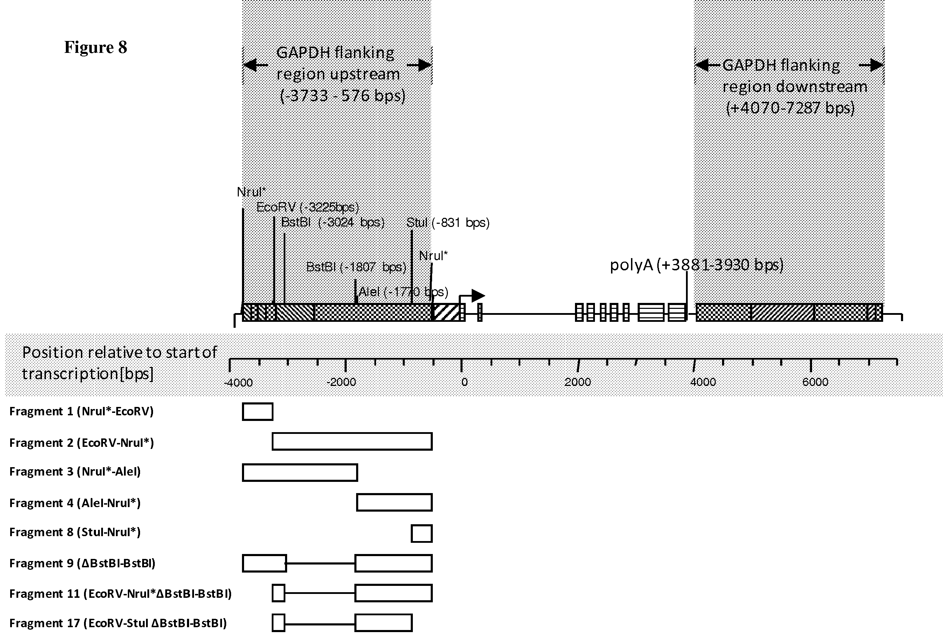

**Figure 8** shows details of the human GAPDH gene, the GAPDH up- and downstream elements and the fragments created for the analysis of the GAPDH upstream fragmentation study. The NruI restriction site was introduced to facilitate cloning steps and is not part of the genomic 5' GAPDH upstream sequence (it is therefore highlighted using an asterisk). The sizes of the fragments are: Fragment 1 (SEQ ID NO: 9): 511 bps, Fragment 2 (SEQ ID NO: 10): 2653 bps, Fragment 3 (SEQ ID NO: 11): 1966 bps, Fragment 4 (SEQ ID NO: 12): 1198 bps, Fragment 8 (SEQ ID NO: 13): 259 bps, Fragment 9 (SEQ ID NO: 14): 1947 bps, Fragment 11 (SEQ ID NO: 15): 1436 bps, and Fragment 17 (SEQ ID NO: 16): 1177 bps.

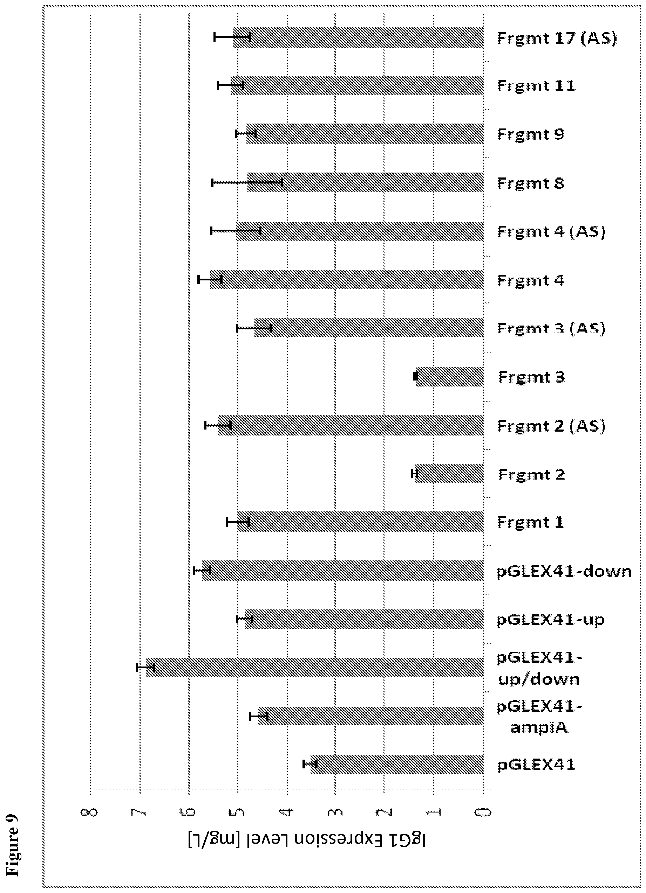

**Figure 9** shows expression results of fragmentation of the GAPDH upstream and downstream elements. Expression results were obtained in transient transfection in CHO cells on day 10 after transfection. The quantification was done using the Octet instrument. Vector pGLEX41 serves as negative control. pGLEX41-ampiA also is a negative control showing the basal expression of the vector without the GAPDH flanking elements. pGLEX41-up/down contains the full length flanking (upstream and downstream) regions and serves as positive control. pGLEX41-up contains only the upstream flanking region and pGLEX41-down contains only the downstream flanking region. All other constructs contain the fragments described in Figure 8. The fragments 2 and 3 were either cloned in the same direction as IgG1 LC and IgG1 HC or in opposite direction in relation to IgG1 LC and IgG1 HC (AS).

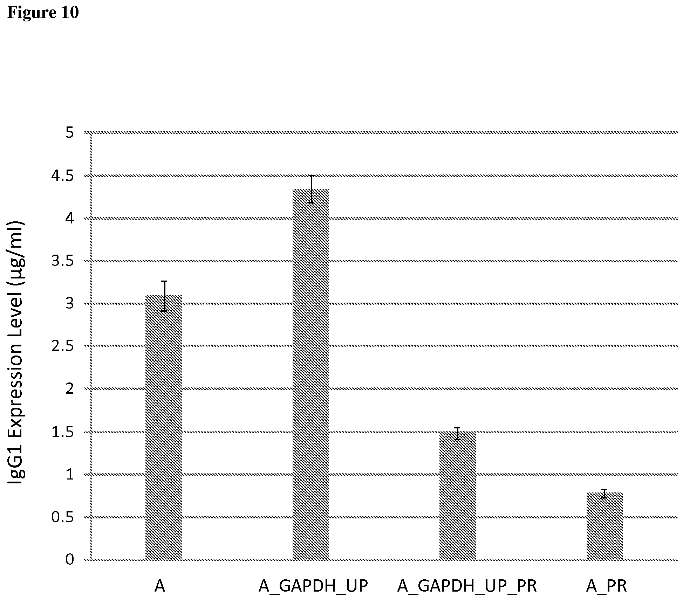

**Figure 10** shows transient expression of IgG1 antibody in CHO-S cells on day 8 post-transfection (Mean of IgG titers are plotted for three independent transfections; error bars: SD +/-). Cells were transfected using vectors with the Chinese hamster GAPDH upstream element in combination with the mouse CMV (A_GAPDH_UP) or the Chinese hamster GAPDH promoter (A_GAPDH_UP_PR). The plasmids having only the mouse CMV (A) or the Chinese hamster GAPDH promoter (A_PR) were transfected as a control. The concentration of the accumulated IgG1 antibody in the supernatant was determined using the Octet QK instrument (Fortebio, Menlo, CA, USA).

### Detailed description of the invention

The present disclosure relates to expression cassettes and expression vectors which comprise a promoter, a polynucleotide sequence encoding a polypeptide, and a non-translated genomic DNA sequence downstream of a eukaryotic Glyceraldehyde 3-phosphate dehydrogenase (GAPDH) promoter, wherein the polypeptide encoded by the polynucleotide sequence is not GAPDH, and wherein the non-translated genomic DNA sequence downstream of the eukaryotic GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the eukaryotic GAPDH promoter is from around 200 to around 2800 nucleotides.

The present disclosure further relates to an expression cassette which comprises a promoter, a polynucleotide sequence encoding a polypeptide, and a non-translated genomic DNA sequence upstream of a eukaryotic GAPDH promoter, wherein the polypeptide encoded by the polynucleotide sequence is not GAPDH, and wherein the non-translated genomic DNA sequence upstream of the eukaryotic GAPDH promoter starts within a region spanning from around the 5' end of the eukaryotic GAPDH promoter to nucleotide position around -3500, wherein the nucleotide position is relative to the transcription start of the GAPDH mRNA, wherein the length of the non-translated genomic DNA sequence upstream of the eukaryotic GAPDH promoter is from 200 to around 2800 nucleotides, with the proviso that the expression cassette does not comprise a eukaryotic GAPDH promoter or fragments thereof.

The term "expression cassette" as used herein includes a polynucleotide sequence encoding a polypeptide to be expressed and sequences controlling its expression such as a promoter and optionally an enhancer sequence, including any combination of cis-acting transcriptional control elements. The sequences controlling the expression of the gene, i.e. its transcription and the translation of the transcription product, are commonly referred to as regulatory unit. Most parts of the regulatory unit are located upstream of coding sequence of the gene and are operably linked thereto. The expression cassette may also contain a downstream 3' untranslated region comprising a polyadenylation site. The regulatory unit of the invention is either operably linked to the gene to be expressed, i.e. transcription unit, or is separated therefrom by intervening DNA such as for example by the 5 '-untranslated region of the heterologous gene. Preferably the expression cassette is flanked by one or more suitable restriction sites in order to enable the insertion of the expression cassette into a vector and/or its excision from a vector. Thus, the expression cassette according to the present invention can be used for the construction of an expression vector, in particular a mammalian expression vector. The expression cassette of the present invention may comprise one or more e.g. two, three or even more non-translated genomic DNA sequences downstream of a eukaryotic GAPDH promoter or fragments thereof, and/or one or more e.g. two, three or even more non-translated genomic DNA sequences upstream of a eukaryotic GAPDH promoter or fragments thereof. If the expression cassette of the present invention comprises more than one DNA sequence downstream and/or upstream of a eukaryotic GAPDH promoter or fragments thereof these DNA sequences may be directly linked, i.e. may comprise linker sequences e.g. linker sequences containing restriction sites that are attached to the 5'- and 3'- ends and that allow comfortable sequential cloning of the sequences or fragments thereof. Alternatively, the DNA sequences downstream and/or upstream of a eukaryotic GAPDH promoter or fragments thereof may be not directly linked, i.e. may be cloned with intervening DNA sequences.

The term "polynucleotide sequence encoding a polypeptide" as used herein includes DNA coding for a gene, preferably a heterologous gene expressing the polypeptide.

The terms "heterologous coding sequence", "heterologous gene sequence", "heterologous gene", "recombinant gene" or "gene" are used interchangeably. These terms refer to a DNA sequence that codes for a recombinant, in particular a recombinant heterologous protein product that is sought to be expressed in a host cell, preferably in a mammalian cell and harvested. The product of the gene can be a polypeptide. The heterologous gene sequence is naturally not present in the host cell and is derived from an organism of the same or a different species and may be genetically modified.

The terms "protein" and "polypeptide" are used interchangeably to include a series of amino acid residues connected to the other by peptide bonds between the alpha-amino and carboxy groups of adjacent residues.

The term "non-translated genomic DNA sequence" as used herein includes DNA that constitutes genetic information of an organism. The genome of almost all organisms is DNA, the only exceptions being some viruses that have a RNA genome. Genomic DNA molecules in most organisms are organized into DNA-protein complexes called chromosomes. The size, number of chromosomes, and nature of genomic DNA varies between different organisms. Viral DNA genomes can be single- or double-stranded, linear or circular. All other organisms have double-stranded DNA genomes. Bacteria have a single, circular chromosome. In eukaryotes, most genomic DNA is located within the nucleus (nuclear DNA) as multiple linear chromosomes of different sizes. Eukaryotic cells additionally contain genomic DNA in the mitochondria and, in plants and lower eukaryotes, the chloroplasts. This DNA is usually a circular molecule and is present as multiple copies within these organelles. A non-translated genomic DNA sequence is normally not operably linked to a promoter and thus is not translated. It may contain gene(s) which are not translated, thus gene(s) that encode e.g. a protein which is not expressed.

The term "non-translated genomic DNA sequence downstream of a eukaryotic GAPDH promoter" as used herein corresponds to non-translated eukaryotic genomic DNA 3' of a eukaryotic GAPDH promoter. Non-translated genomic DNA sequence downstream of a eukaryotic GAPDH promoter normally starts at nucleotide position around +1, preferably at nucleotide position +1, wherein the nucleotide position is relative to the transcription start of the GAPDH mRNA i.e. is relative to the origin of the transcription start of the eukaryotic gene coding for GAPDH. The non-translated genomic DNA sequence downstream of a eukaryotic GAPDH promoter is usually of the same origin as the eukaryotic GAPDH promoter, e.g. if the GAPDH promoter is of human origin the non-translated genomic DNA sequence downstream of the human GAPDH promoter is as well of human origin and corresponds to the naturally occurring human genomic DNA sequence downstream of the human GAPDH promoter.

The term "non-translated genomic DNA sequence upstream of a eukaryotic GAPDH promoter" as used herein corresponds to non-translated eukaryotic genomic DNA 5' of a eukaryotic GAPDH promoter. Non-translated genomic DNA sequence upstream of a eukaryotic GAPDH promoter normally starts at a nucleotide position around the 5' end of the eukaryotic GAPDH promoter, preferably at the nucleotide position immediately after the 5' end of the eukaryotic GAPDH promoter. The non-translated genomic DNA sequence upstream of a eukaryotic GAPDH promoter is usually of the same origin as the eukaryotic GAPDH promoter, e.g. if the GAPDH promoter is of human origin the non-translated genomic DNA sequence upstream of the human GAPDH promoter is as well of human origin and corresponds to the naturally occurring human genomic DNA sequence upstream of the human GAPDH promoter.

Positions of the eukaryotic GAPDH promoter, the non-translated genomic DNA sequence downstream or upstream of the eukaryotic GAPDH promoter and other DNA sequences as indicated herein are relative to the transcription start of the GAPDH mRNA e.g. are relative to the origin of the transcription start of the eukaryotic GAPDH if not specifically otherwise indicated.

The term "non-translated genomic DNA sequence upstream of a eukaryotic GAPDH promoter extends to" or "non-translated genomic DNA sequence downstream of a eukaryotic GAPDH promoter extends to" is used to define extension of the length of non-translated genomic DNA sequence upstream and/or downstream of a eukaryotic GAPDH promoter from the start to a particular genetic element e.g. extension to an intron. This extension includes the full length of the DNA sequence encoding the genetic element e.g. the intron or a part thereof.

The eukaryotic GAPDH promoter and the eukaryotic genomic DNA upstream and/or downstream of the GAPDH promoter can be found for human, rat and mouse in the NCBI public databank (Entries for human, mouse, rat and Chinese hamster GAPDH gene are Gene IDs 2597 (mRNA: NM_002046.3), 14433 (mRMA: NM_008084.2), 24383 (mRNA: NM_017008.3) and 100736557 (mRNA: NM_001244854.2), respectively; National Center for Biotechnology Information (NCBI): http://www.ncbi.nlm.nih.gov/) and are exemplarily shown in Figure 7 and 8 for the human GAPDH gene.

The eukaryotic GAPDH promoter is usually considered to stretch from around bps -500 to around +50 relative to the transcription start of the GAPDH mRNA. The human GAPDH promoter is located on chromosome 12. The human GAPDH promoter is considered by Graven *et al.* (Graven et al., (1999) Biochimica et Biophysics Acta, 147: 203-218) to stretch from bps -488 to +20 relative to the transcription start of the GAPDH mRNA based on a fragmentation study. According to the NCBI public databank the human GAPDH promoter stretches from bps -462 to +46 relative to the transcription start of the GAPDH mRNA as defined by the NCBI public databank. If not specifically otherwise indicated, the human GAPDH promoter as referred to herein stretches from -462 to position +46 relative to the transcription start of the GAPDH mRNA which correspond to the sequence stretching from bps 4071 to 4578 of SEQ ID NO: 17.

The numbering used for the DNA of the GAPDH gene, the IFF01 gene and the NCAPD2 gene of human, mouse and rat origin as referred herein corresponds to the numbering used for these genes in the NCBI public databank (http://www.ncbi.nlm.nih.gov/).

The term "promoter" as used herein defines a regulatory DNA sequence generally located upstream of a gene that mediates the initiation of transcription by directing RNA polymerase to bind to DNA and initiating RNA synthesis.

The term "enhancer" as used herein defines a nucleotide sequence that acts to potentiate the transcription of genes independent of the identity of the gene, the position of the sequence in relation to the gene, or the orientation of the sequence. The vectors of the present invention optionally include enhancers.

The terms "functionally linked" and "operably linked" are used interchangeably and refer to a functional relationship between two or more DNA segments, in particular gene sequences to be expressed and those sequences controlling their expression. For example, a promoter and/or enhancer sequence, including any combination of cis-acting transcriptional control elements is operably linked to a coding sequence if it stimulates or modulates the transcription of the coding sequence in an appropriate host cell or other expression system. Promoter regulatory sequences that are operably linked to the transcribed gene sequence are physically contiguous to the transcribed sequence.

"Orientation" refers to the order of nucleotides in a given DNA sequence. For example, an orientation of a DNA sequence in opposite direction in relation to another DNA sequence is one in which the 5' to 3' order of the sequence in relation to another sequence is reversed when compared to a point of reference in the DNA from which the sequence was obtained. Such reference points can include the direction of transcription of other specified DNA sequences in the source DNA and/or the origin of replication of replicable vectors containing the sequence.

The term "expression vector" as used herein includes an isolated and purified DNA molecule which upon transfection into an appropriate host cell provides for a high-level expression of a recombinant gene product within the host cell. In addition to the DNA sequence coding for the recombinant or gene product the expression vector comprises regulatory DNA sequences that are required for an efficient transcription of the DNA coding sequence into mRNA and for an efficient translation of the mRNAs into proteins in the host cell line.

The terms "host cell" or "host cell line" as used herein include any cells, in particular mammalian cells, which are capable of growing in culture and expressing a desired recombinant product protein.

The term "fragment" as used herein includes a portion of the respective nucleotide sequence e.g. a portion of the non-translated genomic DNA sequence downstream and/or upstream of a eukaryotic GAPDH promoter or a portion of the nucleotide sequence encoding a particular genetic element such as a promoter. Fragments of a non-translated genomic DNA sequence downstream and/or upstream of a eukaryotic GAPDH promoter may retain biological activity and hence alter e.g. increase the expression patterns of coding sequences operably linked to a promoter. Fragments of a non-translated genomic DNA sequence downstream and/or upstream of a eukaryotic GAPDH promoter may range from at least about 100 to about 3000 bp, preferably from about 200 to about 2800 bp, more preferably from about 300 to about 2000 bp nucleotides, in particular from about 500 to about 1500 bp nucleotides. In order to clone the fragments of the non-translated genomic DNA sequence downstream and/or upstream of a eukaryotic GAPDH promoter in the expression cassette of the present invention, usually linker sequences containing restriction sites that allow comfortable cloning are attached to the 5'- and 3'- ends of the fragments.

The term "nucleotide sequence identity" or "identical nucleotide sequence" as used herein include the percentage of nucleotides in the candidate sequence that are identical with the nucleotide sequence of e.g. the non-translated genomic DNA sequence downstream and/or upstream of a eukaryotic GAPDH promoter, after aligning the sequences and introducing gaps, if necessary, to achieve the maximum percent sequence identity. Thus sequence identity can be determined by standard methods that are commonly used to compare the similarity in position of the nucleotides of two nucleotide sequences. Usually the nucleotide sequence identity of the candidate sequence to the non-translated genomic DNA sequence downstream and/or upstream of a eukaryotic GAPDH promoter is at least 80%, preferably at least 85%, more preferably at least 90%, and most preferably at least 95%, in particular 96%, more particular 97%, even more particular 98%, most particular 99%, including for example, 80%, 81%, 82%, 83%, 84%, 85%, 86%, 87%, 88%, 89%, 90%, 91%, 92%, 93%, 94%, 95%, 96%, 97%, 98%, 99%, and 100%.

The term "CpG site" as used herein include regions of DNA where a cytosine nucleotide occurs next to a guanine nucleotide in the linear sequence of bases along its length. "CpG" is shorthand for "-C-phosphate-G-", that is, cytosine and guanine separated by only one phosphate; phosphate links any two nucleosides together in DNA. The "CpG" notation is used to distinguish this linear sequence from the CG base-pairing of cytosine and guanine.

The term "alternative codon usage" as used herein includes usage of alternative codons coding for the same amino acid in order to avoid the CpG sequence motif. This includes using preferably codons not having an internal CpG site (for example GCG coding for Alanine and containing a CpG site, might be replaced by either GCT, GCC or GCA) as well as avoiding joining of two codons that leads to a new CpG site.

The term "around" as used herein in relation to the length of a DNA sequence and in relation to a nucleotide position which is relative to the transcription start of the GAPDH mRNA e.g. is relative to the origin of the transcription start of the eukaryotic GAPDH includes values with deviations of a maximum of ± 50 % , usually of a maximum of ± 10 % of the stated values e.g. "around 3000 nucleotides" includes values of 2700 to 3300 nucleotides, preferably 2900 to 3100 nucleotides, more preferably 2995 to 3005 nucleotides, "around 100 nucleotides" includes values of 50 to 150 nucleotides, preferably 90 to 110 nucleotides, more preferably 95 to 105 nucleotides, "around 15000 nucleotides" includes values of 13500 to 16500 nucleotides, preferably 14500 to 15500 nucleotides, more preferably 14990 to 15010 nucleotides, most preferably 14995 to 15005 nucleotides, "around 200 nucleotides" includes values of 150 to 250 nucleotides, preferably 190 to 210 nucleotides, more preferably 195 to 205 nucleotides, "around 8000 nucleotides" includes values of 7200 to 8800, preferably 7500 to 8500 nucleotides, more preferably 7990 to 8010 nucleotides, most preferably 7995 to 8005 nucleotides, "around 500 nucleotides" includes values of 450 to 550 nucleotides, preferably 475 to 525, more preferably 490 to 510, most preferably 495 to 505 nucleotides, "around 5000 nucleotides" includes values of 4500 to 5500 nucleotides, preferably 4750 to 5250, more preferably 4990 to 5010, most preferably 4995 to 5005 nucleotides, "around 1000 nucleotides" includes values of 900 to 1100 nucleotides, preferably 950 to 1050, more preferably 990 to 1010, most preferably 995 to 1005 nucleotides, "around 4500 nucleotides" includes values of 4050 to 4950 nucleotides, preferably 4250 to 4750, more preferably 4490 to 4510, most preferably 4495 to 4505 nucleotides, "around 1500 nucleotides" includes values of 1350 to 1650 nucleotides, preferably 1450 to 1550, more preferably 1490 to 1510, most preferably 1495 to 1505 nucleotides, "around 4000 nucleotides" includes values of 3600 to 4400 nucleotides, preferably 3800 to 4200, more preferably 3990 to 4010, more preferably 3995 to 4005 nucleotides, "around 2000 nucleotides" includes values of 1800 to 2200 nucleotides, preferably 1900 to 2100, more preferably 1990 to 2010, most preferably 1995 to 2005 nucleotides, "around 3500 nucleotides" includes values of 3150 to 3850 nucleotides, preferably 3300 to 3700, more preferably 3490 to 3510, most preferably 3495 to 3505 nucleotides, "around 2700 nucleotides" includes values of 2430 to 2970 nucleotides, preferably 2600 to 2800, more preferably 2690 to 2710, most preferably 2695 to 2705 nucleotides, "around 3300 nucleotides" includes values of 2970 to 3630 nucleotides preferably 3100 to 3500, more preferably 3290 to 3310, most preferably 3295 to 3305 nucleotides, "around 3200 nucleotides" includes values of 2880 to 3520 nucleotides, preferably 3000 to 3400, more preferably 3190 to 3210, most preferably 3195 to 3205 nucleotides, around +7000 or around position +7000 includes positions +6300 to +7700, preferably positions +6700 to +7300, more preferably positions +6990 to +7010, most preferably positions +6995 to +7005, around +1 or around position +1 includes positions -10 to +10, preferably positions -5 to +5, more preferably positions -1 to +2, around -3500 or around position -3500 includes positions -3150 to -3850, preferably positions -3300 to -3700, more preferably positions -3490 to -5010, most preferably positions -3495 to -3505.
The term "around" as used herein in relation to the numbering used for the DNA of the GAPDH gene, the IFF01 gene and the NCAPD2 gene of human, mouse and rat origin as referred herein or used herein in relation to a position in a sequence of a SEQ ID number includes values with deviations of a maximum of ± 500 bps, preferably ± 100 bps, more preferably ± 10 bps, most preferably ± 5 bps.

In one embodiment, the present disclosure provides an expression cassette which comprises a promoter, a polynucleotide sequence encoding a polypeptide, and a non-translated genomic DNA sequence downstream of a eukaryotic GAPDH promoter, wherein the polypeptide encoded by the polynucleotide sequence is not GAPDH, and wherein the non-translated genomic DNA sequence downstream of the eukaryotic GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the eukaryotic GAPDH promoter is from around 200 to around 2800 nucleotides.
In one embodiment, the length of the non-translated genomic DNA sequence downstream of a eukaryotic GAPDH promoter is at least around 100 nucleotides and extends at its maximum to the second last intron of the IFF01 gene or to a part thereof. In one embodiment, the length of the non-translated genomic DNA sequence downstream of a eukaryotic GAPDH promoter is at least around 100 nucleotides and extends at its maximum to the last intron of the IFF01 gene.

The human IFF01 gene is located in human DNA around bps 6665249 to 6648694 of chromosome 12 (NCBI gene ID: 25900). In one embodiment, the length of the non-translated genomic DNA sequence downstream of a eukaryotic GAPDH promoter extending at its maximum to the last intron of the IFF01 gene in human stretches at its maximum to around bps 6650677 of chromosome 12 coding for the IFF01 gene in human (position +7021). In one embodiment, the length of the non-translated genomic DNA sequence downstream of a eukaryotic GAPDH promoter extending at its maximum to the second last intron of the IFF01 gene in human stretches at its maximum to around bps 6657230 of chromosome 12 coding for the IFF01 gene in human (position + 13574). The non-translated genomic DNA sequences downstream of a eukaryotic GAPDH promoter extending at its maximum to the last intron of the IFF01 gene in human and to the second last intron of the IFF01 gene in human, respectively , are included in SEQ ID NO: 17 which shows bps 6657230 to 6639125 of chromosome 12 (NCBI gene ID: 25900). The non-translated genomic DNA sequence downstream of a eukaryotic GAPDH promoter extending to the last intron stretches to around bps 11553 of the nucleotide sequence as shown by SEQ ID NO: 17 and the non-translated genomic DNA sequence downstream of a eukaryotic GAPDH promoter extending to the second last intron stretches to around bps 18106 of the nucleotide sequence as shown by SEQ ID NO: 17.

The mouse IFF01 gene (NCBI gene ID: 320678) is located in mouse DNA around bps 125095259 to 125111800 of chromosome 6. In one embodiment, the length of the non-translated genomic DNA sequence downstream of a eukaryotic GAPDH promoter extending at its maximum to the last intron of the IFF01 gene in mouse stretches at its maximum to around bps 125109211 of chromosome 6 coding for the IFF01 gene in mouse (position + 6391). In one embodiment, the length of the non-translated genomic DNA sequence downstream of a eukaryotic GAPDH promoter extending at its maximum to the second last intron of the IFF01 gene in mouse stretches at its maximum to around bps 125103521 of chromosome 6 coding for the IFF01 gene in mouse (position +12081). The non-translated genomic DNA sequences downstream of a eukaryotic GAPDH promoter extending at its maximum to the last intron and to the second last intron of the IFF01 gene in mouse, respectively are included in SEQ ID NO: 18 which shows bps 125103521 to 125119832 of chromosome 6 (NCBI gene ID: 320678). The non-translated genomic DNA sequence downstream of a eukaryotic GAPDH promoter extending to the last intron of the IFFO1 gene in mouse stretches to around bps 10622 of the nucleotide sequence as shown by SEQ ID NO: 18 and the non-translated genomic DNA sequence downstream of a eukaryotic GAPDH promoter extending to the second last intron of the IFFO1 gene in mouse stretches to around bps 16312 of the nucleotide sequence as shown by SEQ ID NO: 18.

The rat IFF01 gene (NCBI gene ID: 362437) is located in rat DNA around bps 161264966 to 161282150 of chromosome 4. In one embodiment, the length of the non-translated genomic DNA sequence downstream of a eukaryotic GAPDH promoter extending at its maximum to the last intron of the IFF01 gene in rat stretches at its maximum to around bps 161280937 of the chromosome 4 coding for IFF01 gene in rat (position + 5154). In one embodiment, the length of the non-translated genomic DNA sequence downstream of a eukaryotic GAPDH promoter extending at its maximum to the second last intron of the IFF01 gene in rat stretches at its maximum to around bps 161279451 of chromosome 4 coding for the IFF01 gene in rat (position +6640).

The non-translated genomic DNA sequences downstream of a eukaryotic GAPDH promoter extending at its maximum to the last intron and to the second last intron of the IFF01 gene in rat, respectively are included in SEQ ID NO: 19 which shows bps 161279451 to 161290508 of chromosome 4 (NCBI gene ID: 362437). The non-translated genomic DNA sequence downstream of a eukaryotic GAPDH promoter extending to the last intron of the IFFO1 gene stretches to around bps 9572 of the nucleotide sequence as shown by SEQ ID NO: 19 and the non-translated genomic DNA sequence downstream of a eukaryotic GAPDH promoter extending to the second last intron of the IFFO1 gene stretches to around bps 11058 bps of the nucleotide sequence as shown by SEQ ID NO: 19.

The Chinese hamster IFFO1 gene (NCBI gene ID: 100753382) is located in Chinese hamster DNA around bps 3577293 to 3593683. In one embodiment, the length of the non-translated genomic DNA sequence downstream of a eukaryotic GAPDH promoter extending at its maximum to the last intron of the IFF01 gene in Chinese hamster stretches at its maximum to around bps 3579014 coding for IFF01 gene in Chinese hamster (position +6883). In one embodiment, the length of the non-translated genomic DNA sequence downstream of a eukaryotic GAPDH promoter extending at its maximum to the second last intron of the IFF01 gene in Chinese hamster stretches at its maximum to around bps 3585061 coding for the IFFO1 gene in Chinese hamster (position +12930). The chromosomal location is not yet annotated in the NCBI databank and the current sequence information contains many unknown bases. Therefore the precise annotation of the limits may change with the availability of more accurate sequence information.

The non-translated genomic DNA sequences downstream of a eukaryotic GAPDH promoter extending at its maximum to the last intron and to the second last intron of the IFF01 gene in Chinese hamster, respectively are included in SEQ ID NO: 29 which shows bps 3567932 to 3585061. The non-translated genomic DNA sequence downstream of a eukaryotic GAPDH promoter extending to the last intron of the IFFO1 gene stretches to around bps 11083 of the nucleotide sequence as shown by SEQ ID NO: 29 and the non-translated genomic DNA sequence downstream of a eukaryotic GAPDH promoter extending to the second last intron of the IFFO1 gene stretches to around bps 17130 bps of the nucleotide sequence as shown by SEQ ID NO: 29.

In a further embodiment, the non-translated genomic DNA sequence downstream of a eukaryotic GAPDH promoter starts at the eukaryotic GAPDH polyadenylation site e.g. starts at the first nucleotide encoding the eukaryotic GAPDH polyadenylation site. Preferably the non-translated genomic DNA sequence downstream of the eukaryotic GAPDH promoter starts downstream of the eukaryotic GAPDH polyadenylation site e.g. starts immediately after the last nucleotide encoding the eukaryotic GAPDH polyadenylation site. Even more preferred the non-translated genomic DNA sequence downstream of the eukaryotic GAPDH promoter starts downstream of the eukaryotic GAPDH polyadenylation site and the length of the non-translated genomic DNA sequence downstream of the eukaryotic GAPDH promoter is at least around 100 nucleotides and extends at its maximum to the second last intron of the IFF01 gene.

In one embodiment, the non-translated genomic DNA sequence downstream of the eukaryotic GAPDH promoter starts within a region spanning from nucleotide position around +3881 to nucleotide position around +5000, preferably within a region spanning from nucleotide position around +3931 to nucleotide position around +5000, more preferably within a region spanning from nucleotide position around +4070 to nucleotide position around +5000, wherein the nucleotide position is relative to the transcription start of the GAPDH mRNA.
A non-translated genomic DNA sequence downstream of the eukaryotic GAPDH promoter which starts e.g. downstream of the eukaryotic GAPDH polyadenylation site used in the present invention usually starts at a nucleotide position around position +3931, preferably at a nucleotide position around +4070, wherein the nucleotide position is relative to the transcription start of the GAPDH mRNA.

In human the non-translated genomic DNA sequence downstream of the human GAPDH polyadenylation site starts at around nucleotide position +3931 (relative to the transcription start of the GAPDH mRNA which corresponds to bp 8463 as shown in SEQ ID NO: 17). Preferably, if the non-translated genomic DNA sequence downstream of the GAPDH polyadenylation site is from human, the non-translated genomic DNA sequence downstream of the GAPDH polyadenylation site starts at around +3931 (relative to the transcription start of the GAPDH mRNA; which corresponds to bp 8463 as shown in SEQ ID NO: 17) and its length is around 3357 bps corresponding to the sequence from around bps 8463 to around 11819 as shown in SEQ ID NO: 17, more preferably it starts at around +4070 (relative to the transcription start of the GAPDH mRNA which corresponds to bp 8602 as shown in SEQ ID NO: 17) and its length is around 3218 bps corresponding to the sequence from around bps 8602 to around 11819 as shown in SEQ ID NO: 17 .

In a further embodiment, the non-translated genomic DNA sequence downstream of a eukaryotic GAPDH promoter comprises the nucleotide sequence selected from the group consisting of SEQ ID NOs: 8 and 21 or fragments thereof.

In a further embodiment, the non-translated genomic DNA sequence downstream of the eukaryotic GAPDH promoter comprises a nucleotide sequence complementary to the nucleotide sequence selected from the group consisting of SEQ ID NOs: 8 and 21 or fragments thereof.

In a further embodiment, the non-translated genomic DNA sequence downstream of the eukaryotic GAPDH promoter comprises a nucleotide sequence at least 80% identical to the nucleotide sequence selected from the group consisting of SEQ ID NOs: 8 and 21 or fragments thereof.

In some embodiments, the nucleotide sequence selected from the group consisting of SEQ ID NOs: 8 and 21 or fragments thereof, comprises five or less, preferably four or less, more preferably three or less, most preferred two or less, in particular one nucleic acid modification, wherein the nucleic acid modification(s) are preferably a nucleic acid substitution.

In a further embodiment, the length of the non-translated genomic DNA sequence downstream of the eukaryotic GAPDH promoter is preferably from around 200 to around 8000 nucleotides, more preferably from around 500 to around 5000 nucleotides, even more preferably from around 1000 to around 4500 nucleotides, most preferably from around 1500 to around 4000 nucleotides, in particular from around 2000 to around 3500 nucleotides, more particular from around 2700 to around 3300, even more particular around 3200, most particular 3218 nucleotides. The length of the non-translated genomic DNA sequence downstream of the eukaryotic GAPDH promoter as defined herein does not include any linker sequences added to the non-translated genomic DNA sequence.

In a further embodiment, the non-translated genomic DNA sequence downstream of the eukaryotic GAPDH promoter is orientated in the same direction as the polynucleotide sequence encoding a polypeptide.

In a further embodiment, the non-translated genomic DNA sequence downstream of the eukaryotic GAPDH promoter is orientated in opposite direction in relation to the polynucleotide sequence encoding a polypeptide.

In some embodiments, the expression cassette which comprises a promoter, a polynucleotide sequence encoding a polypeptide, and a non-translated genomic DNA sequence downstream of a eukaryotic GAPDH promoter further comprises a non-translated genomic DNA sequence upstream of a eukaryotic GAPDH promoter, wherein the non-translated genomic DNA sequence upstream of the eukaryotic GAPDH promoter starts within a region spanning from around the 5' end of the eukaryotic GAPDH promoter to nucleotide position around -3500, wherein the nucleotide position is relative to the transcription start of the GAPDH mRNA, and wherein the length of the non-translated genomic DNA sequence upstream of the eukaryotic GAPDH promoter is from around 200 to around 2800 nucleotides.

In a another embodiment, the expression cassette comprises a promoter, a polynucleotide sequence encoding a polypeptide, and a non-translated genomic DNA sequence upstream of a eukaryotic GAPDH promoter, wherein the polypeptide encoded by the polynucleotide sequence is not GAPDH, and wherein the non-translated genomic DNA sequence upstream of the eukaryotic GAPDH promoter starts within a region spanning from around the 5' end of the eukaryotic GAPDH promoter to nucleotide position around -3500, wherein the nucleotide position is relative to the transcription start of the GAPDH mRNA, wherein the length of the non-translated genomic DNA sequence upstream of the eukaryotic GAPDH promoter is from 200 to around 2800 nucleotides, with the proviso that the expression cassette does not comprise a eukaryotic GAPDH promoter or fragments thereof.

In some embodiments, the expression cassette further comprises a non-translated genomic DNA sequence downstream of a eukaryotic GAPDH promoter, wherein the non-translated genomic DNA sequence downstream of the eukaryotic GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the eukaryotic GAPDH promoter is from around 200 to around 2800 nucleotides. In these embodiments the non-translated genomic DNA sequence downstream of a eukaryotic GAPDH promoter used is e.g. as described *supra.*

In a further embodiment, the length of the non-translated genomic DNA sequence upstream of the eukaryotic GAPDH promoter is at least around 100 nucleotides and extends at its maximum to the start codon of the NCAPD2 gene. In a further embodiment, the length of the non-translated genomic DNA sequence upstream of the eukaryotic GAPDH promoter is at least around 100 nucleotides and extends at its maximum to the third last intron of the NCAPD2 gene. In a further embodiment, the length of the non-translated genomic DNA sequence upstream of the eukaryotic GAPDH promoter is at least around 100 nucleotides and extends at its maximum to the second last intron of the NCAPD2 gene. In a further embodiment, the length of the non-translated genomic DNA sequence upstream of the eukaryotic GAPDH promoter is at least around 100 nucleotides and extends at its maximum to the last intron of the NCAPD2 gene.

The human NCAPD2 gene (NCBI gene ID: 9918) is located in human DNA around bps 6603298 to 6641132 of chromosome 12. In one embodiment, the length of the non-translated genomic DNA sequence upstream of a eukaryotic GAPDH promoter extending at its maximum to the last intron of the NCAPD2 gene in human stretches at its maximum to around 6640243 bps of chromosome 12 coding for the NCAPD2 gene in human (position -3414 relative to the transcription start of the GAPDH gene which corresponds to bp 1119 in SEQ ID NO: 17).

In one embodiment, the length of the non-translated genomic DNA sequence upstream of a eukaryotic GAPDH promoter extending at its maximum to the second last intron of the NCAPD2 gene in human stretches at its maximum to around 6639984 bps of chromosome 12 coding for the NCAPD2 gene in human (position -3673 relative to the transcription start of the GAPDH gene which corresponds to bp 860 in SEQ ID NO: 17).

In one embodiment, the length of the non-translated genomic DNA sequence upstream of a eukaryotic GAPDH promoter extending at its maximum to the third last intron of the NCAPD2 gene in human stretches at its maximum to around 6639125 bps of chromosome 12 coding for the NCAPD2 gene in human (position -4532 relative to the transcription start of the GAPDH gene; which corresponds to bp 1 in SEQ ID NO: 17).

The non-translated genomic DNA sequence upstream of a eukaryotic GAPDH promoter extending at its maximum to the last intron, to the second last intron and to the third last intron of the NCAPD2 gene in human, respectively are included in SEQ ID NO: 17, which shows bps 6657230 to 6639125 of chromosome 12 (NCBI gene ID: 9918).

The mouse NCAPD2 gene (Gene ID: 68298) is located in mouse DNA around position 125118025 to 125141604 of chromosome 6. In one embodiment, the length of the non-translated genomic DNA sequence upstream of a eukaryotic GAPDH promoter (estimated to have a a length of 500 bps upstream of the transcription start) extending at its maximum to the last intron of the NCAPD2 gene in mouse stretches at its maximum to around bps 125118607 of chromosome 6 coding for the NCAPD2 gene in mouse.

In one embodiment, the length of the non-translated genomic DNA sequence upstream of a eukaryotic GAPDH promoter extending at its maximum to the second last intron of the NCAPD2 gene in mouse stretches at its maximum to around 125118880 bps of chromosome 6 coding for the NCAPD2 gene in mouse.

In one embodiment, the length of the non-translated genomic DNA sequence upstream of a eukaryotic GAPDH promoter extending at its maximum to the third last intron of the NCAPD2 gene in mouse stretches at its maximum to around 125119832 bps of chromosome 6 coding for the NCAPD2 gene in mouse.
The non-translated genomic DNA sequence upstream of a eukaryotic GAPDH promoter extending at its maximum to the last intron, to the second last intron and to the third last intron of the NCAPD2 gene in mouse, respectively are included in SEQ ID NO: 18, which shows bps 125103521 to 125119832 of chromosome 6 (NCBI gene ID: 68298). The non-translated genomic DNA sequence upstream of a eukaryotic GAPDH promoter extending to the last intron stretches to around bps 1226 of the nucleotide sequence as shown by SEQ ID NO: 18 (-3006 relative to the transcription start of the mouse GAPDH mRNA). The non-translated genomic DNA sequence downstream of a eukaryotic GAPDH promoter extending to the second last intron stretches to around bps 953 of the nucleotide sequence as shown by SEQ ID NO: 18 (-3279 relative to the transcription start of the mouse GAPDH mRNA). The non-translated genomic DNA sequence downstream of a eukaryotic GAPDH promoter extending to the third last intron stretches to around bp 1 of the nucleotide sequence as shown by SEQ ID NO: 18 (-4231 relative to the transcription start of the mouse GAPDH mRNA).

The rat NCAPD2 gene (Gene ID: 362438) is located in eukaryotic DNA around position 161288671 to 161310417 of chromosome 4. In one embodiment, the length of the non-translated genomic DNA sequence upstream of a eukaryotic GAPDH promoter extending at its maximum to the last intron of the NCAPD2 gene in rat stretches at its maximum to around 161289191 bps of chromosome 4 coding for the NCAPD2 gene in rat. In one embodiment, the length of the non-translated genomic DNA sequence upstream of a eukaryotic GAPDH promoter extending at its maximum to the second last intron of the NCAPD2 gene in rat stretches at its maximum to around 161289446 bps of chromosome 4 coding for the NCAPD2 gene in rat. In one embodiment, the length of the non-translated genomic DNA sequence upstream of a eukaryotic GAPDH promoter extending at its maximum to the third last intron of the NCAPD2 gene in rat stretches at its maximum to around 161290508 bps of chromosome 4 coding for the NCAPD2 gene in rat.

The non-translated genomic DNA sequence upstream of a eukaryotic GAPDH promoter extending at its maximum to the last intron, to the second last intron and to the third last intron of the NCAPD2 gene in rat, respectively are included in SEQ ID NO: 19, which shows bps 161279451 to 161290508 of chromosome 4 (NCBI gene ID: 362438). The non-translated genomic DNA sequence upstream of a eukaryotic GAPDH promoter extending to the last intron stretches to around bps 1318 of the nucleotide sequence as shown by SEQ ID NO: 19 (-3101 relative to the transcription start of rat GAPDH mRNA). The non-translated genomic DNA sequence downstream of a eukaryotic GAPDH promoter extending to the second last intron stretches to around bps 1063 of the nucleotide sequence as shown by SEQ ID NO: 19 (position -3356 relative to the transcription start of rat GAPDH mRNA). The non-translated genomic DNA sequence downstream of a eukaryotic GAPDH promoter extending to the third last intron stretches to around bp 1 of the nucleotide sequence as shown by SEQ ID NO: 19 (position -4418 relative to the transcription start of rat GAPDH mRNA).

The Chinese hamster NCAPD2 gene (Gene ID: 100753087) is located in eukaryotic DNA around position 3544184 to 3569879. The chromosomal location is not available on the NCBI database. In one embodiment, the length of the non-translated genomic DNA sequence upstream of a eukaryotic GAPDH promoter extending at its maximum to the last intron of the NCAPD2 gene in Chinese hamster stretches at its maximum to around 3569380 bps in Chinese hamster. In one embodiment, the length of the non-translated genomic DNA sequence upstream of a eukaryotic GAPDH promoter extending at its maximum to the second last intron of the NCAPD2 gene in Chinese hamster stretches at its maximum to around 3569131 bps in Chinese hamster. In one embodiment, the length of the non-translated genomic DNA sequence upstream of a eukaryotic GAPDH promoter extending at its maximum to the third last intron of the NCAPD2 gene in Chinese hamster stretches at its maximum to around 3567932 bps in Chinese hamster.

The non-translated genomic DNA sequence upstream of a eukaryotic GAPDH promoter extending at its maximum to the last intron, to the second last intron and to the third last intron of the NCAPD2 gene in Chinese hamster, respectively are included in SEQ ID NO: 29, which shows bps 3567932 to 3585061. The non-translated genomic DNA sequence upstream of a eukaryotic GAPDH promoter extending to the last intron stretches to around bps 1449 of the nucleotide sequence as shown by SEQ ID NO: 29 (-2752 relative to the transcription start of Chinese hamster GAPDH mRNA). The non-translated genomic DNA sequence downstream of a eukaryotic GAPDH promoter extending to the second last intron stretches to around bps 1200 of the nucleotide sequence as shown by SEQ ID NO: 29 (position -3001 relative to the transcription start of Chinese hamster GAPDH mRNA). The non-translated genomic DNA sequence downstream of a eukaryotic GAPDH promoter extending to the third last intron stretches to around bp 1 of the nucleotide sequence as shown by SEQ ID NO: 29 (position -4200 relative to the transcription start of Chinese hamster GAPDH mRNA).

In human the non-translated genomic DNA sequence upstream of the human GAPDH promoter starts at around nucleotide position -463 (relative to the transcription start of the GAPDH mRNA which corresponds to bp 4533 as shown in SEQ ID NO: 17). Preferably, if the non-translated genomic DNA sequence upstream of the GAPDH promoter is from human, the non-translated genomic DNA sequence upstream of the GAPDH promoter starts at around -500 (relative to the transcription start of the GAPDH mRNA; which corresponds to bp 4533 as shown in SEQ ID NO: 17). More preferably, if the non-translated genomic DNA sequence upstream of the GAPDH promoter is from human, the non-translated genomic DNA sequence upstream of the GAPDH promoter starts at around -576 (relative to the transcription start of the GAPDH mRNA; which corresponds to bp 4533 as shown in SEQ ID NO: 17) and its length is around 3158 bps corresponding to the sequence from around bps 800 to around 3957 as shown in SEQ ID NO: 17.

In a further embodiment, the non-translated genomic DNA sequence upstream of the eukaryotic GAPDH promoter comprises a nucleotide sequence selected from the group consisting of SEQ ID NOs: 7, 9, 10, 11, 12, 13, 14, 15, 16, 20, 22, 23, 24, 25, 26, 27 and 28 or fragments thereof, preferably a nucleotide sequence selected from the group consisting of SEQ ID NOs: 7, 9, 10, 11, 12, 13, 14, 15 and 16 or fragments thereof, or a nucleotide sequence selected from the group consisting of SEQ ID NOs: 20, 22, 23, 24, 25, 26, 27, 28 and 16 or fragments thereof. More preferred is a nucleotide sequence selected from the group consisting of SEQ ID NOs: 10, 12, 15 and 16 or fragments thereof, more preferably a nucleotide sequence selected from the group consisting of SEQ ID NOs: 10, 12, 15 and 16 or fragments thereof, wherein nucleotide sequences comprising SEQ ID NOs: 10 and/or 16 are orientated in opposite direction in relation to the polynucleotide sequence encoding a polypeptide, and nucleotide sequences comprising SEQ ID NOs: 12 and/or 15 are orientated in the same direction as the polynucleotide sequence encoding a polypeptide. Equally more preferred is a nucleotide sequence selected from the group consisting of SEQ ID NOs: 23, 25, 28 and 16 or fragments thereof, more preferably a nucleotide sequence selected from the group consisting of SEQ ID NOs: 23, 25, 28 and 16 or fragments thereof, wherein nucleotide sequences comprising SEQ ID NOs: 23 and/or 16 are orientated in opposite direction in relation to the polynucleotide sequence encoding a polypeptide, and nucleotide sequences comprising SEQ ID NOs: 25 and/or 28 are orientated in the same direction as the polynucleotide sequence encoding a polypeptide.

In a further embodiment, the non-translated genomic DNA sequence upstream of the eukaryotic GAPDH promoter comprises a nucleotide sequence complementary to the nucleotide sequence selected from the group consisting of SEQ ID NOs: 7, 9, 10, 11, 12, 13, 14, 15, 16, 20, 22, 23, 24, 25, 26, 27 and 28 or fragments thereof, preferably a nucleotide sequence complementary to the nucleotide sequence selected from the group consisting of SEQ ID NOs: 7, 9, 10, 11, 12, 13, 14, 15 and 16 or fragments thereof, or a nucleotide sequence complementary to the nucleotide sequence selected from the group consisting of SEQ ID NOs: 20, 22, 23, 24, 25, 26, 27, 28 and 16 or fragments thereof. More preferred is a nucleotide sequence complementary to the nucleotide sequence selected from the group consisting of SEQ ID NOs: 10, 12, 15 and 16 or fragments thereof. Equally more preferred is a nucleotide sequence complementary to the nucleotide sequence selected from the group consisting of SEQ ID NOs: 23, 25, 28 and 16 or fragments thereof.

In a further embodiment, the non-translated genomic DNA sequence upstream of the eukaryotic GAPDH promoter comprises a nucleotide sequence at least 80% identical to the nucleotide sequence selected from the group consisting of SEQ ID NOs: 7, 9, 10, 11, 12, 13, 14, 15, 16, 20, 22, 23, 24, 25, 26, 27 and 28 or fragments thereof, preferably a nucleotide sequence at least 80% identical to the nucleotide sequence selected from the group consisting of SEQ ID NOs: 7, 9, 10, 11, 12, 13, 14, 15 and 16 or fragments thereof, or a nucleotide sequence at least 80% identical to the nucleotide sequence selected from the group consisting of SEQ ID NOs: 20, 22, 23, 24, 25, 26, 27, 28 and 16 or fragments thereof. More preferred is a nucleotide sequence at least 80% identical to the nucleotide sequence selected from the group consisting of SEQ ID NOs: 10, 12, 15 and 16 or fragments thereof, more preferably a nucleotide sequence at least 80% identical to the nucleotide sequence selected from the group consisting of SEQ ID NOs: 10, 12, 15 and 16 or fragments thereof, wherein nucleotide sequences comprising SEQ ID NOs: 10 and/or 16 are orientated in opposite direction in relation to the polynucleotide sequence encoding a polypeptide, and nucleotide sequences comprising SEQ ID NOs: 12 and/or 15 are orientated in the same direction as the polynucleotide sequence encoding a polypeptide. Equally more preferred is a nucleotide sequence at least 80% identical to the nucleotide sequence selected from the group consisting of SEQ ID NOs: 23, 25, 28 and 16 or fragments thereof, more preferably a nucleotide sequence at least 80% identical to the nucleotide sequence selected from the group consisting of SEQ ID NOs: 23, 25, 28 and 16 or fragments thereof, wherein nucleotide sequences comprising SEQ ID NOs: 23 and/or 16 are orientated in opposite direction in relation to the polynucleotide sequence encoding a polypeptide, and nucleotide sequences comprising SEQ ID NOs: 25 and/or 28 are orientated in the same direction as the polynucleotide sequence encoding a polypeptide.

In some embodiments, the nucleotide sequence selected from the group consisting of SEQ ID NOs: 7, 9, 10, 11, 12, 13, 14, 15, 16, 20, 22, 23, 24, 25, 26, 27 and 28 or fragments thereof, comprises five or less, preferably four or less, more preferably three or less, most preferred two or less, in particular one nucleic acid modification, wherein the nucleic acid modification(s) are preferably a nucleic acid substitution.

In some embodiments, the nucleotide sequence selected from the group consisting of SEQ ID NOs: 7, 9, 11, 14, 20, 22, 24 and 27 or fragments thereof, comprises five or less, preferably four or less, more preferably three or less, most preferred two or less, in particular one nucleic acid modification, wherein the nucleic acid modification(s) are preferably a nucleic acid substitution.

In some embodiments, the nucleotide sequence selected from the group consisting of SEQ ID NOs: 7, 9, 11, 14, or fragments thereof, comprises one nucleic acid substitution at position 16 relative to the start of the nucleotide sequence of SEQ ID NOs: 7, 9, 11, 14. Preferably G at position 16 relative to the start of the nucleotide sequence is replaced with T.

In some embodiments, the nucleotide sequence selected from the group consisting of SEQ ID NOs: 20, 22, 24 and 27 or fragments thereof, comprises one nucleic acid substitution at position 13 relative to the start of the nucleotide sequence of SEQ ID NOs: 20, 22, 24 and 27. Preferably G at position 13 relative to the start of the nucleotide sequence is replaced with T.

In a further embodiment, the non-translated genomic DNA sequence upstream of the eukaryotic GAPDH promoter is orientated in the same direction as the polynucleotide sequence encoding a polypeptide.

In a further embodiment, the non-translated genomic DNA sequence upstream of the eukaryotic GAPDH promoter is orientated in opposite direction in relation to the polynucleotide sequence encoding a polypeptide.

In a preferred embodiment, the expression cassette comprises a promoter, a polynucleotide sequence encoding a polypeptide, and a non-translated genomic DNA sequence downstream of a eukaryotic GAPDH promoter and a non-translated genomic DNA sequence upstream of a eukaryotic GAPDH promoter as described *supra.* Preferably the origin of the non-translated genomic DNA sequence downstream of a eukaryotic GAPDH promoter and the non-translated genomic DNA sequence upstream of a eukaryotic GAPDH promoter is the same i.e. is of the same species. More preferably the origin of the non-translated genomic DNA sequence downstream of a eukaryotic GAPDH promoter, the non-translated genomic DNA sequence upstream of a eukaryotic GAPDH promoter and the host cell is the same i.e. is of the same species, e.g. the origin of the non-translated genomic DNA sequence downstream of a eukaryotic GAPDH promoter, the non-translated genomic DNA sequence upstream of a eukaryotic GAPDH promoter and the host cell is from the same mammal e.g. from human.

In some embodiments, if the non-translated genomic DNA sequence downstream and/or upstream of the eukaryotic GAPDH promoter is non-translated genomic DNA sequence from one species, the promoter of the expression cassette is not a GAPDH promoter from the same species.

In some embodiments, if the non-translated genomic DNA sequence downstream and/or upstream of the eukaryotic GAPDH promoter is non-translated genomic DNA sequence downstream and/or upstream of human origin, the promoter of the expression cassette is not a human GAPDH promoter.

In some embodiments, the promoter of the expression cassette is not a GAPDH promoter.
In one embodiment, if the expression cassette comprises a promoter, a polynucleotide sequence encoding a polypeptide, and a non-translated genomic DNA sequence downstream of a eukaryotic Glyceraldehyde 3-phosphate dehydrogenase (GAPDH) promoter, wherein the polypeptide encoded by the polynucleotide sequence is not GAPDH, and wherein the non-translated genomic DNA sequence downstream of the eukaryotic GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the eukaryotic GAPDH promoter is from around 200 to around 2800 nucleotides and wherein the expression cassette further comprises a non-translated genomic DNA sequence upstream of a eukaryotic GAPDH promoter, wherein the non-translated genomic DNA sequence upstream of the eukaryotic GAPDH promoter starts within a region spanning from around the 5' end of the eukaryotic GAPDH promoter to nucleotide position around -3500, wherein the nucleotide position is relative to the transcription start of the GAPDH mRNA, and wherein the length of the non-translated genomic DNA sequence upstream of the eukaryotic GAPDH promoter is from around 200 to around 2800 nucleotides, the promoter of the expression cassette may be a eukaryotic GAPDH promoter, preferably a mammalian GAPDH promoter, more preferably a rodent or human GAPDH promoter. In this embodiment the non-translated genomic DNA sequence upstream of the eukaryotic GAPDH promoter starting within a region spanning from around the 5' end of the eukaryotic GAPDH promoter to nucleotide position around -3500 is preferably located directly upstream of the eukaryotic GAPDH promoter, more preferably in this embodiment the expression cassette comprises the naturally occurring genomic DNA sequence comprising the eukaryotic GAPDH promoter and extending to nucleotide position around -3500, wherein the nucleotide position is relative to the transcription start of the GAPDH mRNA.

In some embodiments, the non-translated genomic DNA sequence downstream and/or upstream of the eukaryotic GAPDH promoter is of mammalian origin, e.g. the eukaryotic GAPDH promoter is a mammalian GAPDH promoter and non-translated genomic DNA sequence downstream and/or upstream of the mammalian GAPDH promoter is used as described herein.

In some embodiments, the non-translated genomic DNA sequence downstream and/or upstream of the eukaryotic GAPDH promoter is of rodent or human origin, e.g. the eukaryotic GAPDH promoter is a rodent or human GAPDH promoter and non-translated genomic DNA sequence downstream and/or upstream of the rodent or the human GAPDH promoter is used as described herein.

Preferably the non-translated genomic DNA sequence downstream and/or upstream of the eukaryotic GAPDH promoter is selected from human, rat or mouse origin, more preferably from human or mouse origin, most preferably from human origin.

In some embodiments, the non-translated genomic DNA sequence downstream and/or upstream of the eukaryotic GAPDH promoter is not operably linked to the polynucleotide sequence encoding the polypeptide.

In some embodiments, the expression cassette comprises a polyadenylation site. Preferably the polyadenylation site is selected from the group consisting of SV40 poly(A) and BGH (Bovine Growth Hormone) poly(A).

In some embodiments, the promoter and the polynucleotide sequence encoding a polypeptide of the expression cassette are operably linked.

In some embodiments, the promoter of the expression cassette is selected from the group consisting of SV40 promoter, human tk promoter, MPSV promoter, mouse CMV, human CMV, rat CMV, human EF1 alpha, Chinese hamster EF1 alpha, human GAPDH, hybrid promoters including MYC, HYK and CX promoter.

In some embodiments, the polypeptide encoded by the expression cassette can be a non-glycosylated and glycosylated polypeptide. Glycosylated polypeptides refer to polypeptides having at least one oligosaccharide chain.

Examples for non-glycosylated proteins are e. g. non-glycosylated hormones; non-glycosylated enzymes; non-glycosylated growth factors of the nerve growth factor (NGF) family, of the epithelial growth factor (EGF) and of the fibroblast growth factor (FGF) family and non-glycosylated receptors for hormones and growth factors.

Examples for glycosylated proteins are hormones and hormone releasing factors, clotting factors, anti-clotting factors, receptors for hormones or growth factors, neurotrophic factors cytokines and their receptors, T-cell receptors, surface membrane proteins, transport proteins, homing receptors, addressins, regulatory proteins, antibodies, chimeric proteins, such as immunoadhesins, and fragments of any of the glycosylated proteins. Preferably the polypeptide is selected from the group consisting of antibodies, antibody fragments or antibody derivates (e.g. Fc fusion proteins and particular antibody formats like bispecific antibodies). Antibody fragment as used herein includes, but is not limited to, (i) a domain, (ii) the Fab fragment consisting of VL, VH, CL or CK and CH1 domains, including Fab' and Fab'-SH, (iii) the Fd fragment consisting of the VH and CH1 domains, (iv) the dAb fragment (Ward ES et al., (1989) Nature, 341(6242): 544-6) which consists of a single variable domain (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site (Bird RE et al., (1988) Science, 242(4877): 423-6; Huston JS et al., (1988) Proc Natl Acad Sci U S A, 85(16): 5879-83), (vii) "diabodies" or "triabodies", multivalent or multispecific fragments constructed by gene fusion (Holliger P et al., (1993) Proc Natl Acad Sci U S A, 90(14): 6444-8; Holliger P et al., (2000) Methods Enzymol, 326: 461-79), (viii) scFv, diabody or domain antibody fused to an Fc region and (ix) scFv fused to the same or a different antibody.

In some embodiments the expression cassette further comprises a genetic element selected from the group consisting of an additional promoter, an enhancer, transcriptional control elements, and a selectable marker, preferably a selectable marker which is expressed in animal cells. Transcriptional control elements are e.g. Kozak sequences or transcription terminator elements.

In one embodiment, the genetic element is a selectable marker wherein the content of CpG sites contained in the polynucleotide sequence encoding the selectable marker is 45 or less, preferably 40 or less, more preferably 20 or less, in particular 10 or less, more particular 5 or less, most particular 0 (CpG sites have been completely removed).

In a further aspect, the present disclosure provides an expression vector, preferably a mammalian expression vector comprising an expression cassette as described *supra.*
In some embodiments, the expression vector comprises at least two separate transcription units. An expression vector with two separate transcription units is also referred to as a double-gene vector. An example thereof is a vector, in which the first transcription unit encodes the heavy chain of an antibody or a fragment thereof and the second transcription unit encodes the light chain of an antibody. Another example is a double-gene vector, in which the two transcription units encode two different subunits of a protein such as an enzyme. However, it is also possible that the expression vector of the present invention comprises more than two separate transcription units, for example three, four or even more separate transcription units each of which comprises a different nucleotide sequence encoding a different polypeptide chain. An example therefore is a vector with four separate transcription units, each of which contains a different nucleotide sequence encoding one subunit of an enzyme consisting of four different subunits.

In some embodiments, the expression vector further comprises a genetic element selected from the group consisting of an additional promoter, an enhancer, transcriptional control elements, an origin of replication and a selectable marker.

In some embodiments, the expression vector further comprises an origin of replication and a selectable marker wherein the content of the CpG sites contained in the polynucleotide sequence of the expression vector encoding the origin of replication and the selectable marker is 200 or less, preferably 150 or less, in particular 100 or less, more particular 50 or less, most particular 30 or less.

Any selection marker commonly employed such as thymidine kinase (tk), dihydrofolate reductase (DHFR), puromycin, neomycin or glutamine synthetase (GS) may be used for the expression cassette or the expression vector of the present invention. Preferably, the expression vectors of the invention also comprise a limited number of useful restriction sites for insertion of the expression cassette for the secretion of a heterologous protein of the present invention. Where used in particular for transient/episomal expression only, the expression vectors of the invention may further comprise an origin of replication such as the oriP origin of Epstein Barr Virus (EBV) or SV40 virus for autonomous replication/episomal maintenance in eukaryotic host cells but may be devoid of a selectable marker. Transient expression in cell lacking relevant factors to facilitate replication of the vector is also possible. The expression vector harbouring the expression cassette may further comprise an expression cassette coding for a fluorescent marker, an expression cassette coding for an ncRNA, an expression cassette coding for an antiapoptotic protein, or an expression cassette coding for a protein increasing the capacity of the secretory pathway.

In a further aspect, the present disclosure provides an expression vector, which comprises in order:
a) a non-translated genomic DNA sequence upstream of a mammalian GAPDH promoter, wherein the non-translated genomic DNA sequence upstream of the mammalian GAPDH promoter starts within a region spanning from the 5' end of the mammalian GAPDH promoter to nucleotide position around -3500, wherein the nucleotide position is relative to the transcription start of the GAPDH mRNA, wherein the length of the genomic DNA sequence upstream of the mammalian GAPDH promoter is from 200 to around 2800 nucleotides
b) a promoter
c) a polynucleotide sequence encoding a polypeptide
d) a polyadenylation site
e) an enhancer
f) a non-translated genomic DNA sequence downstream of a mammalian GAPDH promoter,
wherein the polypeptide encoded by the polynucleotide sequence is not GAPDH, wherein the genomic DNA sequence downstream of the mammalian GAPDH promoter starts within a region spanning from nucleotide position around+ 1 to nucleotide position around +7000, wherein the nucleotide position is relative to the transcription start of the GAPDH mRNA, and wherein the length of the non translated genomic DNA sequence downstream of the mammalian GAPDH promoter is from around 200 to around 2800 nucleotides.

In some embodiments, the present disclosure provides an expression vector, which comprises in order:
a) a non-translated genomic DNA sequence upstream of a eukaryotic GAPDH promoter
b) a promoter
c) a polynucleotide sequence encoding a polypeptide
d) a polyadenlyation site
e) an enhancer
f) a non-translated genomic DNA sequence downstream of a eukaryotic GAPDH promoter,
wherein inclusion of the enhancer is optional.

In a further aspect, the present disclosure provides an expression vector, which comprises in order:
a) a non-translated genomic DNA sequence upstream of a eukaryotic GAPDH promoter
b) an enhancer
c) a promoter
d) a polynucleotide sequence encoding a polypeptide
e) a polyadenlyation site
f) a non-translated genomic DNA sequence downstream of a eukaryotic GAPDH,
wherein inclusion of the enhancer is optional.

In a further aspect, the present disclosure provides an expression vector, which comprises in order:
a) an enhancer
b) a non-translated genomic DNA sequence upstream of a eukaryotic GAPDH
c) a promoter
d) a polynucleotide sequence encoding a polypeptide
e) a polyadenlyation site
f) non-translated genomic DNA sequence downstream of a eukaryotic GAPDH,
wherein inclusion of the enhancer is optional.

In a further aspect, the present disclosure provides an expression vector, which comprises in order:
a) a non-translated genomic DNA sequence downstream of a eukaryotic GAPDH promoter
b) a promoter
c) a polynucleotide sequence encoding a polypeptide
d) a polyadenlyation site
e) an enhancer
f) a non-translated genomic DNA sequence upstream of a eukaryotic GAPDH promoter,
wherein inclusion of the enhancer is optional.

In a further aspect, the present disclosure provides an expression vector, which comprises in order:
a) a non-translated genomic DNA sequence downstream of a eukaryotic GAPDH promoter
b) an enhancer
c) a promoter
d) a polynucleotide sequence encoding a polypeptide
e) a polyadenlyation site
f) a non-translated genomic DNA sequence upstream of a eukaryotic GAPDH,
wherein inclusion of the enhancer is optional.

In a further aspect, the present disclosure provides an expression vector, which comprises in order:
a) an enhancer
b) a non-translated genomic DNA sequence downstream of a eukaryotic GAPDH
c) a promoter
d) a polynucleotide sequence encoding a polypeptide
e) a polyadenlyation site
f) non-translated genomic DNA sequence upstream of a eukaryotic GAPDH,
wherein inclusion of the enhancer is optional.

Non-translated genomic DNA sequence upstream of a eukaryotic GAPDH, enhancer, promoter, polynucleotide sequence encoding a polypeptide, polyadenlyation site and non-translated genomic DNA sequence downstream of a eukaryotic GAPDH promoter of the expression vectors are e.g. as described *supra.*

In a further aspect, the present disclosure provides a host cell comprising an expression cassette or an expression vector as described *supra.* The host cell can be a human or non-human cell. Preferred host cells are mammalian cells. Preferred examples of mammalian host cells include, without being restricted to, Human embryonic kidney cells (Graham FL et al., J. Gen. Virol. 36: 59-74), MRC5 human fibroblasts, 983M human melanoma cells, MDCK canine kidney cells, RF cultured rat lung fibroblasts isolated from Sprague-Dawley rats, B16BL6 murine melanoma cells, P815 murine mastocytoma cells, MT1 A2 murine mammary adenocarcinoma cells, PER:C6 cells (Leiden, Netherlands) and Chinese hamster ovary (CHO) cells or cell lines (Puck et al., 1958, J. Exp. Med. 108: 945-955).

In a particular preferred embodiment the host cell is a Chinese hamster ovary (CHO) cell or cell line. Suitable CHO cell lines include e.g. CHO-S (Invitrogen, Carlsbad, CA, USA), CHO K1 (ATCC CCL-61), CHO pro3-, CHO DG44, CHO P12 or the dhfr- CHO cell line DUK-BII (Chasin et al., PNAS 77, 1980, 4216-4220), DUXBI 1 (Simonsen et al., PNAS 80, 1983, 2495-2499), or CHO-K1SV (Lonza, Basel, Switzerland).

In a further aspect, the present disclosure provides an *in vitro* method for the expression of a polypeptide, comprising transfecting a host cell with the expression cassette or an expression vector as described *supra* and recovering the polypeptide. The polypeptide is preferably a heterologous, more preferably a human polypeptide.

For transfecting the expression cassette or the expression vector into a host cell according to the present invention any transfection technique such as those well-known in the art, e.g. electoporation, calcium phosphate co-precipitation, DEAE-dextran transfection, lipofection, can be employed if appropriate for a given host cell type. It is to be noted that the host cell transfected with the expression cassette or the expression vector of the present invention is to be construed as being a transiently or stably transfected cell line. Thus, according to the present invention the present expression cassette or the expression vector can be maintained episomally i.e. transiently transfected or can be stably integrated in the genome of the host cell i.e. stably transfected.

A transient transfection is characterised by non-appliance of any selection pressure for a vector borne selection marker. In transient expression experiments which commonly last 2 to up to 10 days post transfection, the transfected expression cassette or expression vector are maintained as episomal elements and are not yet integrated into the genome. That is the transfected DNA does not usually integrate into the host cell genome. The host cells tend to lose the transfected DNA and overgrow transfected cells in the population upon culture of the transiently transfected cell pool. Therefore expression is strongest in the period immediately following transfection and decreases with time. Preferably, a transient transfectant according to the present invention is understood as a cell that is maintained in cell culture in the absence of selection pressure up to a time of 2 to 10 days post transfection.

In a preferred embodiment of the invention the host cell e.g. the CHO host cell is stably transfected with the expression cassette or the expression vector of the present invention. Stable transfection means that newly introduced foreign DNA such as vector DNA is becoming incorporated into genomic DNA, usually by random, non-homologous recombination events. The copy number of the vector DNA and concomitantly the amount of the gene product can be increased by selecting cell lines in which the vector sequences have been amplified after integration into the DNA of the host cell. Therefore, it is possible that such stable integration gives rise, upon exposure to further increases in selection pressure for gene amplification, to double minute chromosomes in CHO cells. Furthermore, a stable transfection may result in loss of vector sequence parts not directly related to expression of the recombinant gene product, such as e.g. bacterial copy number control regions rendered superfluous upon genomic integration. Therefore, a transfected host cell has integrated at least part or different parts of the expression cassette or the expression vector into the genome.

In a further aspect, the present disclosure provides the use of the expression cassette or an expression vector as described *supra* for the expression of a heterologous polypeptide from a mammalian host cell, in particular the use of the expression cassette or an expression vector as described *supra* for the *in vitro* expression of a heterologous polypeptide from a mammalian host cell.

Expression and recovering of the protein can be carried out according to methods known to the person skilled in the art.

For the expression of a polypeptide, the non-translated genomic DNA sequence downstream and/or upstream of a eukaryotic GAPDH promoter of the expression cassette or of the expression vector as described *supra* and the host cell as described *supra* are used and are usually of the same origin. Surprisingly it has been found that an increase of expression is obtained if the non-translated genomic DNA sequence downstream and/or upstream of a eukaryotic GAPDH promoter of the expression cassette or of the expression vector and the host cell are of different origin e.g. if human DNA sequences downstream and/or upstream of a eukaryotic GAPDH promoter are used in CHO cells.

In a further aspect, the present disclosure provides the use of the expression cassette or the expression vector as described *supra* for the preparation of a medicament for the treatment of a disorder.

In a further aspect, the present disclosure provides the expression cassette or the expression vector as described *supra* for use as a medicament for the treatment of a disorder.

In a further aspect, the present disclosure provides the expression cassette or the expression vector as described *supra* for use in gene therapy.

### Examples

### Example 1: Cloning of expression vectors:

### I. Materials and Methods

### I.1 Plasmids constructs

### I.1.1. LB culture plates

500 ml of water were mixed and boiled with 16 g of LB Agar (Invitrogen, Carlsbad, CA, USA) (1 litre of LB contains 10 g tryptone, 5 g yeast extract and 10 g NaCl). After cooling down, the respective antibiotic was added to the solution which is then plated (ampicillin plates at 100 µg/ml and kanamycin plates at 50 µg/ml).

### I.1.2. Polymerase Chain Reaction (PCR)

All PCR were performed using 1 µl of dNTPs (10 mM for each dNTP; Invitrogen, Carlsbad, CA, USA), 2 units of Phusion® DNA Polymerase (Finnzymes Oy, Espoo, Finland), 25 nmol of Primer A (Mycrosynth, Balgach, Switzerland), 25 nmol of Primer B (Mycrosynth, Balgach, Switzerland), 10 µl of 5X HF buffer (7.5 mM MgCl₂, Finnzymes, Espoo, Finland), 1.5 µl of Dimethyl sulfoxide (DMSO, Finnzymes, Espoo, Finland) and 1-3 µl of the template (1-2 µg) in a 50 µl final volume. All primers used are listed in Table 1.

The PCR were started by an initial denaturation at 98°C for 3 minutes, followed by 35 cycles of 30 sec denaturation at 98°C, 30 sec annealing at a primer-specific temperature (according to CG content) and 2 min elongation at 72°C. A final elongation at 72°C for 10 min was performed before cooling and keeping at 4°C.

**Table 1: Summary of primers used in PCRs. GAPDH: Glyceraldehyde 3-phosphate dehydrogenase sequence, 5': upstream sequence, 3: downstream sequence. The "T" (underlined) in primer GlnPrl 172 was introduced in order to avoid the formation of primer dimers.**

| **Primer** | **Primer sequence** | **Sequence amplified** | **Seq ID** |
|---|---|---|---|
| GlnPr 1171 | | 5' GAPDH | SEQ ID No: 1 |
| GlnPr 1172 | | | SEQ ID No: 2 |
| GlnPr 1173 | | 3'GAPDH | SEQ ID No: 3 |
| GlnPr 1174 | | | SEQ ID No: 4 |

### I.1.3. Restriction digest

For all restriction digests around 1 µg of plasmid DNA (quantified with NanoDrop, ND-1000 Spectrophotometer (Thermo Scientific, Wilmington, DE, USA)) was mixed to 10-20 units of each enzyme, 4 µl of corresponding 10X NEBuffer (NEB, Ipswich, MA, USA), and the volume was completed to 40 µl with sterile H₂O. Without further indication, digestions were incubated 1 h at 37°C.

After each preparative digestion of backbone, 1 unit of Calf Intestinal Alkaline Phosphatase (CIP; NEB, Ipswich, MA, USA) was added and the mix was incubated 30 min at 37°C.
If the digest was done in NEBuffer 3 (NEB, Ipswich, MA, USA), the buffer was changed to NEB buffer 4 before adding the CIP because this enzyme has a strong activity in this buffer and may also digest some of the nucleotides at the external ends.

### I.1.4. PCR purification and agarose gel electrophoresis

### I.1.4.1. PCR clean up

To allow digestion all PCR fragments were cleaned prior to restriction digests using the Macherey Nagel Extract II kit (Macherey Nagel, Oensingen, Switzerland) following the manual of the manufacturer using 40 µl of elution buffer. This protocol was also used for changing buffers of DNA samples.

### I.1.4.2. DNA extraction

For gel electrophoresis, 1% gels were prepared using UltraPure™ Agarose (Invitrogen, Carlsbad, CA, USA) and 50X Tris Acetic Acid EDTA buffer (TAE, pH 8.3; Bio RAD, Munich, Germany). For staining of DNA 1 µl of Gel Red Dye (Biotum, Hayward, CA, USA) was added to 100 ml of agarose gel. As a size marker 2 µg of the 1 kb DNA ladder (NEB, Ipswich, MA, USA) was used. The electrophoresis was run for around 1 hour at 125 Volts. The bands of interests were cut out from the agarose gel and purified using the kit Extract II (Macherey-Nagel, Oensingen, Switzerland), following the manual of the manufacturer using 40 µl of elution buffer.

### I.1.5. Ligation

For each ligation, 4 µl of insert were mixed to 1 µl of vector, 400 units of ligase (T4 DNA ligase, NEB, Ipswich, MA, USA), 1 µl of 10X ligase buffer (T4 DNA ligase buffer; NEB, Ipswich, MA, USA) in a 10 µl volume. The mix was incubated for 1-2 h at RT.

### I.1.6. Transformation of ligation products into competent bacteria

For the cloning of pGLEX41-[REP] and for constructs made with the pCR-Blunt vector which contain a standard origin of replication, TOP 10 (One Shot® TOP 10 Competent *E*. *coli*; Invitrogen, Carlsbad, CA, USA) were used.

For replication initiation of plasmid containing the R6K origin of replication, the expression of the π protein, coded by the pir sequence, is required. The π protein is expressed by One Shot® PIR1 competent *E. coli* (Invitrogen, Carlsbad, CA, USA). These bacteria were used for all vectors containing the R6K sequence.

To transform competent bacteria with the ligation product, 25-50 µl of bacteria were thawed on ice for 5 minutes. Then, 3-5 µl of ligation product were added to competent bacteria and incubated for 20-30 min on ice before the thermic shock for 1 minute at 42°C. Then, 500 µl of S.O.C medium (Invitrogen, Carlsbad, CA, USA) were added per tube and incubated for 1 hour at 37°C under agitation. Finally, the bacteria are put on a LB plate with ampicillin (Sigma-Aldrich, St. Louis, MO, USA) and incubated overnight at 37°C. For the cloning in pCR-Blunt vectors, plates with kanamycin (Sigma-Aldrich, St. Louis, MO, USA) were used.

### I.1.7. Plasmid preparation in small (mini) and medium scale (midi)

### I.1.7.1. Minipreparation

For minipreparation, colonies of transformed bacteria were grown for 6-16 hours in 2.5 ml of LB and ampicillin or kanamycin at 37°C, 200 rpm. The DNA was extracted with a plasmid purification kit for *E.coli* (QuickPure, Macherey Nagel, Oensingen, Switzerland), following the provided manual.

Plasmid DNA from minipreparations was quantified once with the NanoDrop ND-1000 Spectrophotometer (Thermo Scientific, Wilmington, DE, USA) by measuring the absorbance at 260 nm and assessing the ratio of the OD260 nm/OD280 nm that had to be between 1.8 and 2. A control digestion was performed before sending the sample to Fasteris SA (Geneva, Switzerland) for sequence confirmation.

For BAC extraction, the QuickPure kit (Macherey Nagel, Oensingen, Switzerland) was used with the following modification of the protocol: 10 ml of LB and chloramphenicol (12.5 µg/ml) (Sigma-Aldrich, St. Louis, MO, USA) were seeded with bacteria containing pBACe3.6 vector. After incubation on a shaking platform at 37°C over night, the culture was centrifuged for 5 min at 13 300 rpm before being resuspended in 500 µl of A1 Buffer. 500 µl of A2 Lysis Buffer were added and the solution was incubated 5 min at RT. Then, it was neutralized with 600 µl of A3 buffer and centrifuged 10 min at 13 300 rpm. The supernatant was loaded on a column and from this step onwards the standard protocol of QuickPure miniprep kit was used.

### I.1.7.2. Midipreparation

For midipreparation, transformed bacteria were grown at 37°C overnight in 200 to 400 ml of LB and ampicillin (or kanamycin). Then, the culture was centrifuged 20 min at 725 g and the plasmid was purified using a commercial kit (NucleoBond Xtra Midi; Macherey Nagel, Oensingen, Switzerland) following the low plasmid protocol provided in the manual of the manufacturer.

Plasmid-DNA from midipreparation was quantified three times with the NanoDrop ND-1000 Spectrophotometer, confirmed by restriction digest and finally sent for sequencing (Fasteris SA, Geneva, Switzerland).

### II. Results and Discussion

### II.1. Cloning of DNA regions upstream and downstream of the GAPDH expression cassette (5' and 3'GAPDH)

The BAC clone RPCIB753F11841Q was ordered at Imagene (Berlin, Germany). This clone contains the human GAPDH sequence in a pBACe3.6 vector backbone, containing a chloramphenicol resistance gene. After DNA extraction by minipreparation, the vector concentration was determined by Nanodrop to 27 ng/µl.

DNA sequences immediately surrounding the GAPDH expression cassette upstream of the promoter and downstream of the poly-adenylation site were amplified by PCR using 27 ng of the purified clone RPCIB753F11841Q as template. The 3 kb fragment upstream of the promoter was amplified with primers GlnPrl 171 (SEQ ID NO: 1) and GlnPrl 172 (SEQ ID NO: 2) leading to the amplicon with SEQ ID No. 5. As primer GlnPrl 172 (SEQ ID NO: 2) carries a base change (G to T) relative to the template sequence, all sequences derived from this PCR reaction will carry this base change, too. The change is located in position -3721 relative to the transcription start of the GAPDH gene (bp 812 of SEQ ID NO: 17, position 23 relative to the start of SEQ ID NO: 5). The 3kb fragment downstream of the polyadenlyation site was amplified with primers GlnPrl 173 (SEQ ID NO: 3) and GlnPrl 174 (SEQ ID NO: 4) leading to the amplicon with SEQ ID NO: 6 (Table 1). The annealing temperature used for these PCRs was 72°C.

The 5'and 3'GAPDH fragments (SEQ ID NOs: 5 and 6) were cloned in pCR-Blunt, a commercially available PCR-product cloning vector (pCR-Blunt, PCR Zero Blunt cloning kit, Invitrogen). The ligation products were transformed into TOP10 competent bacteria and plated on kanamycin LB-agar plates. Colonies were amplified and plasmids were isolated by minipreps. Control digests were performed to identify positives clones yielding pCR-Blunt-5'GAPDH and pCR-Blunt-3'GAPDH constructs.

### II.2. Preparation of the DNA fragment coding for the reporter proteins GFP and a recombinant IgG1 monoclonal antibody (LC-IRES-HC-IRES-GFP)

The reporter construct (REP) used in the present work consisted in a polycistronic gene: IgG1 monoclonal antibody light chain (LC)-IRES- IgG1 monoclonal antibody heavy chain(HC)-IRES- green fluorescent protein (GFP). The presence of Internal Ribosomal Entry Sites (IRES) derived from Encephalomyocarditis virus (Gurtu et al., Biochem Biophys Res Commun.; 229(1): 295-298, 1996)) allows the translation of the 3 peptides IgG1 monoclonal antibody light chain (LC), IgG1 monoclonal antibody heavy chain (HC) and GFP (Fig. 1). Transfected cells will therefore secrete the IgG1 monoclonal antibody and accumulate intracellular GFP in a dependent manner. However, polycistronic mRNAs are not common in eukaryotic cells and their translation is not very efficient, leading to relative low titers of IgG1 and GFP expression.

A vector containing the REP construct was digested using the restriction enzymes NheI and BstBI (BstBI is used at 65°C). The REP fragment containing the expression construct was cut out, purified and used for further cloning steps.

### II.3. Cloning of expression vectors

The vector pGLEX41, an expression vector derived from pcDNA3.1 (+) (Invitrogen, Carlsbad, CA) was used for stable cell line production. It was used as initial backbone that had been modified to generate the second generations of vectors A and B with and without the GAPDH sequences. For all vectors the same promoter-intron combination (mCMV and a donor-acceptor fragment coding for the first intron (IgDA)) was used (Gorman et al., (1990) Proc Natl Acad Sci USA, 87: 5459-5463).

### Cloning of intermediate vector pGLEX41-HM-MCS-ampiA:

The development of the new vector generation was started from pGLEX41. This vector was cut using the restriction enzymes NruI and BspHI in order to release the ampicillin resistance cassette. The backbone fragment was CIPed and purified by gel electrophoresis. The DNA fragment coding for a codon optimized (for expression in *E. coli*) version of the ampicillin resistance gene (including the bla promoter) has been ordered from GeneArt. The insert was cut out of the GeneArt cloning vector #1013237 using the restriction enzymes NruI and BspHI (the same enzymes as used for the backbone), purified and cloned into the backbone. Minipreps were analyzed by restriction digest. The clone pGLEX41-HM-MCS-ampiA#2 had the expected restriction profile and the integration of the correct fragment was confirmed by sequencing.

### Cloning of intermediate vector pGLEX41-MCS-R6K-ampiA

In order to exchange the pUC origin of replication of the vector pGLEX41-HM-MCS-ampiA#2 the vector was digested using PvuI and BspHI. The backbone fragment was CIPed and purified. The new insert fragment contains the R6K origin of replication and a modified SV40 poly(A) sequence as part of the expression cassette. Unnecessary bacterial or viral backbone sequences around the SV40 poly(A) had been eliminated (see Table 2 below). The insert fragment has been ordered from GeneArt; it was cut out of the GeneArt cloning vector #1013238 using the enzymes PvuI and BspHI (the same as used for the backbone), purified and cloned into the backbone fragment. Minipreps were prepared and were confirmed by sequence analysis. The clone pGLEX41-MCS-R6K-ampiA#1 had the correct sequence.

**Table 2: Content of CpG in the different vectors**

| | **CpG content in expression vectors** | | |
|---|---|---|---|
| | **pGLEX41** | **Codon optimized Vectors: "A"** | **CpG reduced Vectors "B"** |
| Ampicillin resistance | 49 | 43 | 19 |
| Puromycin resistance | 93 | 36 | 1 |
| Geneticin resistance | 74 | 51 | 0 |
| Origin of replication | 45 | 9 | 9 |
| Sum: | 261 | 139 | 29 |

### Cloning of intermediate vector pGLEX41-MCS-R6K-ampiB

The vector pGLEX41- MCS-R6K-ampiA#1 was opened using the restriction enzyme BspHI and CIPed in order to release the ampicillin resistance. The new insert fragment contains the ampicillin resistance codon optimized for expression in *E. coli,* but all the CpG sequences that could be eliminated by alternative codon usage had been replaced (see Table 2 above). This fragment was ordered at GeneArt. In order to release the insert fragment, the GeneArt cloning vector #1016138 was digested using BspHI. After purification of both insert and backbone fragments by gel electrophoresis, they were ligated and transformed into PIR1 bacteria. The minipreps were directly sent for sequencing. pGLEX41-R6K-MCS-ampiB#1 has the correct sequence and was used for further cloning steps.

### Cloning of the reporter construct in pGLEX41-derived expression vectors

In order to clone the reporter construct REP in the expression vectors pGLEX41- MCS-R6K-ampiA and pGLEX41-MCS-R6K-ampiB, the vectors were cut using the restriction enzymes NheI and Clal. The expression vector pGLEX41-HM-MCS was opened using the restriction enzymes NheI and BstBI (at 65°C). All vector backbones were treated with CIP after digestion and the backbones purified by gel electrophoresis. The backbones were ligated with the NheI/BstBI (BstBI is compatible with ClaI) fragment coding for the reporter construct REP. The ligation products were transformed into PIR1 or TOP 10 competent bacteria and plated on ampicillin LB-agar plates. Colonies were amplified and plasmids were isolated by minipreps. Positive clones could be identified by restriction digest of minipreps and subsequent sequence confirmation by Fasteris SA.

### Addition of flanking GAPDH sequences in pGLEX41 derived expression vectors

All restriction digest of this paragraph were performed in a 80 µl final volume and incubated over night at 37°C.

5'GAPDH sequence (SEQ ID NO: 7) was excised from pCR-blunt-5'GAPDH using the restriction enzyme NruI and ligated in the expression vectors pGLEX41-R6K-ampiA-[REP] and pGLEX41-R6K-ampiB-[REP] which were linearized using NruI and treated with CIP in order to avoid re-circularization. After amplification of PIR1 colonies (obtained by transformation of ligation products) minipreps were analyzed by restriction digest. Clones pGLEX41-R6K-ampiA-5'GAPDH-[REP] #2 and pGLEX41-R6K-ampiB-5'GAPDH-[REP] #1 showed bands of the expected size in the restriction analysis, were subsequently confirmed by sequencing and used for further cloning steps. These new vectors were then opened with ScaI and treated with CIP. The 3'GAPDH fragment (SEQ ID NO: 8) was excised from pCR-Blunt-3'GAPDH using the same enzyme and ligated into the two backbones in order to generate pGLEX41-R6K-ampiA-GAPDH-[REP] and pGLEX41-R6K-ampiB-GAPDH-[REP] expression vectors.

The control digest of clones pGLEX41-R6K-ampiA-GAPDH-[REP] #2 and pGLEX41-R6K-ampiB-GAPDH-[REP] #8 showed bands of the expected size in the restriction analysis .The insertion of the 3'GAPDH fragment in the correct orientation was subsequently confirmed by sequencing (Fasteris).

### II.4. Cloning of resistance vectors

Starting point for the cloning of the resistance vectors was the vector pGLEX-MCS-R6K-ampiA#1. As for expression of resistance genes a weak promoter is sufficient, the mCMV promoter was replaced by the SV40 promoter. The genes coding for the resistance genes were ordered from GeneArt SA (Regensburg, Germany) and either optimized for expression in Chinese hamster (puromycin: puroA and neomycin: neoA) or reduced in CpG content by selective codon usage (puromycin: puroB and neomycin: neoB).

### Cloning of pGLEX-R6K-AmpiA-PuroA/PuroB:

In order to clone the puromycin resistance in the expression cassette, the vector pGLEX41-MCS-R6K-ampiA#1 was opened using the restriction enzymes NruI and XbaI followed by treatment with CIP. The insert fragment was ordered from GeneArt and was provided as insert in GeneArt cloning vector #1013239. It contains the SV40 promoter and the codon optimized gene for the puromycin resistance (for codon usage of CHO cells). The insert was cut out of the GeneArt cloning vector using the enzymes NruI and XbaI (the same as used for the backbone), purified and cloned into the backbone fragment. Minipreps were prepared and analyzed by restriction digest. The clone pGLEX-MCS-R6K-ampiA-puroA#1 showed the correct profile and could be confirmed by sequencing.

This vector was used for the cloning of the vector pGLEX-MCS-R6K-ampiA-puroB by exchange of the coding region for the puromycin resistance gene, while leaving the SV40 promoter. The new insert fragment contains a codon-optimized version of the puromycin gene, where all the CpG sequences that could be eliminated due to alternative codon usage had been replaced. The fragment has been ordered by GeneArt and was delivered in the cloning vector # 1016139. In order to release the insert fragment, the GeneArt vector was digested using the restriction enzymes XbaI and NotI. The insert fragment was purified by gel electrophoresis and cloned into the backbone of pGLEX-MCS-R6K-ampiA-puroA, after release of the puromycin open reading frame by restriction digest using XbaI and NotI, followed by CIP treatment. The resulting vector pGLEX-MCS-R6K-ampiA-puroB#1 was confirmed directly by sequence analysis.

### Cloning of the vectors pGLEX-R6K-ampiA-NeoA and pGLEX-R6K-ampiA-NeoB

In order to clone the neomycin resistance in the expression cassette, the vector pGLEX-R6K-puroA#1 was opened using the restriction enzymes XbaI and NotI, followed by treatment with CIP. The insert fragments were ordered from GeneArt and were provided as inserts in GeneArt cloning vectors #1013242 (neoA) and #1026894 (neoB). They contain the codon optimized gene for the neomycin resistance for codon usage of CHO cells and the CpG reduced version of the neomycin resistance, respectively. The inserts were cut out of the GeneArt cloning vectors using the enzymes XbaI and NotI (the same as used for the backbone), purified and cloned into the backbone fragment. Minipreps were prepared and the clones were confirmed by sequencing.

### Cloning of vectors pGLEX-R6K-ampiB-NeoB and pGLEX41-R6K-ampiB-puroB:

The vector pGLEX41-R6K-puroB#1 was opened using the restriction enzyme BspHI and subsequently CIPed. The insert fragment contains the ampicillin resistance gene that was codon optimized for expression in *E. coli,* while all CpG sequences that could be eliminated due to alternative codon usage had been replaced. This fragment has been ordered at GeneArt and arrived in the cloning vector #1016138. In order to release the insert fragment the GeneArt cloning vector was digested using BspHI. After purification of both insert and backbone fragment by gel electrophoresis, they were ligated and transformed into PIR1 bacteria. The minipreps were directly sent for sequencing and could be confirmed (pGLEX41-ampiB-R6K-puroB#1).

The cloning leading to vector pGLEX-R6K-neoB-ampiB was done by opening pGLEX-R6K-neoB-ampiA using the restriction enzymes BspHI in order to create the backbone fragment. Digestion of pGLEX-R6K-ampiB-hygroB using the same restriction enzyme combination yielded the insert fragment coding for ampiB. The ampiB insert was cloned into the pGLEX-R6K-neoB-ampiA backbone.

### II.5 Addition of sequences upstream and downstream of the human GAPDH gene into resistance vectors

The vector pCR-blunt-5'GAPDH was digested with NruI in order to obtain the 5'GAPDH insert (3164 bps). The vectors coding for resistance genes were digested with NruI, subsequently treated with CIP (Calf intestinal phosphatase, NEB, Ipswich, MA) in order to prepare the backbone fragments. The 4 different backbone fragments (pGLEX-R6K-neoA-ampiA, pGLEX-R6K-neoB-ampiB, pGLEX-R6K-puroA-ampiA and pGLEX-puroB-ampiB) were ligated with the 3164 bps 5'GAPDH insert and transformed into PIR1 competent bacteria. Restriction digest of minipreps using ApalI allowed the identification of clones pGLEX-R6K-neoB-ampiB-5'GAPDH#5, pGLEX-R6K-neoA-ampiA-5'GAPDH #6, pGLEX-R6K-puroA-ampiA-5'GAPDH #16 and pGLEX-puroB-ampiB-5'GAPDH #5.

These intermediate vectors were then cut with the restriction enzyme ScaI and treated with CIP in order to prepare the backbones for ligation. The vector carrying the second insert fragment, pCR-Blunt-3'GAPDH, was cut using ScaI in order to release the insert fragment (3224 bps) the GAPDH downstream flanking region. The four different backbone molecules were ligated with the purified 3224 bps insert fragment and transformed into PIR1 competent cells. Minipreps were analyzed by restriction digest. Clones showing restriction fragments of the expected size were pGLEX-R6K-neoB-ampiB-GAPDH #8, pGLEX-R6K-neoA-ampiA-GAPDH #1, pGLEX-R6K-puroA-ampiA-GAPDH #1 and pGLEX-puroB-ampiB-GAPDH #4. The clones were subsequently confirmed by sequencing analysis (Fasteris, Geneva, Switzerland).

### II.1.5. Midipreparations of plasmids cloned for transfection

In order to have sufficient quantities of plasmids, midipreps were prepared using the Macherey Nagel kit (NucleoBond Xtra Midi; Macherey Nagel, Oensingen, Switzerland). After confirmation by restriction digest and sequencing, the plasmids were linearized and used for transfection in CHO-S cells. Table 3 summarizes the concentrations of plasmid DNA batches obtained in midipreparations, linearized DNA preps that had been prepared for transfection, the enzymes used for linearization and the sequence files from Fasteris SA confirming the identity and the sequence information of the respective plasmid. All midipreps were confirmed by sequencing before being used for transfections.

**Table 3: Summary of plasmids cloned. Concentration of DNA midipreparation and linearized midipreparation (with the corresponding enzyme). The GSC number codes for the respective plasmid and allows to identify relevant sequencing files.**

| **Plasmids** | **Conc**. **of Midipreparation (µg/ml)** | **Enzyme for linearization** | **Conc. of linearized plasmids (µg/ml)** | **Glenmark plasmid code** |
|---|---|---|---|---|
| pGLEX41-R6K-AmpiA-[REP]-GAPDH | 1538 | EcoRV | 1019 | GSC 2774 |
| pGLEX41-R6K-AmpiB-[REP]-GAPDH | 1243 | EcoRV | 1233 | GSC 2775 |
| pGLEX-R6K-AmpiA-neoA-GAPDH | 890 | AseI | 766 | GSC 2776 |
| pGLEX-R6K-AmpiB-neoB-GAPDH | 594 | AseI | 979 | GSC 2777 |
| pGLEX-R6K- AmpiA-puroA-GAPDH | 917 | AseI | 859 | GSC 2778 |
| pGLEX-AmpiB-puroB-GAPDH | 869 | AseI | 1049 | GSC 2779 |
| pGLEX41-[REP] | 2119 | BspHI | 868 | GSC 2239 |
| pGLEX41-R6K-AmpiA-[REP] | 865 | BspHI | 779 | GSC 2240 |
| pGLEX41-R6K-AmpiB-[REP] | 1751 | BspHI | 806 | GSC 2249 |
| pGLEX-R6K-AmpiA-neoA | 890 | BspHI | 764 | GSC 2214 |
| pGLEX-R6K-AmpiB-neoB | 767 | BspHI | 654 | GSC 2244 |
| pGLEX-R6K-AmpiA-puroA | 708 | BspHI | 659 | GSC 2220 |
| pGLEX-R6K-AmpiB-puroB | 574 | BspHI | 746 | GSC 2213 |

### Example 2: Transfection of cells with expression vectors:

### 1. Materials and Methods

### CHO-S cells and HEK293 cells

Mammalian cells are the preferred host to express proteins because they are capable of correct folding, assembly and post-transcriptional modification of recombinant proteins. The CHO cell line was used because they are well characterized and do not serve as a host for most human pathogenic viruses, making them a relatively safe host for stable therapeutic protein production. Chinese Hamster Ovary cells (CHO-S, Invitrogen, Carlsbad, CA, USA) were cultured in suspension in PowerCHO-2 CD medium (Lonza, Verviers, Belgium), supplemented with 4 mM L-glutamine (Applichem, Germany) and incubated in a shaking incubator (200 rpm with a circular stroke of 2.5 cm) at 37°C, 5% CO₂ and 80% humidity. HEK293 cells are used because they are easy to transfect and allow rapid production of recombinant proteins up to lower gram amounts. The cells used are HEK293-EBNA cells (ATCC, Manassas, VA) and are routinely cultured in suspension in Ex-cell 293 medium (Sigma-Aldrich, St. Louis, MI).

Subcultures of CHO-S and HEK293 EBNA cells were routinely carried out every 3-4 days using a seeding density of 0.5x10⁶ viable cells/ml in fresh medium. The cells were cultivated using 10 ml of medium in 50 ml bioreactor tubes (Tubespin Bioreactor 50; TPP, Trasadingen, Switzerland) containing a permeable filter allowing gas exchange. The cell viability and concentration were determined with the Countess automated cell counter (Invitrogen, Carlsbad, CA, USA) using the trypan blue cell exclusion method. Cell concentration was confirmed by determination of the packed cell volume (PCV) method using PCV tubes (TPP, Trasadingen, Switzerland) for CHO-S cells.

### Packed cell volume (PCV)

The PCV method is based on the centrifugation of a specific volume of culture liquid in a mini-PCV tube (PCV Packed Cell Volume Tube; TPP, Trasadingen, Switzerland) for 1 min at 5000 rpm. During centrifugation, the cells are pelleted in the graduated capillary at the base of the tube. The percentage of packed cell volume is then determined by assessing the volume of the pellet in relationship to the amount of cell culture fluid centrifuged. For example, 1% PCV indicated that 10 µl of cell pellet was present in 1 ml of culture fluid.

For routine cell counting of cells, 200 µl of each sample was pipetted in a PCV tube and the volume of the corresponding pellet (in µl) was read with a ruler ("easy read" measuring device; TPP, Trasadingen, Switzerland). This volume was multiplied by 5 to have the value for 1 ml and then it was multiplied using a cell specific correlation factor to obtain an estimation of the concentration of viable cells (in millions of cells/ml).

### "Automatized" cell counting

Cell concentration and viability was determined with the Countess® Automated Cell Counter (Invitrogen, Carlsbad, CA, USA) in mixing the sample with the same amount of trypan blue. The solution is then pipetted into the Countess® chamber slide before being read by the instrument. This instrument allows an automatic read-out of the Neubauer chamber which, after calibration, determines cell viability and the concentration of dead and living cells.

### Flow Cytometry analysis

Flow Cytometry is a technique for the analysis of multiple parameters of individual cells. This technique allows the quantitative and qualitative analysis of cells that are phenotypically different from each other, for instance dead from viable cells (according to the size and the granularity of cells). It also allows the quantification of cells which express a protein of interest, such as GFP. Cells were collected from the culture by sterile pipetting 300 µl of samples and were analyzed with a Fluorescence-Associated Cell Sorting (FACS) Calibur flow cytometer (Becton, Dickinson and Company, Franklin Lakes, NJ, USA) equipped with an air-cooled argon laser emitting at 488 nm. The analyses were made with the CellQuest software. GFP emission was detected with the FL-1, using a 530/30-nm band pass filter.

In the first gate, cell debris as well as dead cells were excluded from the analysis in a SSC/FSC dotplot on linear scale. Then, the GFP fluorescence of living cells was displayed in a histogram on logarithmic scale. The median value of the fluorescence distribution was used to assess the GFP expression level of the analyzed cell populations.

### IgG quantification method: OCTET QK

The Octet QK system (FortéBio, Menlo Park, CA, USA) performs label-free quantitation of antibodies, proteins, peptides, DNA and other biomolecules and provides kinetic characterization of biomolecular binding interactions. A correlation between the binding rate (nm) and the accumulated IgG1 concentration (µg/ml) of the sample allows quantification of the IgG titer with a calibration curve.

Cell samples were centrifuged 5 min at 300 g. The supernatant was then diluted (1/5 for IgG1 antibody) with the Octet Buffer in a 96 well plate before being analyzed with the Octet using Protein A biosensors (Protein A DIP and READ™ Biosensor, Forté Bio, USA) to obtain the antibody concentration per well.

### Transient transfection using JetPEI

Transient and stable transfection of CHO-S and HEK293 EBNA cells was performed using polyethyleneimine (PEI; JetPEI, Polyplus-transfection, Illkirch, France). PEI is a cationic polymer which can complex with negatively charged molecules such as DNA. The positive charged DNA-PEI complex binds to the negatively charged cell surface and is internalized by endocytosis. It reaches the lysosome compartment from where it is released by lysis to the nucleus. The high transfection efficiency with DNA-PEI complexes is due to the ability of PEI to protect DNA from lysosomal degradation. The cells were transfected according to the manual provided by the manufacturer.

All plasmids were linearized before stable transfection (100 µg of DNA re-suspended in 100 µl Tris-EDTA, pH 7.5). For transient transfection circular plasmids were directly used from midipreparation DNA. In this study, transient transfections were kept in 50 ml bioreactor tubes and no antibiotics were added.

Stable CHO-S clones expressing IgG1 and GFP were obtained by co-transfecting one expression vector and two resistance vectors (coding for puromycin or neomycin resistance, respectively).

### Selection of stable pools and minipools

Transfection efficiency was determined 24h after transfection by Flow Cytometry (BD FACS Calibur cytometer, #1293) by analysing the intracellular GFP expression. If the percentage of GPF positive cells was higher than 20 %, the transfected cells were diluted with selective medium and distributed into 96 well plates (for limiting dilution to generate isolated stable minipools) or in T-Flasks (to generate stable pools). The selective medium used was PowerCHO-2, 4 mM glutamine, supplemented with different concentrations of geneticin and puromycin.

Seven days after transfection, the selection stringency was renewed by adding selection medium to the cells. As soon as colonies in 96 well plates were confluent, the plates were read using a fluorescence reader.

The pools in T-Flasks were expanded to tubespin scale using antibiotic-free PowerCHO-2, 4 mM L-glutamine. Their viability and concentration were evaluated with the Countess automated cell counter (Invitrogen, Carlsbad, CA, USA). As soon as the cell density allowed it, a seed train was started for every pool by seeding cells at a density of 0.5x10⁶ cells/ml in 10 ml medium in 50 ml bioreactor tubes (incubated in a shaker (200 rpm) at 5% CO₂, 37°C and 80% humidity). Each seed train was passaged twice a week by seeding the cells at 0.5x10⁶ cells/ml in growth medium (cell concentration was determined by PCV analysis). The seed train was used for the inoculum of all productions runs (batches).

For the next 4-5 weeks productions runs were seeded once a week in duplicates. The pool stability was evaluated by FACS and IgG expression as described above for clonal populations.

### Production runs (batch fermentation)

The batch runs of cell pools were seeded at a concentration of 0.5x10⁶ cells/ml using the seed train for inoculation and cells were then cultured for 7 days in Feed media. On day 4 and 8, 200 µl of cells were centrifuged for 5 min at 300 g and the supernatant was analyzed for accumulated IgG using the Octet. In addition, the GFP expression of each batch was analyzed by FACS.

### 2. Results

### 2.1 Expression in transient in CHO cells:

The vectors compared in this study differ mainly in their backbone. The entire expression cassette (Promoter, first intron, expression construct, poly (A)) is exactly the same for all vectors. The vectors are derived from the vector pGLEX41 as described in Example 1. In one vector, the ampicillin resistance gene was codon optimized for expression in *E. coli* and the bacterial backbone was reduced to a minimum: pGLEX41-R6K-AmpiA-[REP] (in short A). In a second vector, the ampicillin resistance gene was codon optimized for expression in *E*. *coli,* but all CpG sequences were avoided, by using alternate codons (when possible): This vector is called pGLEX41-R6K-AmpiB-[REP] (in short B). The third modification included the use of the GAPDH flanking sequences that were cloned upstream and downstream of the expression cassette of the vectors A and B giving the vectors pGLEX41-R6K-AmpiA-[REP]-GAPDH (in short GAPDH A) and pGLEX41-R6K-AmpiB-[REP]-GAPDH (in short GAPDH_B).

Transient transfections of CHO-S cells (Invitrogen) were done in order to compare the expression level of the reporter proteins expressed in the context of the different plasmid backbones. The transfections (in duplicate) were performed in 50 ml bioreactor tubes (TPP, Trasadingen, Switzerland) using 10 ml of final medium volume and analyzed on day 5 after transfection by Octet (Fig. 2).

All vectors (A and B) with corrected backbone show a slightly higher expression level than the control vectors pGLEX41. There is only a minor difference between the vectors A and B. This is expected, because the only difference in the backbone is the ampicillin resistance which should not have an impact on transient expression.

The most striking observation is the positive effect of the GAPDH sequences on expression. A 2-fold higher expression level is obtained with the plasmid harbouring the GAPDH flanking sequences compared to the ones without the GAPDH sequences. This is true for both A and B constructs. Compared to the pGLEX41 vector, a 3-fold higher expression can be observed. This is even more surprising if the size of the plasmids is taken into account. The vector A (7048 bps) is almost half the size compared to the vector GAPDH-A (13436 bps). Therefore, assumed that the amount of delivered DNA during the process of transient transfection is the same for all plasmids, only half the molar amount of GAPDH-A is delivered to the nucleus.

### 2.2 Expression in transient in HEK293 cells

Transient transfections of HEK293 EBNA cells were done in order to compare the expression level of the reporter proteins expressed in the context of the different plasmid backbones. The transfections (in duplicate) were performed in 50 ml bioreactor tubes (TPP, Trasadingen, Switzerland) using 10 ml of final medium volume and were analyzed on day 10 after transfection by Octet (Fig. 3).

The results shown in figure 3 show a significant increase in expression that can be obtained using the GAPDH flanking regions in HEK293 EBNA cells. The GAPDH-B vector is showing a threefold increase in expression, whereas the GAPDH-A vector shows an even higher increase in expression of 5-fold. These vectors do not contain the oriP element and might therefore have a potential for even higher titers.

### 2.3 Expression in stable CHO cell lines

### Establishment of stable transfected cells

Stable populations were generated by co-transfecting an expression vector and vectors coding for resistance genes, followed by selection pressure mediated by antibiotics. The selection pressure was removed 14 days after transfection. These steps allowed the generation of stable minipools and stable pools which were cultured in regular intervals in production runs in order to compare the expression levels of the reporter proteins (IgG1 antibody and GFP) of the different constructs and the stability of expression.

### Reporter protein expression study on production runs performed with cell pools

Pools were generated by stable transfection. During the selection procedure (the first 14 days after transfection) the pools were analyzed by FACS. An increase of the GFP positive cell fraction together with the viability of the culture could be observed over the time. The selection pressure mediated by the antibiotics was removed from the pools after 14 days. Using this approach no cell pools transfected with the "B" plasmids could be obtained. The expression level of the generated pools was assayed as soon as the cells could be cultured in 50 ml bioreactor tubes. Batches were done in duplicates. The cells were analyzed by FACS for GFP expression and the accumulation of IgG in the supernatant was assayed by Octet after 8 days of expression.

A proportional relationship could be observed between the IgG titers and the GFP expression of the pools. Therefore, only the IgG data are shown in figure 4. All pools transfected with vectors containing GAPDH sequence show higher expression compared to the vector pGLEX41 or with the same vector without GAPDH sequence (factor of 2.8 between A and A-GAPDH. No conclusion could be drawn between B and B-GAPDH as no B pools survived). Transfections performed with A-GAPDH and B-GADPH induced a higher expression of IgG (2.7 and 3.5 folds more respectively) than pGLEX41 transfection (for batch-2). Therefore in pools, the GAPDH flanking sequences seem to be favourable for the production of proteins. Finally, transfections performed with B-GADPH vectors induced a higher expression of IgG than the transfection performed with A-GAPDH (factor of 1.25). Therefore, the CpG reduction in resistance genes seems to be favourable for the stable production of proteins, too.

### Expression level study on clonal populations

Cells were transfected and distributed in 96 well plates in selective medium in order to obtain clonal or oligoclonal populations. After 7 days the selection pressure was refreshed by addition of selective medium to the cells. The expression of GFP was assessed 14 days after transfection by using an ELISA-plate reader. The results are shown in figure 5.

Confirming the results obtained in cellular pools, cells transfected with vectors containing GAPDH flanking sequences expressed significantly more GFP than the same backbone without GAPDH up-and downstream sequences (factors from 1.7 to 2 fold) or the other vectors used as control (pGLEX41: 2.5 fold) (Fig. 5). In addition, populations with vectors containing resistance sequences which had been CpG reduced (B) induced a higher expression than the corresponding vectors which had only been codon optimized (A) (1.5 fold between A and B; 1.2 fold between B and B-GAPDH).

From the expression study several conclusions could be drawn. First, the GAPDH up- and downstream sequence allows higher expression than the standard vector that was used as a benchmark (pGLEX41). Also a lower expression level is obtained when cells are transfected with the same vector backbone without the GAPDH sequences confirming that the beneficial effect on the expression is related to the inserted GAPDH flanking sequences. In addition, the reduction of CpG number in the expression and selection plasmids seems to be slightly favourable for expression, too.

### Example 3: Transient expression level of CHO-S GMP cells transfected with new designed vectors

It has been described in the literature that the 5' region of the GAPDH promoter harbours a potential insulin as well as a phorbol ester response element (Alexander-Bridges et al., (1992) Advan Enzyme Regul, 32: 149-159). The phorbol ester response element (-1040 -1010 bps) is situated upstream of what is usually referred to as the GAPDH promoter (-488 - +20). In a deletion study performed in stable H35 Hepatoma cell lines, the authors were not able to demonstrate a significant effect of the deletion of basepairs -1200 to -488 (relative to the transcription starting point). Therefore the phorbol ester response element might not be functionally linked to the expression driven from the GAPDH promoter. Nevertheless a transient transfection experiment was performed in order to evaluate the contribution of insulin and PMA (phorbol-12-myristate-13-acetate, the most common phorbol ester) in the increase in transient and stable expression that was observed using the plasmids containing the GAPDH flanking elements.

In order to obtain insulin free growth medium, PowerCHO2 was prepared from powder medium and no insulin was added. PMA was purchased from Sigma (St. Louis, MO), and was dosed at a final concentration of 1.6 µM (corresponding to the concentration used by Alexander-Bridges on H35 Hepatoma cell lines) in PowerCHO2 (+/- Insulin).

Transfections were performed in 50 ml bioreactor tubes (Tubespins, TPP, Trasadingen, Switzerland) as described previously. In order to avoid the presence of insulin provided by OptiMEM (Life technologies, Carlsbad, CA), the transfection medium was changed to RPMI1640 (PAA, Pasching, Austria) supplemented with 4 mM Gln and 25 mM HEPES. After transfection, the cells were distributed in 12 well plates and 1 ml of the four different media was added (PowerCHO2, 4mM Gln, +/-insulin; PowerCHO2, 4mM Gln, 1.6 µM PMA, +/- insulin). Again, the reporter construct expressing IgG1 and GFP using two IRES was used (described in example 2). This vector allowed verification of the transfection efficiency. The percentage and the viability of transfected cells were found similar in all four different media preparations.

As shown in figure 6, no significant effect of insulin depletion and/or PMA addition could be observed during this experiment. Similar titers were obtained in all media used for expression. This suggests that the potential phorbol ester and the insulin response elements present in the upstream flanking sequence of the GAPDH gene do not affect transient transgene expression.

### Example 4: Fragmentation analysis of DNA flanking the GAPDH expression cassette upstream of the promoter and downstream of the poly A site in order to study the effect on reporter gene expression

The human GAPDH locus is located on chromosome 12 of the human genome. GAPDH is described to be constitutively active in all cells of mammalian origin, as the enzyme is a key player in the metabolism of glucose. Upstream of the promoter, the GAPDH gene is flanked by NCAPD2, a gene that stretches over more than 30000 bps. Downstream of the polyadenylation site, the GAPDH gene is flanked by IFFO1 (see figure 7 for details).

Not only GAPDH and the promoter, but also the flanking regions are well conserved between different species (see Table 4).

**Table 4: Stretches of high homologies between human, rat and mouse GAPDH flanking regions. Analysis was done using clone manager 9 (ScieED, Cary, NC, USA). The numbering is relative to the first base of the upstream or the downstream flanking element, respectively (Sequence ID NO: 7 and Sequence ID NO: 8, respectively). Sequences used for alignment were for mouse bases 532-3731 (upstream) and 8164-11364 (downstream) of Sequence ID No 18 and for rat bases 719-3918 (upstream) and 8495-11058 (downstream) of Sequence ID No 19.**

| **Upstream region** | | | | **Downstream** | | | |
|---|---|---|---|---|---|---|---|
| **Sequences of homology [rat]** | | **Sequences of homology mouse]** | | **Sequences of homology [rat]** | | **Sequences of homology [mouse]** | |
| **>80 %** | **>90 %** | **>80** % | **>90** % | **>80 %** | **>90** % | **>80 %** | **>90 %** |
| 161-249 | 279-331 | 15-69 | 278-329 | 1608-1764 | 1706-1764 | 1614-1671 | 1904-2061 |
| 256-338 | 554-623 | 159-249 | 546-626 | 1894-2067 | 1912-2061 | 1888-2072 | 2927-3071 |
| 515-659 | | 273-342 | | | | 2918-3082 | |
| 2296-2349 | | 515-647 | | | | | |
| 2381-2513 | | 1143-1223 | | | | | |
| 2736-2818 | | 1957-2009 | | | | | |
| | | 2029-2080 | | | | | |
| | | 2375-2485 | | | | | |
| | | 2730-2821 | | | | | |

A comparison of the DNA homology between rodent and human shows a minimum of DNA conservation of 38%. The presence of a conserved stretch of DNA outside of a promoter region or a region coding for a gene indicates that there might be a selection pressure on the cell to maintain the DNA sequence or to allow only certain/minor changes. In our specific case, the GAPDH flanking regions might be important for the cells because they maintain a high expression level of the GAPDH genes. Changes in the DNA sequence leading to decrease of expression would be selected against.

In order to evaluate the contribution of the upstream and the downstream GAPDH element to the observed increase in expression, constructs were made containing only the upstream GAPDH flanking region (SEQ ID NO: 7), fragments of the upstream GAPDH flanking region or the downstream GAPDH flanking region (SEQ ID NO: 8). The reporter IgG1 type antibody was expressed by an IRES construct (Light chain-IRES-heavy chain), therefore avoiding co-transfection of multiple plasmids. Details on the fragmentation of the GAPDH upstream fragment are shown in figure 8. The following fragments of the upstream GAPDH flanking region were used: Fragment 1 (SEQ ID NO: 9), fragment 2 (SEQ ID NO: 10), fragment 3 (SEQ ID NO: 11), fragment 4 (SEQ ID NO: 12), fragment 8 (SEQ ID NO: 13), fragment 9 (SEQ ID NO: 14), fragment 11 (SEQ ID NO: 15), fragment 17 (SEQ ID NO: 16).

The upstream GAPDH flanking region (SEQ ID NO: 7) used does contain 2 times 3 (in total 6) nucleotides of the NruI restriction site of which three are linked to the genomic DNA at its 5' and three are linked to the genomic DNA its 3' end. The downstream GAPDH flanking region (SEQ ID NO: 8) used does contain two times 3 (in total 6) nucleotides of the ScaI restriction site of which three are linked to the genomic DNA at its 5' and three are linked to the genomic DNA its 3' end. The upstream GAPDH flanking region and the downstream GAPDH flanking region without the nucleotides of the respective restriction site are shown in SEQ ID NO: 20 (upstream GAPDH flanking region without restriction sites) and SEQ ID NO: 21 (downstream GAPDH flanking region without restriction sites). The fragments of the upstream GAPDH flanking region used does each contain 3 nucleotides of the respective restriction site at its 5' and/or its 3' end linked to the genomic DNA (Fragment 1 contains 3 nucleotides of the NruI restriction site at its 5'end; Fragment 2 contains 3 nucleotides of the NruI restriction site at its 3'end; Fragment 3 contains 3 nucleotides of the NruI restriction site at its 5'end: Fragment 4 contains 3 nucleotides of the NruI restriction site at its 3'end; Fragment 8 contains 3 nucleotides of the NruI restriction site at its 3'end; Fragment 9 contains 3 nucleotides of the NruI restriction site at its 5' end and 3 nucleotides of the NruI restriction site at its 3'end; Fragment 11 contains 3 nucleotides of the NruI restriction site at its 3'end). Fragment 17 does not contain nucleotides of a restriction site. The fragments of the upstream GAPDH flanking region without the nucleotides of the respective restriction site are shown in SEQ ID NO: 22 (fragment 1 without restriction site), SEQ ID NO: 23 (fragment 2 without restriction site) SEQ ID NO: 24 (fragment 3 without restriction site), SEQ ID NO: 25 (fragment 4 without restriction site), SEQ ID NO: 26 (fragment 8 without restriction site), SEQ ID NO: 27 (fragment 9 without restriction sites), SEQ ID NO: 28 (fragment 11 without restriction site).

The effect of the upstream and the downstream GAPDH elements on expression was assessed on day 10 after transfection using the Octet (Fortebio, Menlo, CA, USA) in order to quantify the amount of secreted IgG1 in the supernatant (see figure 9). pGLEX41, the original vector is giving lower expression results (80%) compared to the improved new vector design used in the pGLEX41-ampiA backbones. Compared to the original pGLEX41 backbone the new design includes codon optimization of the ampiA gene necessary for ampicillin resistance in *E. coli,* a different origin of replication (R6K instead of pUC origin of replication) and elimination of unnecessary linker (or spacer) sequences of bacterial origin. Both vectors have approximately the same size.

Surprisingly, pGLEX41-ampiA including the upstream (SEQ ID NO: 7) and downstream element (SEQ ID NO: 8), (named pGLEX41-up/down in figure 9 showing the expression results) is giving higher expression (factor 1.5) compared to the same vector without the upstream and downstream sequences. If one considers the difference in size (up/down fragments increase the size of the plasmid by approximately 6000 bps) and therefore the differences in delivered plasmid copies during transfection, the effect might even more important on a per plasmid basis.

The vector containing only the upstream fragment (up) is showing an expression level similar to the original expression construct pGLEX41-ampiA. The vector containing only the downstream fragment (down) is showing a significant increase (factor 1.2) in expression compared to the original expression construct pGLEX41-ampiA. A further increase in expression can be observed if both, the up- and the downstream fragment are present. This is confirmed by the fragmentation of the upstream fragments. Fragment 9 and the promoter proximal fragment 8 do not show any difference in expression compared to pGLEX41-ampiA. Fragment 1, 11 and 17 show an increase in expression. The highest increase was observed for fragment 4. It should be highlighted that the promoter proximal fragment 8 is not showing any effect. Therefore the increase in expression cannot be explained by previously published sequences (Alexander-Bridges et al., (1992) Advan Enzyme Regul, 32: 149-159), Graven et al., (1999) Biochimica et Biophysics Acta 147: 203-218).

Interestingly, fragments 2 and 3 lead to a significant decrease in expression. This is unexpected, especially in view of the fact that these fragments cloned in the opposite direction (antisense (AS) in figure 9) do not cause this effect. For the fragments 1, 8, 9, 11 and 17 no difference in expression was observed for fragments that were integrated in sense or antisense orientation (data not shown). Fragment 11, although a part of fragment 2, does not show this effect. Therefore the sequence element that seems to be detrimental to expression should be at least partially on the BstBI-BstBI fragment that was deleted in fragment 2 in order to obtain fragment 11.

In addition, the hypothesis that a negative element is located (at least partially) on the BstBI-BstBI fragment is supported by the increase in expression observed between fragment 3 (which includes the BstBI-BstBI fragment) and fragment 1.

While it seems easy to localize the fragment having a negative effect (BstBI-BstBI), from this study it is less obvious how this negative effect observed for fragment 2 and 3 is compensated by sequence elements present in the complete upstream fragment. It could be that this negative effect is balanced out by the small positive effect that was observed by fragment 1 and fragment 4 (but the increase in expression for fragment 1 is less than for fragment 4). Nevertheless the positive effect for fragment 4 (factor 1.25) observed seems less important compared to the negative effect (factor 0.4). Furthermore fragment 9, which is the entire upstream region without the BstBI-BstBI fragment does not show increased expression compared to the entire GAPDH upstream flanking region (nevertheless, fragment 9 includes the EcoRV-BstBI fragment which is part of fragment 2 and 3 and might have a negative effect on expression).

It can only be speculated about the mechanism behind the observed effects. The orientation dependency of the negative effect on expression observed with fragments 2 and 3 excludes the expression of non-identified open reading frames (for example expression of an ncRNA), because there are no surrounding promoters that could trigger the expression of only one orientation. The fact that the expression is reduced below the basal level shows not only the absence of a positive effect (for example an enhancer activity), but rather the presence of an orientation dependent negative effect.

In summary, a surprising increase of expression in transient in CHO cells is observed if both flanking regions, the upstream and the downstream region, are present in the expression plasmid. Although fragment 4 seems to have a significant positive effect on expression, no single fragment could be identified that is responsible for the entire increase of expression that was observed. The increase of expression of the expression vector pGLEX41-ampiA (up/down) seems to be the summary effect of both, up- and downstream flanking region.

### Example 5: Cloning of the non-translated genomic DNA sequence upstream of the Chinese hamster GAPDH gene and the Chinese hamster promoter

### 1.1 Cloning of the non-translated genomic DNA sequence upstream of the Chinese hamster GAPDH gene into an expression vector

The non-translated genomic DNA sequence upstream of the Chinese hamster GAPDH gene was amplified from genomic DNA of CHO-S (Life Technologies) cells by PCR. Genomic DNA was extracted as described in Example 1. Constructs were prepared using the mouse CMV promoter or the Chinese hamster GAPDH promoter for the expression of the reporter gene construct [REP] described in Example 1.

For cloning of the genomic DNA sequence upstream of the Chinese hamster GAPDH gene in combination with the mouse CMV promoter, primers GlnPr1896 and GlnPr1897 were used for amplification of the 3 kbs fragment (bps 672 to 3671 of SEQ ID No 29) using the PCR protocol described in Example 1 and leading to the amplicon with the SEQ ID No 30. The amplicon contains the genomic DNA sequence upstream of the Chinese hamster GAPDH gene and 5' and 3' restriction sites that were introduced by the primers.

For cloning of the genomic DNA sequence upstream of the Chinese hamster GAPDH gene in combination with the Chinese hamster GAPDH promoter, primers GlnPr1902 and GlnPr1905 were used in order to amplify the 3508 bps fragment containing the genomic DNA sequence including the genomic DNA sequence upstream of the Chinese hamster GAPDH gene and the GAPDH promoter (bps 672 to 4179 of SEQ ID No 29) leading to the amplicon with the SEQ ID No 31. In a second PCR, GlnPr1901 and GlnPr1902 were used for amplification of the 508 bps fragment containing only the promoter region (bps 3672 to 4179 of SEQ ID No 29), leading to the SEQ ID No 32. The intron used in the vector "A" (described in Example 1) was amplified using primers GlnPr1903 and GlnPr1904.

A first fusion PCR was performed with primers GlnPr1904 and GlnPr1901 using the amplicon with SEQ ID NO: 32 and the amplicon with the intron sequence as templates. The amplicon contains the Chinese hamster GAPDH promoter, an intron and 5' and 3' restriction sites that were introduced by the primers. All primers are shown in Table 5.

A second fusion PCR was performed with primers GlnPr1905 and GlnPr1904 using the amplicon with SEQ ID No. 31 and the amplicon with the intron sequence as templates. The amplicon contains the genomic DNA sequence upstream of the Chinese hamster GAPDH gene, the Chinese hamster GAPDH promoter, an intron and 5' and 3' restriction sites that were introduced by the primers.

After purification on a 1% agarose gel, the bands of interest were cut out and purified using the kit "NucleoSpin Gel and PCR Clean-up" (Macherey Nagel, Oensingen, Switzerland). The purified fragments were cloned into the plasmid pCR Blunt using the Zero Blunt PCR cloning Kit (Invitrogen, Carlsbad, CA, USA). Ligation products were transformed into competent *E.coli* TOP10 (One Shot® TOP 10 Competent *E. coli;* Invitrogen, Carlsbad, CA, USA) and analyzed by restriction analysis of minipreps. This led to the plasmids pCR_blunt[CHO-upstreamGAPDH], containing the genomic DNA sequence upstream of the Chinese hamster GAPDH gene, pCR_Blunt[CHO-upstreamGAPDH_GAPDHpromoter] containing the genomic DNA sequence upstream of the Chinese hamster GAPDH gene and the GAPDH promoter and intron from vector "A" and pCR_Blunt[CHO-GAPDHpromoter] containing the GAPDH promoter and the intron from vector "A".

For evaluation of the amplicons on their effect on expression of a secreted gene, the vector "A" (described in Example 1) was used. As described previously, the expression cassette used in this vector contains a polycistronic gene coding for a secreted IgG1 and GFP (see Example 1). Transfected cells will therefore secrete the IgG1 monoclonal antibody and accumulate intracellular GFP in a dependent manner.

In order to release the 3 kb insert fragment containing the genomic DNA sequence upstream of the Chinese hamster GAPDH gene, the plasmid pCR_Blunt[CHO-upstreamGAPDH] was digested using the restriction enzyme Nael. This insert was cloned in the backbone of "A", digested using the restriction enzyme NruI and CIPed (CIP; NEB, Ipswich, MA, USA). Backbone and insert were ligated together using T4 DNA ligase (T4 DNA ligase, NEB, Ipswich, MA, USA) and subsequently transformed into competent *E.coli* PIR1. Clones were picked for miniprep preparation and subsequent restriction analysis. The resulting plasmid was called "A_GAPDH_UP", confirmed by sequencing analysis and produced in midiprep scale using the NucleoBond Xtra Midi kit (Macherey Nagel, Oensingen, Switzerland).

For the cloning of expression constructs using the Chinese hamster GAPDH promoter, the insert fragments were released from plasmids pCR_Blunt[CHO-upstreamGAPDH_GAPDH promoter] and pCR_Blunt[CHO-GAPDHpromoter] by digestion using the restriction enzymes NheI and NruI. The resulting fragments were cloned in the backbone of vector "A", opened using the same enzymes and CIPed. After ligation with T4 DNA ligase and transformation into competent *E.coli* PIR1, clones were picked for miniprep restriction analysis. The resulting plasmids were called "A_GAPDH_UP_Prom" (plasmid with non-translated genomic DNA sequence upstream of the Chinese hamster GAPDH and the promoter) and "A_PR" (plasmid with only the promoter) confirmed by sequencing analysis and produced in midiprep scale using the kit NucleoBond Xtra Midi (Macherey Nagel, Oensingen, Switzerland).

### 2. Assessment of the effect of the non-translated genomic DNA sequence upstream of the Chinese hamster GAPDH gene on the expression of the reporter gene construct

CHO-S cells were transfected in tubespins bioreactors using 10 ml of medium volume (as described in Example 2). The transfected cells were incubated in a shaking incubator with 200 rpm agitation at 37°C, 5 % CO2 and 80 % humidity. The supernatants of the cells were analyzed for IgG1 expression using the Octet QK system with Protein A biosensors, (FortéBio, Menlo Park, CA, USA). The results are shown in Figure 10.

The expression level of the plasmid containing the GAPDH promoter ("A PR") compared to the mouse CMV promoter (A) is reduced by 50 %, indicating that the Chinese hamster GAPDH promoter is not as strong as the viral promoter. The plasmid containing the non-translated genomic DNA sequence upstream of the Chinese hamster GAPDH gene in combination with the Chinese hamster GAPDH promoter ("A GAPDH UPProm") shows a two fold increase in expression compared to the construct having only the GAPDH promoter ("A PR"). The plasmid containing the non-translated genomic DNA sequence upstream of the Chinese hamster GAPDH gene and the mouse CMV promoter "(A GAPDH UP") shows the highest expression and an increase of more than 40% over the plasmid containing only the mouse CMV promoter ("A"). This confirms that the non-translated genomic DNA sequence upstream of the Chinese hamster GAPDH gene has an enhancer effect on the expression of the reporter protein.

**Table 5: Primers used for cloning in Example 5**

| **Primer** | **SEQ ID No** | **Sequence** | **Orientation** | **Restrn. site** |
|---|---|---|---|---|
| GlnPr 1896 | SEQ ID No 33 | TACGGCCGGCTTCACTGTACAGTGGCACAT | forward | NaeI |
| GlnPr 1897 | SEQ ID No 34 | TCAGGCCGGCCGTGGTTCTTCGGTAGTGAC | reverse | NaeI |
| GlnPr 1901 | SEQ ID No 35 | TACTCGCGAAGAAGATCCTCAACTTTTCCACAGCC | forward | NruI |
| GlnPr 1902 | SEQ ID No 36 | GTTCACTAAACGAGCTCTGCTATTTATAGGAACTGGGGTG | reverse | / |
| GlnPr 1903 | SEQ ID No 37 | CACCCCAGTTCCTATAAATAGCAGAGCTCGTTTAGTGAAC | forward | / |
| GlnPr 1904 | SEQ ID No 38 | CGCTAGCACCGGTCGATCGA | reverse | NheI |
| GlnPr 1905 | SEQ ID No 39 | TACTCGCGATTCACTGTACAGTGGCACATAC | forward | NruI |

## Claims

1. An expression cassette which comprises a promoter, a polynucleotide sequence encoding a polypeptide, and expression enhancing element wherein expression enhancing element comprises a non-translated genomic DNA sequence downstream of a mammalian Glyceraldehyde 3-phosphate dehydrogenase (GAPDH) promoter, wherein the polypeptide encoded by the polynucleotide sequence is not GAPDH, and wherein the genomic DNA sequence downstream of the mammalian GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the mammalian GAPDH promoter is from around 200 to around 2800 nucleotides and wherein the expression cassette further comprises a non-translated genomic DNA sequence upstream of a mammalian GAPDH promoter, wherein the non-translated genomic DNA sequence upstream of the mammalian GAPDH promoter starts within a region spanning from around the 5' end of the mammalian GAPDH promoter to nucleotide position around -3500, wherein the nucleotide position is relative to the transcription start of the GAPDH mRNA, and wherein the length of the non-translated genomic DNA sequence upstream of the mammalian GAPDH promoter is from around 200 to around 2800 nucleotides.

2. An expression cassette which comprises a promoter, a polynucleotide sequence encoding a polypeptide, and a non-translated genomic DNA sequence upstream of a mammalian GAPDH promoter, wherein the polypeptide encoded by the polynucleotide sequence is not GAPDH, and wherein the non-translated genomic DNA sequence upstream of the mammalian GAPDH promoter starts within a region spanning from around the 5' end of the mammalian GAPDH promoter to nucleotide position around -3500, wherein the nucleotide position is relative to the transcription start of the GAPDH mRNA, wherein the length of the non-translated genomic DNA sequence upstream of the mammalian GAPDH promoter is from 200 to around 2800 nucleotides, with the proviso that the expression cassette does not comprise a mammalian GAPDH promoter or fragments thereof.

3. The expression cassette of claim 2, wherein the expression cassette further comprises a non-translated genomic DNA sequence downstream of a mammalian GAPDH promoter, wherein non-translated genomic DNA sequence downstream of the mammalian GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the mammalian GAPDH promoter is from around 200 to around 2800 nucleotides.

4. The expression cassette of any one of claims 1 to 3, wherein the non-translated genomic DNA sequence downstream and/or upstream of the mammalian GAPDH promoter is not operably linked to the polynucleotide sequence encoding the polypeptide.

5. The expression cassette of claim 1 or 3, wherein the non-translated genomic DNA sequence downstream of the mammalian GAPDH promoter starts downstream of the mammalian GAPDH polyadenylation site and wherein the length of the non-translated genomic DNA sequence downstream of the mammalian GAPDH promoter is at least around 100 nucleotides and extends at its maximum to the second last intron of the IFF01 gene.

6. The expression cassette of claim 1 or 2, wherein the length of the non-translated genomic DNA sequence upstream of the mammalian GAPDH promoter is at least around 100 nucleotides and extends at its maximum to the third last intron of the NCAPD2 gene.

7. The expression cassette of any one of claims 1 to 3, wherein the non-translated genomic DNA sequence downstream and/or upstream of the mammalian GAPDH promoter is of rodent or human origin.

8. The expression cassette of claim 1 or 3, wherein the non-translated genomic DNA sequence downstream of the mammalian GAPDH promoter comprises the nucleotide sequence selected from the group consisting of: SEQ ID NOs: 8 and 21; a nucleotide sequence at least 80% identical to SEQ ID NOs: 8 or 21; fragments thereof; or a complementary nucleotide sequence thereof.

9. The expression cassette of claim 1 or 2, wherein the non-translated genomic DNA sequence upstream of the mammalian GAPDH promoter comprises a nucleotide sequence selected from the group consisting of SEQ ID NO: 7, 9, 10, 11, 12, 13, 14, 15, 16, 20, 22, 23, 24, 25, 26, 27 and 28 comprising five or less nucleic acid modifications; a nucleotide sequence at least 80% identical to the nucleotide sequence selected from the group consisting of SEQ ID NO: 7, 9, 10, 11, 12, 13, 14, 15, 16, 20, 22, 23, 24, 25, 26, 27 and 28 or fragments thereof; fragments thereof; or a complementary nucleotide sequence thereof.

10. The expression cassette of any one of claims 1 to 3, wherein the promoter is selected from the group consisting of SV40 promoter, MPSV promoter, mouse CMV, human tk, human CMV, rat CMV, human EF1alpha, Chinese hamster EF1alpha, human GAPDH, hybrid promoters including MYC, HYK and CX promoter.

11. The expression cassette of any one of claims 1 to 3, wherein the polypeptide is selected from the group consisting of antibodies, antibody fragments or antibody derivates.

12. An expression vector, which comprises in order:
a) a non-translated genomic DNA sequence upstream of a mammalian GAPDH promoter, wherein the non-translated genomic DNA sequence upstream of the mammalian GAPDH promoter starts within a region spanning from around the 5' end of the mammalian GAPDH promoter to nucleotide position around -3500, wherein the nucleotide position is relative to the transcription start of the GAPDH mRNA, and wherein the length of the non-translated genomic DNA sequence upstream of the mammalian GAPDH promoter is from around 200 to around 2800 nucleotides
b) a promoter
c) a polynucleotide sequence encoding a polypeptide
d) a polyadenylation site
e) an enhancer
f) a non-translated genomic DNA sequence downstream of a mammalian GAPDH promoter, wherein the polypeptide encoded by the polynucleotide sequence is not GAPDH, wherein the genomic DNA sequence downstream of the mammalian GAPDH promoter starts within a region spanning from nucleotide position around +1 to nucleotide position around +7000, wherein the nucleotide position is relative to the transcription start of the GAPDH mRNA, and wherein the length of the non-translated genomic DNA sequence downstream of the mammalian GAPDH promoter is from around 200 to around 2800 nucleotides.

13. A host cell comprising an expression cassette of any one of claims 1 to 11 or an expression vector of claim 12.

14. The expression cassette of any one of claims 1 to 11 or the expression vector claim 12 for use as a medicament for the treatment of a disorder or for use in gene therapy.

15. An *in vitro* method for the expression of a polypeptide, comprising transfecting a host cell with the expression cassette of any one of claims 1 to 11 or the expression vector of claim 12 and recovering the polypeptide.

16. Use of an expression cassette of any one of claims 1 to 11 or the expression vector of claim 12 for the in vitro expression of a heterologous polypeptide from a mammalian host cell.

## Patentansprüche

1. Expressionskassette, die einen Promotor, eine Polynukleotidsequenz, welche ein Polypeptid codiert, und ein expressionsverstärkendes Element umfasst, wobei das expressionsverstärkende Element eine einem Säugetier-Glyceraldehyd-3-phosphat-Dehydrogenase-(GAPDH)-Promotor nachgeschaltete nicht-translatierte genomische DNA-Sequenz umfasst, wobei das Polypeptid, welches durch die Polynukleotidsequenz codiert ist, nicht GAPDH ist, und wobei die dem Säugetier-GAPDH-Promotor nachgeschaltete genomische DNA-Sequenz innerhalb einer Region beginnt, welche sich etwa von der Nukleotidposition +1 bis etwa zur Nukleotidposition +7000 erstreckt, wobei die Nukleotidposition auf den Transkriptionsstart der GAPDH-mRNA bezogen ist, und wobei die Länge der dem Säugetier-GAPDH-Promotor nachgeschalteten nicht-translatierten genomischen DNA-Sequenz zwischen etwa 200 und etwa 2800 Nukleotide beträgt, und wobei die Expressionskassette weiter eine einem Säugetier-GAPDH-Promotor vorgeschaltete nicht-translatierte genomischer DNA-Sequenz umfasst, wobei die dem Säugetier-GAPDH-Promotor vorgeschaltete nicht-translatierte genomische DNA-Sequenz innerhalb einer Region beginnt, welche sich etwa von dem 5'-Ende des Säugetier-GAPDH-Promotors bis etwa zur Nukleotidposition -3500 erstreckt, wobei die Nukleotidposition auf den Transkriptionsstart der GAPDH-mRNA bezogen ist, und wobei die Länge der dem Säugetier-GAPDH-Promotor vorgeschalteten nicht-translatierten genomischen DNA-Sequenz zwischen etwa 200 und etwa 2800 Nukleotide beträgt.

2. Expressionskassette, die einen Promotor, eine Polynukleotidsequenz, welche ein Polypeptid codiert, und eine einem Säugetier-GAPDH-Promotor vorgeschaltete nicht-translatierte genomische DNA-Sequenz umfasst, wobei das Polypeptid, welches durch die Polynukleotidsequenz codiert ist, nicht GAPDH ist, und wobei die dem Säugetier-GAPDH-Promotor vorgeschaltete nicht-translatierte genomische DNA-Sequenz innerhalb einer Region beginnt, welche sich etwa von dem 5'-Ende des Säugetier-GAPDH-Promotors bis etwa zur Nukleotidposition -3500 erstreckt, wobei die Nukleotidposition auf den Transkriptionsstart der GAPDH-mRNA bezogen ist, wobei die Länge der dem Säugetier-GAPDH-Promotor vorgeschalteten nicht-translatierten genomischen DNA-Sequenz zwischen 200 und etwa 2800 Nukleotide beträgt, mit der Maßgabe, dass die Expressionskassette keinen Säugetier-GAPDH-Promotor oder Fragmente davon umfasst.

3. Expressionskassette nach Anspruch 2, wobei die Expressionskassette weiter eine einem Säugetier-GAPDH-Promotor nachgeschaltete nicht-translatierte genomische DNA-Sequenz umfasst, wobei die dem Säugetier-GAPDH-Promotor nachgeschaltete nicht-translatierte genomische DNA-Sequenz innerhalb einer Region beginnt, welche sich etwa von Nukleotidposition +1 bis etwa zur Nukleotidposition +7000 erstreckt, wobei die Nukleotidposition auf den Transkriptionsstart der GAPDH-mRNA bezogen ist, und wobei die Länge der dem Säugetier-GAPDH-Promotor nachgeschalteten nicht-translatierten genomischen DNA-Sequenz zwischen etwa 200 und etwa 2800 Nukleotide beträgt.

4. Expressionskassette nach einem der Ansprüche 1 bis 3, wobei die dem Säugetier-GAPDH-Promotor nachgeschaltete und/oder vorgeschaltete nicht-translatierte genomische DNA-Sequenz mit der Polynukleotidsequenz, welche das Polypeptid codiert, nicht funktionsfähig verknüpft ist.

5. Expressionskassette nach Anspruch 1 oder 3, wobei die dem Säugetier-GAPDH-Promotor nachgeschaltete nicht-translatierte genomische DNA-Sequenz nach der Polyadenylierungsstelle des Säugetier-GAPDHs beginnt, und wobei die Länge der dem Säugetier-GAPDH-Promotor nachgeschalteten nicht-translatierten genomischen DNA-Sequenz mindestens etwa 100 Nukleotide beträgt und sich bis maximal zum vorletzten Intron des IFF01-Gens erstreckt.

6. Expressionskassette nach Anspruch 1 oder 2, wobei die Länge der dem Säugetier-GAPDH-Promotor vorgeschalteten nicht-translatierten genomischen DNA-Sequenz mindestens etwa 100 Nukleotide beträgt und sich maximal bis zum drittletzten Intron des NCAPD2-Gens erstreckt.

7. Expressionskassette nach einem der Ansprüche 1 bis 3, wobei die dem Säugetier-GAPDH-Promotor nachgeschaltete und/oder vorgeschaltete nicht-translatierte genomische DNA-Sequenz Nagetier- oder menschlichen Ursprungs ist.

8. Expressionskassette nach Anspruch 1 oder 3, wobei die dem Säugetier-GAPDH-Promotor nachgeschaltete nicht-translatierte genomische DNA-Sequenz die Nukleotidsequenz, ausgewählt aus der Gruppe, bestehend aus: SEQ ID NOs: 8 und 21; einer Nukleotidsequenz, welche mindestens zu 80 % mit den SEQ ID NOs: 8 oder 21 identisch ist; Fragmenten davon oder einer komplementären Nukleotidsequenz davon, umfasst.

9. Expressionskassette nach Anspruch 1 oder 2, wobei die dem Säugetier-GAPDH-Promotor vorgeschaltete nicht-translatierte genomische DNA-Sequenz eine Nukleotidsequenz, ausgewählt aus der Gruppe, bestehend aus den SEQ ID NO: 7, 9, 10, 11, 12, 13, 14, 15, 16, 20, 22, 23, 24, 25, 26, 27 und 28, welche fünf oder weniger Nukleinsäuremodifikationen umfassen; einer Nukleotid Sequenz, welche mindestens zu 80 % mit den Nukleinsäure Sequenzen, ausgewählt aus der Gruppe, bestehend aus den SEQ ID NO: 7, 9, 10, 11, 12, 13, 14, 15, 16, 20, 22, 23, 24, 25, 26, 27 und 28 oder Fragmenten davon, identisch ist; Fragmenten davon; oder einer komplementären Nukleinsäuresequenz davon, umfasst.

10. Expressionskassette nach einem der Ansprüche 1 bis 3, wobei der Promotor ausgewählt ist aus der Gruppe bestehend aus dem SV40-Promotor, dem MPSV-Promotor, dem Maus-CMV, dem menschlichen tk, dem menschlichen CMV, dem Ratten-CMV, dem menschlichen EF1alpha, dem EFlalpha des chinesischen Hamsters, dem menschlichen GAPDH, hybriden Promotoren, einschließlich dem MYC-, HYK- und CX-Promotor.

11. Expressionskassette nach einem der Ansprüche 1 bis 3, wobei das Polypeptid ausgewählt ist aus der Gruppe, bestehend aus Antikörpern, Antikörperfragmenten oder Antikörperderivaten.

12. Expressionsvektor, der in Reihenfolge umfasst:
a) eine einem Säugetier-GAPDH-Promotor vorgeschaltete nicht-translatierte genomische DNA-Sequenz, wobei die dem Säugetier-GAPDH-Promotor vorgeschaltete nicht-translatierte genomische DNA-Sequenz innerhalb einer Region beginnt, welche sich etwa von dem 5'-Ende des Säugetier-GAPDH-Promotors bis etwa zur Nukleotidposition -3500 erstreckt, wobei die Nukleotidposition auf den Transkriptionsstart der GAPDH-mRNA bezogen ist, und wobei die Länge der dem Säugetier-GAPDH-Promotor vorgeschalteten nicht-translatierten genomischen DNA-Sequenz zwischen etwa 200 und etwa 2800 Nukleotide beträgt
b) einen Promotor
c) eine Polynukleotidsequenz, welche ein Polypeptid codiert
d) eine Polyadenylierungsstelle
e) einen Verstärker
f) eine einem Säugetier-GAPDH-Promotor nachgeschaltete nicht-translatierte genomische DNA-Sequenz, wobei das Polypeptid, welches durch die Polynukleotidsequenz codiert ist, nicht GAPDH ist, wobei die dem Säugetier-GAPDH-Promotor nachgeschaltete genomische DNA-Sequenz innerhalb einer Region beginnt, welche sich etwa von der Nukleotidposition +1 bis etwa zur Nukleotidposition +7000 erstreckt, wobei die Nukleotidposition auf den Transkriptionsstart der GAPDH-mRNA bezogen ist, und wobei die Länge der dem Säugetier-GAPDH-Promotor nachgeschalteten nicht-translatierten genomischen DNA-Sequenz zwischen etwa 200 und etwa 2800 Nukleotide beträgt.

13. Wirtszelle, welche eine Expressionskassette nach einem der Ansprüche 1 bis 11 oder einen Expressionsvektor nach Anspruch 12 umfasst.

14. Expressionskassette nach einem der Ansprüche 1 bis 11 oder Expressionsvektor nach Anspruch 12 zur Verwendung als Medikament zur Behandlung einer Störung oder zur Verwendung in der Gentherapie.

15. *In vitro* Verfahren zur Expression eines Polypeptids, welches das Transfizieren einer Wirtszelle mit der Expressionskassette nach einem der Ansprüche 1 bis 11 oder dem Expressionsvektor nach Anspruch 12 umfasst und Gewinnen des Polypeptids.

16. Verwendung einer Expressionskassette nach einem der Ansprüche 1 bis 11 oder des Expressionsvektors nach Anspruch 12 für die *in vitro* Expression eines herterologen Polypeptids aus einer Säugetierwirtszelle.

## Revendications

1. Cassette d'expression qui comprend un promoteur, une séquence polynucléotidique codant pour un polypeptide, et un élément d'amélioration d'expression dans laquelle un élément d'amélioration d'expression comprend une séquence d'ADN génomique non traduite en aval d'un promoteur de la glycéraldéhyde 3-phosphate déshydrogénase (GAPDH) mammalienne, dans laquelle le polypeptide codé par la séquence nucléotidique n'est pas la GAPDH, et dans laquelle la séquence d'ADN génomique en aval du promoteur de la GAPDH mammalienne commence à l'intérieur d'une région s'étendant d'une position nucléotidique proche de +1 à une position nucléotidique proche de +7000, dans laquelle la position nucléotidique est relative au début de la transcription de l'ARNm de GAPDH, et dans laquelle la longueur de la séquence d'ADN génomique non traduite en aval du promoteur de la GAPDH mammalienne va d'environ 200 à environ 2800 nucléotides et dans laquelle la cassette d'expression comprend en outre une séquence d'ADN génomique non traduite en amont d'un promoteur de GAPDH mammalienne, dans laquelle la séquence d'ADN génomique non traduite en amont du promoteur de GAPDH mammalienne commence à l'intérieur d'une région s'étendant d'environ l'extrémité 5' du promoteur de GAPDH mammalienne à une position nucléotidique proche de -3500, dans laquelle la position nucléotidique est relative au début de la transcription de l'ARNm de GAPDH, et dans laquelle la longueur de la séquence d'ADN génomique non traduite en amont du promoteur de GAPDH mammalienne va d'environ 200 à environ 2800 nucléotides.

2. Cassette d'expression qui comprend un promoteur, une séquence polynucléotidique codant pour un polypeptide, et une séquence d'ADN génomique non traduite en amont d'un promoteur de GAPDH mammalienne, dans laquelle le polypeptide codé par la séance polynucléotidique n'est pas la GAPDH, et dans laquelle la séquence d'ADN génomique non traduite en amont du promoteur de GAPDH mammalienne commence à l'intérieur d'une région s'étendant d'environ l'extrémité 5' du promoteur de GAPDH mammalienne à une position nucléotidique proche de -3500, dans laquelle la position nucléotidique est relative au début de la transcription de l'ARNm de GAPDH, dans laquelle la longueur de la séquence d'ADN génomique non traduite en amont du promoteur de GAPDH mammalienne va d'environ 200 à environ 2800 nucléotides, à condition que la cassette d'expression ne comprenne pas de promoteur de GAPDH mammalienne ou des fragments de celui-ci.

3. Cassette d'expression selon la revendication 2, dans laquelle la cassette d'expression comprend en outre une séquence d'ADN génomique non traduite en aval d'un promoteur de GAPDH mammalienne, dans laquelle une séquence d'ADN génomique non traduite en aval du promoteur de GAPDH mammalienne commence à l'intérieur d'une région s'étendant d'une position nucléotidique proche de +1 à une position nucléotidique proche de +7000, dans laquelle la position nucléotidique est relative au début de la transcription de l'ARNm de GAPDH, et dans laquelle la longueur de la séquence d'ADN génomique non traduite en aval du promoteur de GAPDH mammalienne va d'environ 200 à environ 2800 nucléotides.

4. Cassette d'expression selon l'une quelconque des revendications 1 à 3, dans laquelle la séquence d'ADN génomique non traduite en aval et/ou en amont du promoteur de GAPDH mammalienne n'est pas liée de manière fonctionnelle à la séquence polynucléotidique codant pour le polypeptide.

5. Cassette d'expression selon la revendication 1 ou 3, dans laquelle la séquence d'ADN génomique non traduite en aval du promoteur de GAPDH mammalienne commence en aval du site de polyadénylation de GAPDH mammalienne et dans laquelle la longueur de la séquence d'ADN génomique non traduite en aval du promoteur de GAPDH mammalienne est d'au moins environ 100 nucléotides et s'étend à son maximum à l'avant-dernier intron du gène IFF01.

6. Cassette d'expression selon la revendication 1 ou 2, dans laquelle la longueur de la séquence d'ADN génomique non traduite en amont du promoteur de GAPDH mammalienne est d'au moins environ 100 nucléotides et s'étend à son maximum à l'antépénultième intron du gène NCAPD2.

7. Cassette d'expression selon l'une quelconque des revendications 1 à 3, dans laquelle la séquence d'ADN génomique non traduite en aval et/ou en amont du promoteur de GAPDH mammalienne provient de rongeurs ou est d'origine humaine.

8. Cassette d'expression selon la revendication 1 ou 3, dans laquelle la séquence d'ADN génomique non traduite en aval du promoteur de GAPDH mammalienne comprend la séquence nucléotidique choisie parmi le groupe constitué de : SEQ ID NO : 8 et 21 ; une séquence nucléotidique au moins à 80 % identique aux SEQ ID NO : 8 ou 21 ; des fragments de celles-ci ; ou une séquence nucléotidique complémentaire de celles-ci.

9. Cassette d'expression selon la revendication 1 ou 2, dans laquelle la séquence d'ADN génomique non traduite en amont du promoteur de GAPDH mammalienne comprend une séquence nucléotidique choisie parmi le groupe constitué de SEQ ID NO : 7, 9, 10, 11, 12, 13, 14, 15, 16, 20, 22, 23, 24, 25, 26, 27 et 28 comprenant cinq ou moins de cinq modifications d'acides nucléiques ; une séquence nucléotidique au moins à 80 % identique à la séquence nucléotidique choisie parmi le groupe constitué de SEQ ID NO : 7, 9, 10, 11, 12, 13, 14, 15, 16, 20, 22, 23, 24, 25, 26, 27 et 28 ou des fragments de celles-ci ; des fragments de celles-ci ; ou une séquence nucléotidique complémentaire de celles-ci.

10. Cassette d'expression selon l'une quelconque des revendications 1 à 3, dans laquelle le promoteur est choisi parmi le groupe constitué du promoteur de SV40, du promoteur de MPSV, du CMV murin, de la tk humaine, du CMV humain, du CMV du rat, de l'EF1alpha humain, de l'EF1alpha du hamster chinois, de la GAPDH humaine, des promoteurs hybrides incluant un promoteur de MYC, de HYK et de CX.

11. Cassette d'expression selon l'une quelconque des revendications 1 à 3, dans laquelle le polypeptide est choisi parmi le groupe constitué des anticorps, des fragments d'anticorps ou des dérivés d'anticorps.

12. Vecteur d'expression, qui comprend dans l'ordre :
a) une séquence d'ADN génomique non traduite en amont d'un promoteur de GAPDH mammalienne, dans lequel la séquence d'ADN génomique non traduite en amont du promoteur de GAPDH mammalienne commence à l'intérieur d'une région s'étendant d'environ l'extrémité 5' du promoteur de GAPDH mammalienne à une position nucléotidique proche de -3500, dans lequel la position nucléotidique est relative au début de la transcription de l'ARNm de GAPDH, et dans lequel la longueur de la séquence d'ADN génomique non traduite en amont du promoteur de GAPDH mammalienne va d'environ 200 à environ 2800 nucléotides
b) un promoteur
c) une séquence polynucléotidique codant pour un polypeptide
d) un site de polyadénylation
e) un amplificateur
f) une séquence d'ADN génomique non traduite en aval d'un promoteur de GAPDH mammalienne, dans lequel le polypeptide codé par la séquence polynucléotidique n'est pas la GAPDH, dans lequel la séquence d'ADN génomique en aval du promoteur de GAPDH mammalienne commence à l'intérieur d'une région s'étendant d'une position nucléotidique proche de +1 à une position nucléotidique proche de +7000, dans lequel la position nucléotidique est relative au début de la transcription de l'ARNm de GAPDH, et dans lequel la longueur de la séquence d'ADN génomique non traduite en aval du promoteur de GAPDH mammalienne va d'environ 200 à environ 2800 nucléotides.

13. Cellule hôte comprenant une cassette d'expression selon l'une quelconque des revendications 1 à 11 ou un vecteur d'expression selon la revendication 12.

14. Cassette d'expression selon l'une quelconque des revendications 1 à 11 ou vecteur d'expression selon la revendication 12 pour une utilisation en tant que médicament pour le traitement d'un trouble ou pour une utilisation en thérapie génique.

15. Procédé *in vitro* pour l'expression d'un polypeptide, comprenant la transfection d'une cellule hôte avec la cassette d'expression selon l'une quelconque des revendications 1 à 11 ou le vecteur d'expression selon la revendication 12 et la récupération du polypeptide.

16. Utilisation d'une cassette d'expression selon l'une quelconque des revendications 1 à 11 ou du vecteur d'expression selon la revendication 12 pour l'expression in vitro d'un polypeptide hétérologue à partir d'une cellule hôte mammalienne.
